# EUROPEAN PATENT APPLICATION

(11) **EP 4 635 969 A1**
(43) Date of publication of application: **22.10.2025**
(21) Application number: 23902832.7
(22) Date of filing: 15.12.2023
(51) Int. Cl.: C07J 71/00, A61P 29/00, A61P 27/02, A61P 9/10, A61P 9/00, A61P 27/06, A61P 27/12, A61P 17/06, A61P 17/00, A61P 37/08, A61P 37/02, A61P 11/00, A61P 11/02, A61P 11/06, A61P 13/12, A61K 31/585

(54) **NOVEL GLUCOCORTICOID, METHOD FOR PREPARING SAME, AND USE THEREOF**

(30) Priority: 16.12.2022 CN 202211625242
(71) Applicant: Tianjin Pharmaceutical Biotechnology Co., Ltd., Tianjin 300171 (CN)
(72) Inventor: REN, Nan, Tianjin 300171 (CN); ZHANG, Jie, Tianjin 300171 (CN); SUN, Shuangyong, Tianjin 300171 (CN); WANG, Ruina, Tianjin 300171 (CN); WANG, Yan, Tianjin 300171 (CN); ZHANG, Yanwen, Tianjin 300171 (CN); ZHAO, Xinying, Tianjin 300171 (CN); LIU, Dengke, Tianjin 300171 (CN)
(74) Representative: Bayramoglu et al.
(86) International application number: PCT/CN2023/139198
(87) International publication number: WO 2024/125639

(57) **Abstract**

The present disclosure relates to the technical field of medicinal chemistry, and particularly, to a novel glucocorticoid, a method for preparing same, and use thereof. The novel glucocorticoid comprises a compound represented by formula I, an optical isomer thereof, a pharmaceutically acceptable salt thereof, or a solvate thereof, wherein the structure of the compound represented by formula I is shown herein. The compound provided by the present disclosure exhibits a high plasma clearance rate, a short half-life, reduced systemic exposure, and enhanced glucocorticoid receptor (GR) selectivity, thereby significantly reducing the adverse effects associated with systemic absorption of the glucocorticoid drug. The present disclosure has significant anti-inflammatory activity in animals with asthma, uveitis, dry eye syndrome, inflammatory bowel disease, psoriasis, and the like. The compound provided by the present disclosure demonstrates good safety, with no abnormal intraocular pressure observed after continuous ocular administrations in guinea pigs and no significant changes in the thickness of the epidermal layer after continuous dermal administrations in rats.

## Description

### Technical Field

The present disclosure relates to the technical field of medicinal chemistry, and particularly, to a novel glucocorticoid, a method for preparing same, and use thereof in the manufacture of medicaments for the treatment of glucocorticoid receptor-mediated diseases, including, but not limited to, various ocular diseases, respiratory diseases, autoimmune diseases, skin diseases, renal diseases, and the like.

### Background Art

Glucocorticoids have anti-inflammatory properties and are widely used in the treatment of inflammatory disorders or inflammatory diseases, such as respiratory diseases, rheumatic immune diseases, renal system diseases, and skin system diseases, which can control disease symptoms, slow down disease progression, and exhibit significant clinical effects. The main mechanisms of action of glucocorticoids in the treatment of various inflammatory diseases are to inhibit the migration and activation of inflammatory cells such as eosinophils and neutrophils; inhibit the production of cytokines such as prostaglandins and leukotrienes; inhibit the release of inflammatory mediators; enhance the responsiveness of β2 receptors in bronchial smooth muscle cells; and the like. Since the glucocorticoid receptors *in vivo* are widely distributed in effector cells, the effects of glucocorticoids are dose-dependent. When administered as drugs at doses exceeding physiological levels, glucocorticoids exhibit a wide range of pharmacological effects, particularly anti-inflammatory, immunosuppressive, and anti-shock effects, and other important pharmacological effects. However, they also cause many adverse reactions, such as obesity, moon face, buffalo hump, edema, spontaneous fracture, osteoporosis and femoral head necrosis, peptic ulcers, acute gastrointestinal bleeding, insomnia, hirsutism, and decreased resistance, which limits the clinical application of glucocorticoid drugs. For example, the main administration route of topical glucocorticoids is application. However, if used excessively for a long time, glucocorticoids may cause various side effects including weakened skin barrier function, and significantly increase the systemic exposure to drugs in patients, thereby leading to systemic absorption side effects of glucocorticoids. In particular, glucocorticoid-induced osteoporosis accounts for 30% to 50% of patients treated with long-term glucocorticoids. Currently, there is no effective treatment for this, and once discovered, glucocorticoid drugs can only be discontinued.

Although local administration of glucocorticoid drugs is preferred, attention still needs to be paid to the levels of the steroid drugs entering the systemic circulation. After entering the systemic circulation, commonly used glucocorticoids have a relatively long plasma half-life in the human body. For example, the half-life of fluticasone propionate is 180 min, while those of dexamethasone and betamethasone are as long as 100-300 min. The local administration of glucocorticoids by inhalation for the treatment of respiratory diseases poses a risk of systemic exposure, as part of the drug enters the systemic circulation through the lungs. For the treatment of ocular diseases by local administration, part of the drug enters the systemic circulation due to low corneal permeability, efficient drainage, and the presence of eyelid blood vessels. For dermal administration, local injection, and implantable medical devices, there is a serious risk of leakage into the systemic circulation. Therefore, when applied locally, glucocorticoid drugs preferably have the characteristic of significantly avoiding systemic exposure.

In view of the above, there is an urgent need to develop a class of glucocorticoids with high anti-inflammatory activity and minimal side effects. The compound described in the present disclosure (including the compound itself, an optical isomer thereof, a pharmaceutically acceptable salt thereof, or a solvate thereof) and a pharmaceutically acceptable composition thereof can be used for treating or alleviating various glucocorticoid receptor-mediated conditions. More specifically, the compound of the present disclosure is preferably used for treating at least one glucocorticoid receptor-mediated disease. For example, the compound of the present disclosure can be used for treating the following diseases:
Ocular diseases: including, but not limited to, external ocular diseases such as blepharitis, conjunctivitis, keratitis, allergic conjunctivitis, epidemic keratoconjunctivitis, scleritis, and episcleritis; anterior ocular diseases such as iritis, iridocyclitis, and uveitis; posterior ocular diseases such as dry eye syndrome, diabetic retinopathy, wet age-related macular degeneration, choroidal neovascularization, posterior uveitis; postoperative inflammation following cataract, glaucoma, retinal detachment, and strabismus correction surgery; abnormal corneal wound healing; and ocular pain.
Airway diseases: including, but not limited to, pulmonary fibrosis, emphysema, chronic bronchitis, asthma, pneumonia, cystic fibrosis, chronic obstructive pulmonary disease (COPD), bronchitis, and respiratory distress syndrome.
Laryngeal, nasal, and otic diseases: including, but not limited to, sinus problems, hearing problems, toothache, tonsillitis, ulcers, and rhinitis.
Skin diseases: including, but not limited to, hyperkeratosis, parakeratosis, granular layer thickening, acanthosis, dyskeratosis, spongiosis, and ulcers.
Intestinal diseases: including, but not limited to, inflammatory bowel disease (IBD), enteritis, gastroenteritis, intestinal obstruction, ileitis, appendicitis, ulcerative colitis, and Crohn's disease.
Inflammatory diseases: including, but not limited to, contact dermatitis, atopic dermatitis, psoriasis, rheumatoid arthritis, juvenile rheumatoid arthritis, ankylosing spondylitis, and psoriatic arthritis.
Nervous system diseases: including, but not limited to, neuropathic pain, and for the prevention of neurodegeneration in various nervous system diseases and stimulation of nerve regeneration.
Renal diseases: including, but not limited to, renal fibrosis, renal insufficiency, and chronic glomerulonephritis.
Bone diseases: including, but not limited to, osteoarthritis.

### Summary of the Invention

The major objective of the present disclosure is to provide a novel glucocorticoid, a method for preparing same, and use thereof, in order to at least partially solve at least one of the above technical problems.

The compound represented by formula I provided by the present disclosure has the ability to interact with the glucocorticoid receptor and thus has a wide range of therapeutic uses as described below, which is primarily due to the role of the glucocorticoid receptor in the physiology of all mammals. Therefore, the present disclosure relates to a compound represented by formula I, an optical isomer thereof, a pharmaceutically acceptable salt thereof, or a pharmaceutically acceptable solvate of the compound or the salt, for use in the treatment or prevention of glucocorticoid receptor-mediated diseases. The present disclosure further relates to the use of the compound represented by formula I, an optical isomer thereof, a pharmaceutically acceptable salt thereof, or a pharmaceutically acceptable solvate of the compound or the salt, in the manufacture of medicaments for the treatment of glucocorticoid receptor-mediated diseases. The present disclosure further relates to a method for treating a mammal, including a human, using a glucocorticoid receptor agonist, the method including treating the mammal with an effective amount of the compound represented by formula I, an optical isomer thereof, a pharmaceutically acceptable salt thereof, or a pharmaceutically acceptable solvate of the compound or the salt. Moreover, the present disclosure also relates to a method for preparing the compound, a pharmaceutical composition comprising the compound, and others.

The present application provides the following technical solutions:
In a first aspect, provided is Embodiment 1:
   A compound represented by formula I, having the following structural formula: 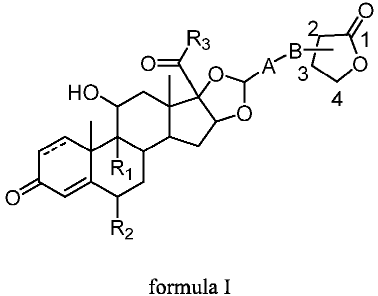
   an optical isomer thereof, a pharmaceutically acceptable salt thereof, or a solvate thereof,
   Wherein A, B, R₁, R₂, and R₃ as shown in the structural formula of the compound represented by formula I are selected independently of each other, and:
      A is selected from substituted or unsubstituted aryl having 6 to 10 carbon atoms, substituted or unsubstituted heteroaryl or heterocyclyl having 4 to 10 carbon atoms, and substituted or unsubstituted cycloalkyl having 3 to 10 carbon atoms, wherein the substituents are selected from one or more, or one, of the following: halogen, hydroxyl, carbonyl, amino, cyano, carboxyl, aryl having 6 to 10 carbon atoms, heteroaryl having 4 to 10 carbon atoms, cycloalkyl having 3 to 6 carbon atoms, alkoxy having 1 to 10 carbon atoms, alkylamino having 1 to 10 carbon atoms, or alkyl ester having 1 to 10 carbon atoms;
      Preferably, A is selected from substituted or unsubstituted phenyl, substituted or unsubstituted piperidinyl, substituted or unsubstituted cyclohexyl, substituted or unsubstituted pyridinyl, and substituted or unsubstituted pyrimidinyl, wherein the substituents are selected from one or more, or one, of the following: halogen or cyano;
      More preferably, A is selected from substituted or unsubstituted phenyl, substituted or unsubstituted cyclohexyl, substituted or unsubstituted pyridyl, and substituted or unsubstituted pyrimidinyl, wherein the substituents are selected from one or more, or one, of the following: halogen or cyano;
      Further preferably, A is selected from substituted or unsubstituted phenyl, substituted or unsubstituted cyclohexyl, and substituted or unsubstituted pyridyl, wherein the substituents are selected from one or more, or one, of the following: halogen;
      Even more preferably, A is selected from substituted or unsubstituted phenyl and substituted or unsubstituted pyridyl, wherein the substituents are selected from one or more, or one, of the following: halogen;
      B is selected from absent, O, S, NH, CH₂, OCH₂, SCH₂, NHCH₂, CH₂O, CH₂S, CH₂SO, CH₂SO₂, CH₂NH, C=O, or NH(CO);
      Preferably, B is selected from O, S, NH, CH₂, OCH₂, SCH₂, NHCH₂, CH₂O, CH₂S, CH₂SO, CH₂SO₂, CH₂NH, C=O, or NH(CO);
      More preferably, B is selected from S, CH₂, OCH₂, SCH₂, NHCH₂, CH₂O, CH₂S, CH₂SO, CH₂SO₂, CH₂NH, or NH(CO);
      Further preferably, B is selected from S, CH₂O, CH₂S, CH₂SO, or CH₂NH;
      Even more preferably, B is selected from CH₂O, CH₂S, or CH₂NH;
      B is attached to the 2-, 3-, or 4-position of the ring 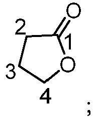
      Preferably, B is attached to the 2- or 3-position of the ring 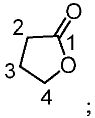
      R₁ is selected from H or halogen;
      Preferably, R₁ is selected from halogen;
      R₂ is selected from H, CH₃, or halogen;
      Preferably, R₂ is selected from H or halogen;
      R₃ is selected from CH₂R₄, wherein R₄ is selected from OH, halogen, OR₅, or OCOR₅, or R₃ is selected from SCH₂R₆ or OCH₂R₆;
      Wherein R₅ is selected from alkyl having 1 to 6 carbon atoms, alkenyl having 2 to 6 carbon atoms, alkynyl having 2 to 6 carbon atoms, aryl having 6 to 10 carbon atoms, heteroaryl or heterocyclyl having 4 to 10 carbon atoms; R₆ is selected from halogen or CN;
      Preferably, R₃ is selected from CH₂R₄ wherein R₄ is selected from OH or halogen, or R₃ is selected from SCH₂R₆ or OCH₂R₆ wherein R₆ is selected from halogen or CN;
      More preferably, R₃ is selected from CH₂R₄ wherein R₄ is selected from OH, or R₃ is selected from SCH₂R₆ or OCH₂R₆ wherein R₆ is selected from halogen or CN;
      Preferably, the halogen is selected from F or Cl; 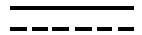 is a single bond or a double bond, preferably a double bond.
Embodiment 2: The compound according to Embodiment 1, wherein the compound represented by formula I has the following structural formula I': 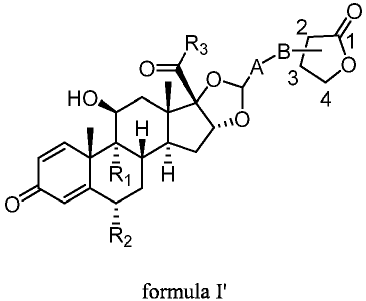 Wherein the variables A, B, R₁, R₂, R₃, and the position of attachment of B to the ring 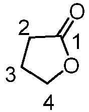 are as defined in Embodiment 1.
Embodiment 3: The compound according to Embodiments 1 or 2, wherein the compound represented by formula I is selected from the following structural formulas I-1 or 1-2, and the compound represented by formula I' is selected from the following structural formulas I'-1 or I'-2: 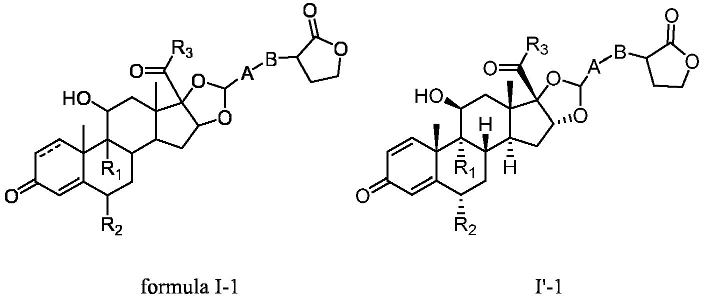 Wherein A, B, R₁, R₂, and R₃ as shown in the structural formulas of the compounds represented by formulas I-1 and I'-1 are selected independently of each other, and:
   A is selected from substituted or unsubstituted cyclohexyl, substituted or unsubstituted piperidinyl, substituted or unsubstituted phenyl, substituted or unsubstituted pyridinyl, and substituted or unsubstituted pyrimidinyl, wherein the substituents are selected from one or more, or one, of the following: halogen, preferably F or Cl;
   Preferably, A is selected from: 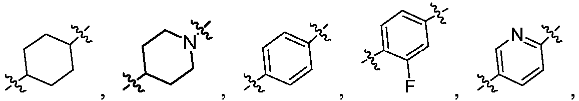 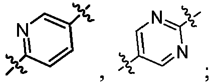
   B is selected from O, S, NH, CH₂, OCH₂, SCH₂, NHCH₂, CH₂O, CH₂S, CH₂SO, CH₂SO₂, CH₂NH;
   R₁ is selected from H or halogen; preferably, R₁ is selected from H, F, or Cl;
   R₂ is selected from H or halogen; preferably, R₂ is selected from H or F;
   R₃ is selected from the following: CH₂R₄ wherein R₄ is selected from OH or halogen; SCH₂R₆ or OCH₂R₆ wherein R₆ is selected from halogen or CN;
   Preferably, R₃ is selected from the following: CH₂R₄ wherein R₄ is selected from OH or Cl; SCH₂R₆ or OCH₂R₆ wherein R₆ is selected from F or CN; 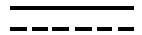 is a single bond or a double bond, preferably a double bond; 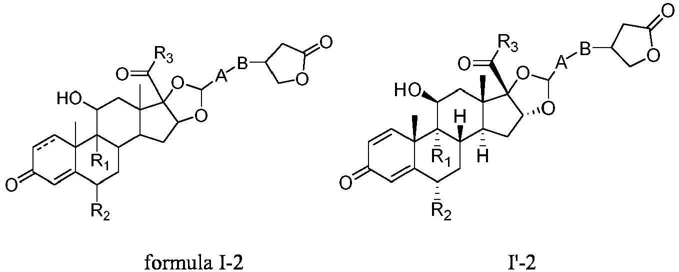 Wherein A, B, R₁, R₂, and R₃ as shown in the structural formulas of the compounds represented by formulas 1-2 and I'-2 are selected independently of each other, and:
      A is selected from substituted or unsubstituted cyclohexyl, substituted or unsubstituted piperidinyl, and substituted or unsubstituted phenyl, wherein the substituents are selected from one or more, or one, of the following: halogen, preferably F or Cl, or cyano;
      Preferably, A is selected from: 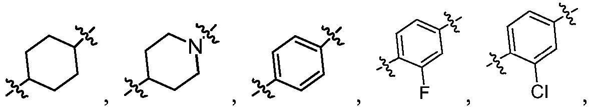 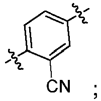
      B is selected from C=O, NH(CO), CH₂O, CH₂S, CH₂NH;
      R₁ is selected from H or halogen; preferably, R₁ is selected from H, F, or Cl;
      R₂ is selected from H or halogen; preferably, R₂ is selected from H or F;
      R₃ is selected from the following: CH₂R₄ wherein R₄ is selected from OH or halogen; SCH₂R₆ wherein R₆ is selected from halogen;
      Preferably, R₃ is selected from the following: CH₂R₄ wherein R₄ is selected from OH or Cl; SCH₂R₆ wherein R₆ is selected from F; 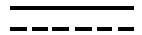 is a single bond or a double bond, preferably a double bond.
Embodiment 4: The compound according to Embodiments 1 or 2, wherein
   A, B, R₁, R₂, and R₃ are selected independently of each other, and:
   A is selected from substituted or unsubstituted cyclohexyl, substituted or unsubstituted phenyl, substituted or unsubstituted pyridyl, and substituted or unsubstituted pyrimidinyl, wherein the substituents are selected from one or more, or one, of the following: halogen, preferably F or Cl, or cyano;
   Preferably, A is selected from: 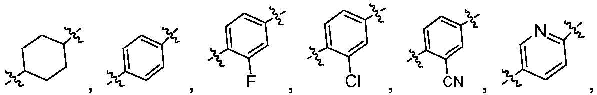 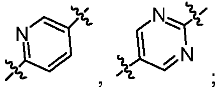
   B is selected from S, CH₂, OCH₂, SCH₂, NHCH₂, CH₂O, CH₂S, CH₂SO, CH₂SO₂, CH₂NH, NH(CO);
   R₁ is selected from H or halogen; preferably, R₁ is selected from H, F, or Cl;
   R₂ is selected from H or halogen; preferably, R₂ is selected from H or F;
   R₃ is selected from the following: CH₂R₄ wherein R₄ is selected from OH or halogen; SCH₂R₆ or OCH₂R₆ wherein R₆ is selected from halogen or CN;
   Preferably, R₃ is selected from the following: CH₂R₄ wherein R₄ is selected from OH or Cl;
   SCH₂R₆ or OCH₂R₆ wherein R₆ is selected from F or CN; 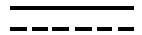 is a single bond or a double bond, preferably a double bond.
Embodiment 5: The compound according to any one of Embodiments 1, 2, and 4, wherein the compound represented by formula I is selected from the following structural formulas I-1 or I-2, and the compound represented by formula I' is selected from the following structural formulas I'-1 or I'-2: 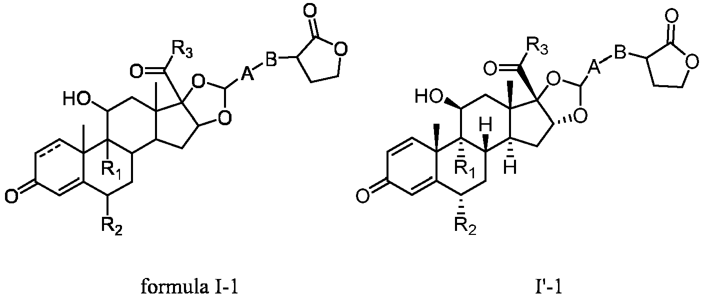 Wherein A, B, R₁, R₂, and R₃ as shown in the structural formulas of the compounds represented by formulas I-1 and I'-1 are selected independently of each other, and:
   A is selected from substituted or unsubstituted cyclohexyl, substituted or unsubstituted phenyl, substituted or unsubstituted pyridyl, and substituted or unsubstituted pyrimidinyl, wherein the substituents are selected from one or more, or one, of the following: halogen, preferably F or Cl;
   Preferably, A is selected from: 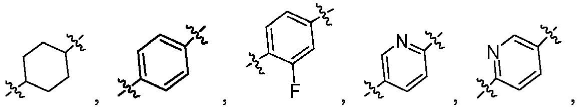 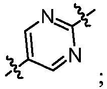
   B is selected from S, CH₂, OCH₂, SCH₂, NHCH₂, CH₂O, CH₂S, CH₂SO, CH₂SO₂, CH₂NH;
   R₁ is selected from H or halogen; preferably, R₁ is selected from H, F, or Cl;
   R₂ is selected from H or halogen; preferably, R₂ is selected from H or F;
   R₃ is selected from the following: CH₂R₄ wherein R₄ is selected from OH or halogen; SCH₂R₆ or OCH₂R₆ wherein R₆ is selected from halogen or CN;
   Preferably, R₃ is selected from the following: CH₂R₄ wherein R₄ is selected from OH or Cl; SCH₂R₆ or OCH₂R₆ wherein R₆ is selected from F or CN; 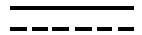 is a single bond or a double bond, preferably a double bond; 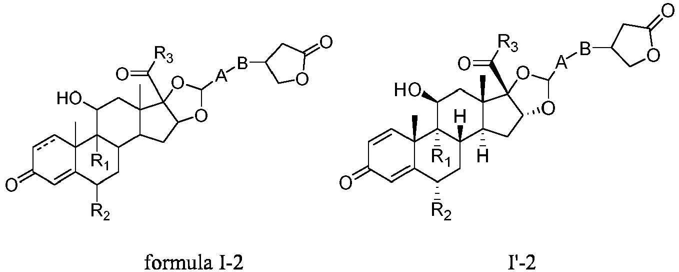 Wherein A, B, R₁, R₂, and R₃ as shown in the structural formulas of the compounds represented by formulas 1-2 and I'-2 are selected independently of each other, and:
      A is selected from substituted or unsubstituted phenyl, wherein the substituents are selected from one or more, or one, of the following: halogen, preferably F or Cl, or cyano;
      Preferably, A is selected from: 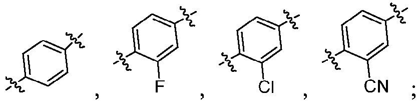
         B is selected from NH(CO), CH₂O, CH₂S, CH₂NH;
         R₁ is selected from H or halogen; preferably, R₁ is selected from H, F, or Cl;
         R₂ is selected from H or halogen; preferably, R₂ is selected from H or F;
         R₃ is selected from the following: CH₂R₄ wherein R₄ is selected from OH or halogen; SCH₂R₆ wherein R₆ is selected from halogen;
         Preferably, R₃ is selected from the following: CH₂R₄ wherein R₄ is selected from OH or Cl; SCH₂R₆ wherein R₆ is selected from F; 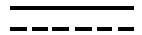 is a single bond or a double bond, preferably a double bond.
Embodiment 6: The compound according to Embodiments 1 or 2, wherein
   A, B, R₁, R₂, and R₃ are selected independently of each other, and:
   A is selected from substituted or unsubstituted cyclohexyl, substituted or unsubstituted phenyl, and substituted or unsubstituted pyridyl, wherein the substituents are selected from one or more, or one, of the following: halogen, preferably F or Cl;
   Preferably, A is selected from 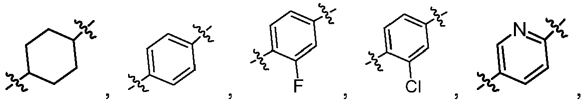 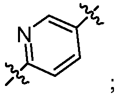
   B is selected from S, CH₂O, CH₂S, CH₂SO, CH₂NH;
   R₁ is selected from halogen; preferably, R₁ is selected from F or Cl;
   R₂ is selected from H or halogen; preferably, R₂ is selected from H or F;
   R₃ is selected from the following: CH₂R₄ wherein R₄ is selected from OH or halogen; SCH₂R₆ or OCH₂R₆ wherein R₆ is selected from halogen or CN;
   Preferably, R₃ is selected from the following: CH₂R₄, wherein R₄ is selected from OH or Cl; SCH₂R₆ or OCH₂R₆, wherein R₆ is selected from F or CN; 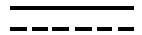 is a single bond or a double bond, preferably a double bond.
Embodiment 7: The compound according to any one of Embodiments 1, 2, and 6, wherein, the compound represented by formula I is selected from the following structural formulas I-1 or I-2, and the compound represented by formula I' is selected from the following structural formulas I'-1 or I'-2: 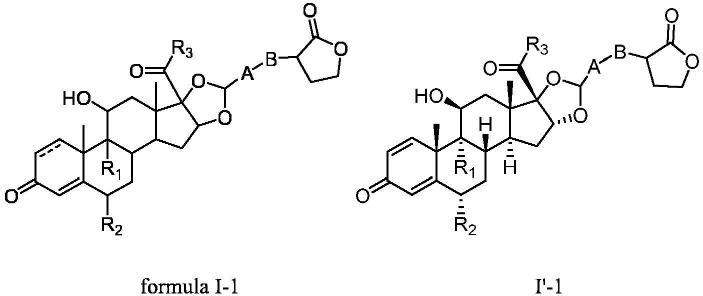 Wherein A, B, R₁, R₂, and R₃ as shown in the structural formulas of the compounds represented by formulas I-1 and I'-1 are selected independently of each other, and:
   A is selected from substituted or unsubstituted cyclohexyl, substituted or unsubstituted phenyl, and substituted or unsubstituted pyridyl, wherein the substituents are selected from one or more, or one, of the following: halogen, preferably F or Cl;
   Preferably, A is selected from: 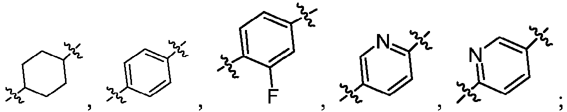
   B is selected from S, CH₂O, CH₂S, CH₂SO, CH₂NH;
   R₁ is selected from halogen; preferably, R₁ is selected from F or Cl;
   R₂ is selected from H or halogen; preferably, R₂ is selected from H or F;
   R₃ is selected from the following: CH₂R₄ wherein R₄ is selected from OH or halogen; SCH₂R₆ or OCH₂R₆ wherein R₆ is selected from halogen or CN;
   Preferably, R₃ is selected from the following: CH₂R₄ wherein R₄ is selected from OH or Cl; SCH₂R₆ or OCH₂R₆ wherein R₆ is selected from F or CN; 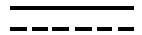 is a single bond or a double bond, preferably a double bond; 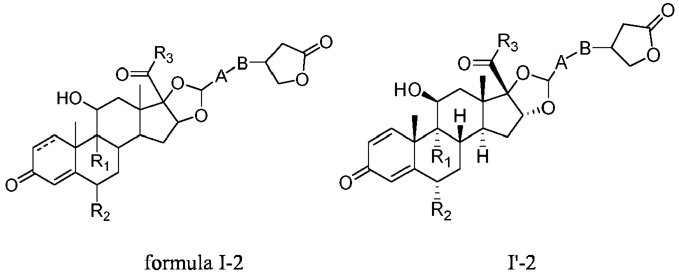 Wherein A, B, R₁, R₂, and R₃ as shown in the structural formulas of the compounds represented by formulas 1-2 and I'-2 are selected independently of each other, and:
      A is selected from substituted or unsubstituted phenyl, wherein the substituents are selected from one or more, or one, of the following: halogen, preferably Cl;
      Preferably, A is selected from: 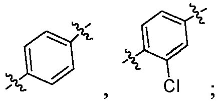
      B is selected from CH₂O, CH₂S, CH₂NH;
      R₁ is selected from halogen; preferably, R₁ is selected from F or Cl;
      R₂ is selected from H or halogen; preferably, R₂ is selected from H or F;
      R₃ is selected from the following: CH₂R₄, wherein R₄ is selected from OH; 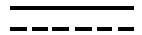 is a single bond or a double bond, preferably a double bond.
Embodiment 8: The compound according to embodiments 1 or 2, wherein
   A, B, R₁, R₂, and R₃ are selected independently of each other, and:
   A is selected from substituted or unsubstituted phenyl and substituted or unsubstituted pyridyl, wherein the substituents selected from are one or more, or one, of the following: halogen, preferably F or Cl;
   Preferably, A is selected from: 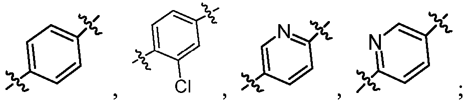
   B is selected from CH₂O, CH₂S, CH₂NH;
   R₁ is selected from halogen; preferably, R₁ is selected from F or Cl;
   R₂ is selected from H or halogen; preferably, R₂ is selected from H or F;
   R₃ is selected from the following: CH₂R₄ wherein R₄ is selected from OH; SCH₂R₆ or OCH₂R₆ wherein R₆ is selected from halogen or CN;
   Preferably, R₃ is selected from the following: CH₂R₄ wherein R₄ is selected from OH; SCH₂R₆ or OCH₂R₆ wherein R₆ is selected from F or CN; 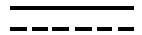 is a single bond or a double bond, preferably a double bond.
Embodiment 9: The compound according to any one of Embodiments 1, 2, and 8, wherein, the compound represented by formula I is selected from the following structural formulas I-1 or I-2, and the compound represented by formula I' is selected from the following structural formulas I'-1 or 1'-2: 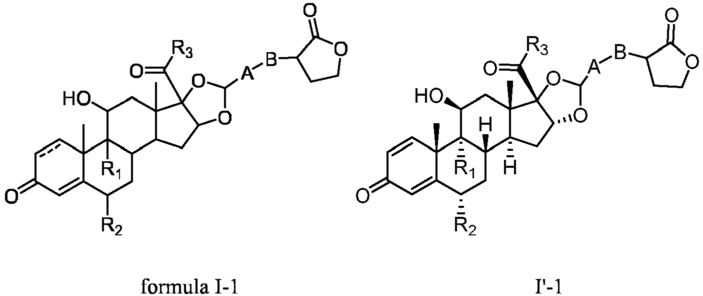 Wherein A, B, R₁, R₂, and R₃ as shown in the structural formulas of the compounds represented by formulas I-1 and I'-1 are selected independently of each other, and:
   A is selected from substituted or unsubstituted phenyl and substituted or unsubstituted pyridyl, wherein the substituents are selected from one or more, or one, of the following: halogen, preferably F or Cl;
   Preferably, A is selected from: 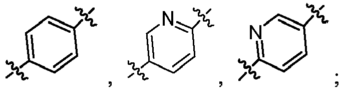
   B is selected from CH₂O, CH₂S;
   R₁ is selected from halogen; preferably, R₁ is selected from F or Cl;
   R₂ is selected from H or halogen; preferably, R₂ is selected from H or F;
   R₃ is selected from the following: CH₂R₄ wherein R₄ is selected from OH; SCH₂R₆ or OCH₂R₆ wherein R₆ is selected from halogen or CN;
   Preferably, R₃ is selected from the following: CH₂R₄ wherein R₄ is selected from OH; SCH₂R₆ or OCH₂R₆ wherein R₆ is selected from F or CN; 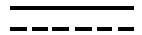 is a single bond or a double bond, preferably a double bond; 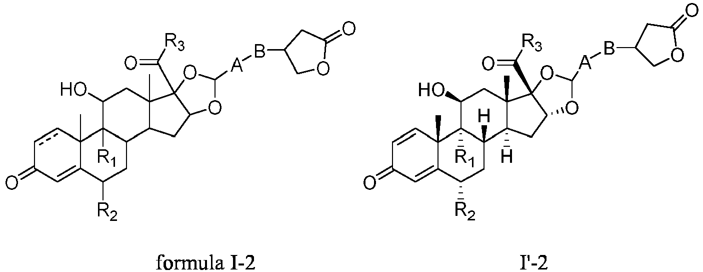 Wherein A, B, R₁, R₂, and R₃ as shown in the structural formulas of the compounds represented by formulas I-1 and I'-1 are selected independently of each other, and:
      A is selected from substituted or unsubstituted phenyl, wherein the substituents are selected from one or more, or one, of the following: halogen, preferably Cl;
      Preferably, A is selected from: 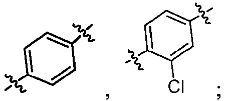
      B is selected from CH₂S, CH₂NH;
      R₁ is selected from halogen; preferably, R₁ is selected from F or Cl;
      R₂ is selected from H;
      R₃ is selected from the following: CH₂R₄, wherein R₄ is selected from OH; 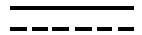 is a single bond or a double bond, preferably a double bond.
Embodiment 10: The compound according to any one of Embodiments 1 to 3, wherein the compound represented by formula I is selected from compounds 1 to 60;
   Preferably compound 5, compound 7, compound 8, compound 9, compound 10, compound 11, compound 12, compound 13, compound 14, compound 15, compound 16, compound 17, compound 18, compound 19, compound 20, compound 21, compound 22, compound 23, compound 24, compound 25, compound 26, compound 27, compound 28, compound 29, compound 30, compound 32, compound 33, compound 34, compound 35, compound 36, compound 37 compound 38, compound 39, compound 41, compound 43, compound 45, compound 46, compound 47, compound 48, compound 49, compound 50, compound 51, compound 52, compound 54, compound 55, compound 56, compound 57, compound 58, compound 59, compound 60;
   Further preferably compound 7, compound 8, compound 10, compound 11, compound 16, compound 17, compound 18, compound 19, compound 20, compound 21, compound 22, compound 23, compound 24, compound 25, compound 26, compound 27, compound 34, compound 35, compound 36, compound 37, compound 38, compound 45, compound 46, compound 47, compound 48, compound 49, compound 50, compound 51, compound 57;
   Even more preferably compound 16, compound 17, compound 20, compound 21, compound 22, compound 23, compound 26, compound 34, compound 35, compound 36, compound 37, compound 46, compound 47, compound 48, compound 51.
   In a second aspect, the provided is Embodiment 12: A method for preparing the compound according to any one of embodiments 1 to 11, wherein the preparation is implemented by Method I or Method II:
      Method I:
         A compound represented by formula II is reacted with a compound represented by formula III under acidic conditions to obtain a compound represented by formula I: 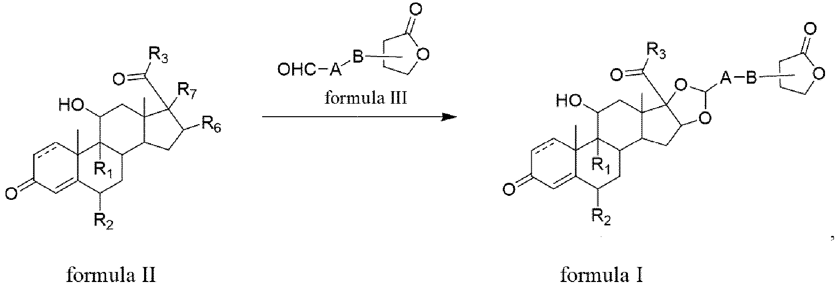
         Wherein A, B, R₁, R₂, and R₃ in the compound represented by formula I are as defined in any one of embodiments 1 to 11;
         R₁, R₂, and R₃ in the compound represented by formula II are as defined in the compound represented by formula I, R₇ and R₈ are each independently selected from OH, or R₇ and R₈
         together form 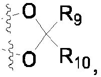 i.e., the positions C₁₆ and C₁₇ are linked via an oxygen bridge, and R₉ and R₁₀ are each independently selected from H or alkyl having 1 to 6 carbon atoms;
         A and B in the compound represented by formula III are as defined in the compound represented by formula I;
            or,
         reacting a compound represented by formula II' with a compound represented by formula III under acidic conditions to obtain a compound represented by formula I': 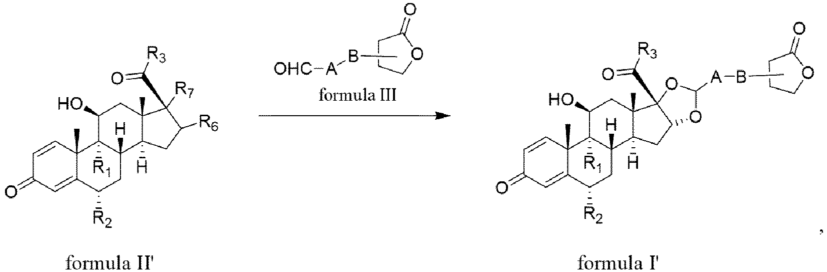
            wherein A, B, R₁, R₂, and R₃ in the compound represented by formula I' are as defined in any one of Embodiments 1 to 11;
            R₁, R₂, and R₃ in the compound represented by formula II' are as defined in the compound represented by formula I', R₇ and R₈ are each selected from OH, or R₇ and R₈ together form 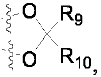 i.e., the positions C₁₆ and C₁₇ are linked via an oxygen bridge, and R₉ and R₁₀ are each independently selected from H or alkyl having 1 to 6 carbon atoms;
            A and B in the compound represented by formula III are as defined in the compound represented by formula I';
      alternatively, Method II: to obtain a compound represented by formula I, wherein B is selected from CH₂NH, the following processes are used:
         (a)reacting a compound represented by formula II with a compound represented by formula V under acidic conditions to obtain a compound represented by formula IV: 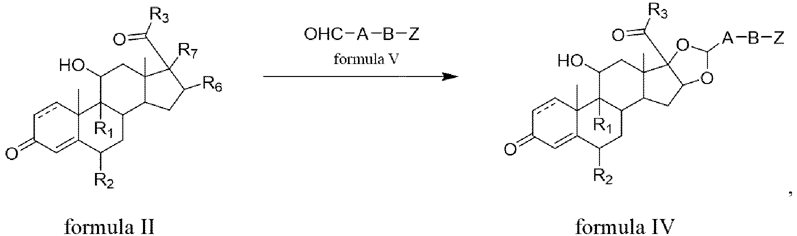
         (b) reacting the compound represented by formula IV with a compound represented by formula VI or a compound represented by formula VII to obtain a compound represented by formula I: 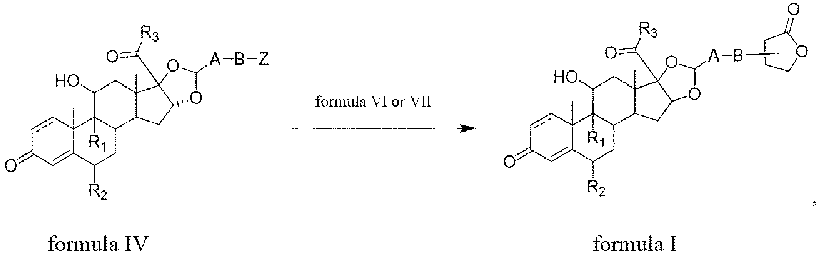
            wherein A, R₁, R₂, and R₃ in the compound represented by formula I are as defined in any one of Embodiments 1 to 11, and B is selected from CH₂NH;
            R₁, R₂, and R₃ in the compound represented by formula II are as defined in the compound represented by formula I, R₇ and R₈ are each selected from OH, or R₇ and R₈ together form 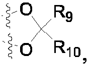 i.e., the positions C₁₆ and C₁₇ are linked via an oxygen bridge, and R₉ and R₁₀ are each independently selected from H or alkyl having 1 to 6 carbon atoms;
            A, B, R₁, R₂, and R₃ in the compound represented by formula IV are as defined in the compound represented by formula I;
            A in the compound represented by formula V is as defined in the compound represented by formula I, B is selected from CH₂NH, and Z is selected from a Boc protecting group;
            The compound represented by formula VI is selected from 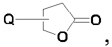 wherein Q is a leaving group selected from Cl or Br;
            The formula VII is selected from 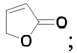 or,
               (a) reacting a compound represented by formula II' with a compound represented by formula V under acidic conditions to obtain a compound represented by formula IV': 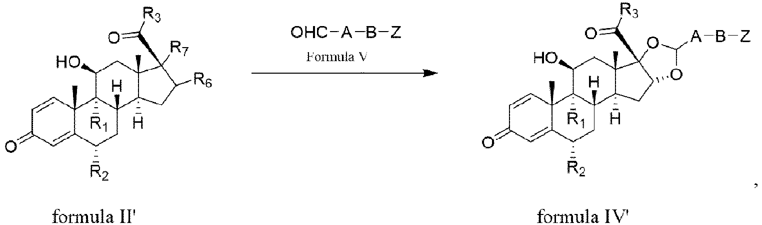
               (b) reacting the compound represented by formula IV' with a compound represented by formula VI or a compound represented by formula VII to obtain a compound represented by formula I': 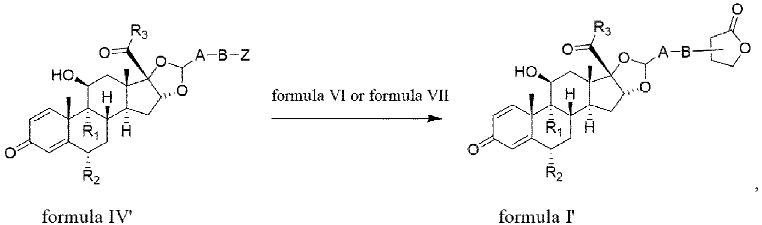
                  wherein A, R₁, R₂, and R₃ in the compound represented by formula I' are as defined in any one of Embodiments 1 to 11, and B is selected from CH₂NH;
                  R₁, R₂, and R₃ in the compound represented by formula II' are as defined in the compound represented by formula I', R₇ and R₈ are each selected from OH, or R₇ and R₈ together form 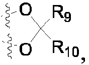 i.e., the positions C₁₆ and C₁₇ are linked via an oxygen bridge, and R₉ and R₁₀ are each independently selected from H or alkyl having 1 to 6 carbons;
                  A, B, R₁, R₂, and R₃ in the compound represented by formula IV' are as defined in the compound represented by formula I';
                  A in the compound represented by formula V is as defined in the compound represented by formula I', B is selected from CH₂NH, and Z is selected from a Boc protecting group;
                  The compound represented by formula VI is selected from 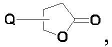 wherein Q is a leaving group selected from Cl or Br;
                  The formula VII is selected from 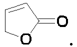
                  In a third aspect, provided is Embodiment 13: A pharmaceutical composition comprising the compound according to any one of Embodiments 1 to 11 or a compound prepared by the method according to Embodiment 12.
Embodiment 14: The pharmaceutical composition according to Embodiment 13, wherein the pharmaceutical composition is in a dosage form selected from creams, ointments, gels, transdermal patches, intradermal injections, eye drops, intraocular injections, ophthalmic implants, nasal sprays, inhalation powders, inhalation aerosols, inhalation sprays, inhalation liquid preparations, vaginal suppositories, vaginal tablets, vaginal gels, and preparations for oral or rectal administration for local gastrointestinal action.
Embodiment 15: Use of the compound according to any one of Embodiments 1 to 11, a compound prepared by the method according to Embodiment 12, or the pharmaceutical composition according to Embodiments 13 or 14, in the manufacture of medicaments for the treatment of glucocorticoid receptor-mediated diseases;
   Preferably, the diseases are selected from blepharitis, conjunctivitis, keratitis, iritis, iridocyclitis, uveitis, dry eye syndrome, diabetic retinopathy, wet age-related macular degeneration, choroidal neovascularisation, posterior uveitis, cataracts, glaucoma, retinal detachment, post-strabismus surgery inflammation, eczema, psoriasis, atopic dermatitis, allergic dermatitis, pruritus, hypersensitivity, rheumatoid arthritis, multiple sclerosis and lupus erythematosus disseminated, rhinitis, sinusitis, asthma, nasal polyps, asthma, chronic obstructive pulmonary disease, inflammatory bowel disease, Crohn's disease, ulcerative colitis, or chronic glomerulonephritis;
   Further preferably, the diseases are selected from conjunctivitis, keratitis, uveitis, dry eye syndrome, asthma, chronic obstructive pulmonary disease, allergic rhinitis, nasal polyps, Crohn's disease, eczema, and psoriasis.
Embodiment 16: A method for treating glucocorticoid receptor-mediated diseases, including administering to a subject in need thereof the compound according to any one of Embodiments 1 to 11, a compound prepared by the method according to Embodiment 12, or the pharmaceutical composition according to Embodiments 13 or 14;
   Preferably, the diseases are selected from blepharitis, conjunctivitis, keratitis, iritis, iridocyclitis, uveitis, dry eye syndrome, diabetic retinopathy, wet age-related macular degeneration, choroidal neovascularisation, posterior uveitis, cataracts, glaucoma, retinal detachment, post-strabismus surgery inflammation, eczema, psoriasis, atopic dermatitis, allergic dermatitis, pruritus, hypersensitivity, rheumatoid arthritis, multiple sclerosis and lupus erythematosus disseminated, rhinitis, sinusitis, asthma, nasal polyps, asthma, chronic obstructive pulmonary disease, inflammatory bowel disease, Crohn's disease, ulcerative colitis, or chronic glomerulonephritis;
   Further preferably, the diseases are selected from conjunctivitis, keratitis, uveitis, dry eye syndrome, asthma, chronic obstructive pulmonary disease, allergic rhinitis, nasal polyps, Crohn's disease, eczema, and psoriasis.
Embodiment 17: The compound according to any one of Embodiments 1 to 11, a compound prepared by the method according to Embodiment 12, or the pharmaceutical composition according to Embodiments 13 or 14, for use in the treatment of glucocorticoid receptor-mediated diseases;
   Preferably, the diseases are selected from blepharitis, conjunctivitis, keratitis, iritis, iridocyclitis, uveitis, dry eye syndrome, diabetic retinopathy, wet age-related macular degeneration, choroidal neovascularisation, posterior uveitis, cataracts, glaucoma, retinal detachment, post-strabismus surgery inflammation, eczema, psoriasis, atopic dermatitis, allergic dermatitis, pruritus, hypersensitivity, rheumatoid arthritis, multiple sclerosis and lupus erythematosus disseminated, rhinitis, sinusitis, asthma, nasal polyps, asthma, chronic obstructive pulmonary disease, inflammatory bowel disease, Crohn's disease, ulcerative colitis, or chronic glomerulonephritis;
   Further preferably, the diseases are selected from conjunctivitis, keratitis, uveitis, dry eye syndrome, asthma, chronic obstructive pulmonary disease, allergic rhinitis, nasal polyps, Crohn's disease, eczema, and psoriasis.

In addition, the present application further relates to the following embodiments:
As a first aspect of the present disclosure, provided is a compound represented by formula I, having the following structural formula: 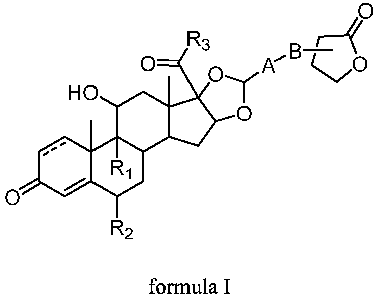
an optical isomer thereof, a pharmaceutically acceptable salt thereof, or a solvate thereof,
wherein A, B, R₁, R₂, and R₃ as shown in the structural formula of the compound represented by formula I are selected independently of each other, and:
   A is selected from substituted or unsubstituted aryl having 6 to 10 carbons, substituted or unsubstituted heteroaryl or heterocyclyl having 4 to 10 carbons, and substituted or unsubstituted cycloalkyl having 3 to 10 carbons, wherein the substituents are selected from halogen, hydroxyl, carbonyl, amino, cyano, carboxyl, aryl having 6 to 10 carbons, heteroaryl having 4 to 10 carbons, cycloalkyl having 3 to 6 carbons, alkoxy having 1 to 10 carbons, alkylamino having 1 to 10 carbons, or alkyl ester having 1 to 10 carbons;
   B is selected from absent, O, S, NH, CH₂, OCH₂, SCH₂, NHCH₂, CH₂O, CH₂S, CH₂SO, CH₂SO₂, CH₂NH, C=O, or NH(CO);
   R₁ is selected from H or halogen;
   R₂ is selected from H, CH₃, or halogen;
   R₃ is selected from CH₂R₄, wherein R₄ is selected from OH, halogen, OR₅, or OCOR₅, or R₃ is selected from SCH₂R₆ or OCH₂R₆;
   wherein R₅ is selected from alkyl having 1 to 6 carbons, alkenyl having 2 to 6 carbons, alkynyl having 2 to 6 carbons, aryl having 6 to 10 carbons, heteroaryl or heterocyclyl having 4 to 10 carbons; R₆ is selected from halogen or CN;
   the halogen is selected from F or Cl; 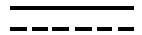 is a single bond or a double bond.

It should be noted that the B group in the structural formula of the compound represented by formula I is attached to the α, β, or γ carbon atom of the lactone ring.

Further, A is selected from substituted or unsubstituted phenyl, substituted or unsubstituted piperidinyl, substituted or unsubstituted cyclohexyl, substituted or unsubstituted pyridinyl, and substituted or unsubstituted pyrimidinyl, wherein the substituents are selected from halogen or cyano.

Further, A is selected from substituted or unsubstituted phenyl, substituted or unsubstituted cyclohexyl, substituted or unsubstituted pyridyl, and substituted or unsubstituted pyrimidyl, wherein the substituents are selected from halogen or cyano.

Further, A is selected from substituted or unsubstituted phenyl, substituted or unsubstituted cyclohexyl, and substituted or unsubstituted pyridyl, wherein the substituents are selected from halogen.

Further, A is selected from substituted or unsubstituted phenyl and substituted or unsubstituted pyridyl, wherein the substituents are selected from halogen.

Further, B is selected from O, S, NH, CH₂, OCH₂, SCH₂, NHCH₂, CH₂O, CH₂S, CH₂SO, CH₂SO₂, CH₂NH, C=O, or NH(CO).

Further, B is selected from S, CH₂, OCH₂, SCH₂, NHCH₂, CH₂O, CH₂S, CH₂SO, CH₂SO₂, CH₂NH, or NH(CO).

Further, B is selected from S, CH₂O, CH₂S, CH₂SO, or CH₂NH.

Further, B is selected from CH₂O, CH₂S, or CH₂NH.

Further, B is selected from CH₂O or CH₂S.

Further, R₁ is selected from H or halogen, and the halogen is selected from F or Cl.

Further, R₁ is selected from halogen, and the halogen is selected from F or Cl.

Further, R₂ is selected from H or halogen, and the halogen is selected from F or Cl.

Further, R₃ is selected from CH₂R₄, wherein R₄ is selected from OH or halogen, or R₃ is selected from SCH₂R₆ or OCH₂R₆, wherein R₆ is halogen or CN, and the halogen is selected from F or Cl.

Further, R₃ is selected from CH₂R₄, wherein R₄ is selected from OH, or R₃ is selected from SCH₂R₆ or OCH₂R₆, wherein R₆ is halogen or CN, and the halogen is selected from F or Cl.

Further, 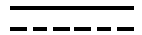 is a double bond.

Further, A, B, R₁, R₂, and R₃ as shown in the structural formula of the compound represented by formula I are selected independently of each other, and:
A is selected from substituted or unsubstituted phenyl, substituted or unsubstituted piperidinyl, substituted or unsubstituted cyclohexyl, substituted or unsubstituted pyridinyl, and substituted or unsubstituted pyrimidinyl, wherein the substituents are selected from halogen or cyano;
B is selected from O, S, NH, CH₂, OCH₂, SCH₂, NHCH₂, CH₂O, CH₂S, CH₂SO, CH₂SO₂, CH₂NH, C=O, or NH(CO);
R₁ is selected from H or halogen;
R₂ is selected from H or halogen;
R₃ is selected from CH₂R₄, wherein R₄ is selected from OH or halogen, or R₃ is selected from SCH₂R₆ or OCH₂R₆, wherein R₆ is selected from halogen or CN;
the halogen is selected from F or Cl; 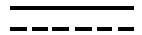 is a double bond.

Further, A, B, R₁, R₂, and R₃ as shown in the structural formula of the compound represented by formula I are selected independently of each other, and:
A is selected from substituted or unsubstituted phenyl, substituted or unsubstituted cyclohexyl, substituted or unsubstituted pyridyl, and substituted or unsubstituted pyrimidinyl, wherein the substituents are selected from halogen or cyano;
B is selected from S, CH₂, OCH₂, SCH₂, NHCH₂, CH₂O, CH₂S, CH₂SO, CH₂SO₂, CH₂NH, or NH(CO);
R₁ is selected from H or halogen;
R₂ is selected from H or halogen;
R₃ is selected from CH₂R₄, wherein R₄ is selected from OH or halogen, or R₃ is selected from SCH₂R₆ or OCH₂R₆, wherein R₆ is selected from halogen or CN;
the halogen is selected from F or Cl; 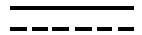 is a double bond.

Further, A, B, R₁, R₂, and R₃ as shown in the structural formula of the compound represented by formula I are selected independently of each other, and:
A is selected from substituted or unsubstituted phenyl, substituted or unsubstituted cyclohexyl, and substituted or unsubstituted pyridyl, wherein the substituents are selected from halogen;
B is selected from S, CH₂O, CH₂S, CH₂SO, or CH₂NH;
R₁ is selected from halogen;
R₂ is selected from H or halogen;
R₃ is selected from CH₂R₄, wherein R₄ is selected from OH or halogen, or R₃ is selected from SCH₂R₆ or OCH₂R₆, wherein R₆ is selected from halogen or CN;
the halogen is selected from F or Cl; 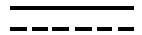 is a double bond.

Further, A, B, R₁, R₂, and R₃ as shown in the structural formula of the compound represented by formula I are selected independently of each other, and:
A is selected from substituted or unsubstituted phenyl and substituted or unsubstituted pyridyl, wherein the substituents are selected from halogen;
B is selected from CH₂O, CH₂S, or CH₂NH;
R₁ is selected from halogen;
R₂ is selected from H or halogen;
R₃ is selected from CH₂R₄, wherein R₄ is selected from OH, or R₃ is selected from SCH₂R₆ or OCH₂R₆, wherein R₆ is selected from halogen or CN;
the halogen is selected from F or Cl; 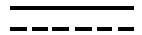 is a double bond.

Further, the compound represented by formula I is selected from the following compounds:

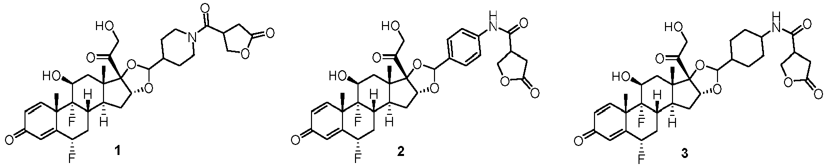

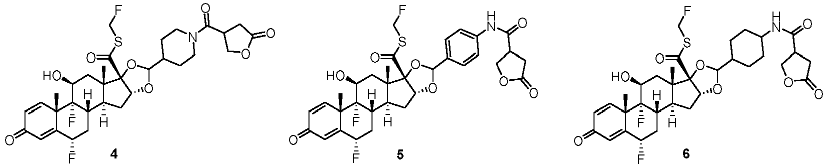

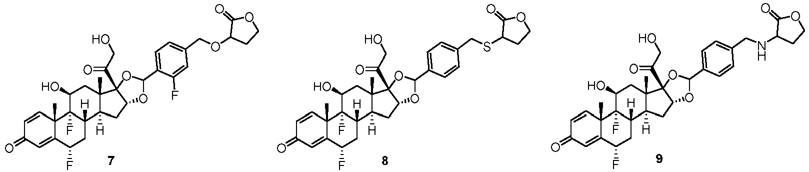

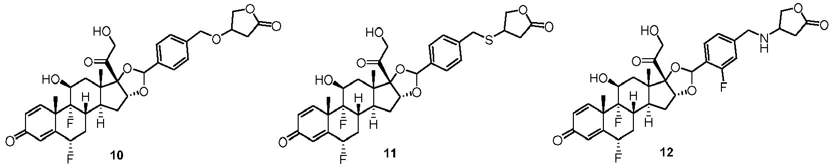

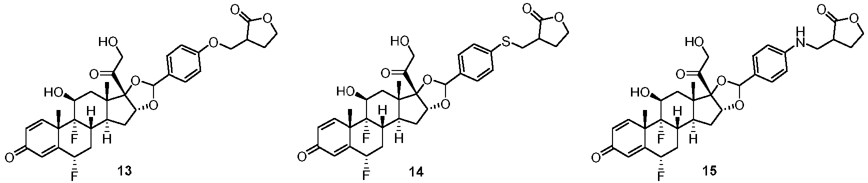

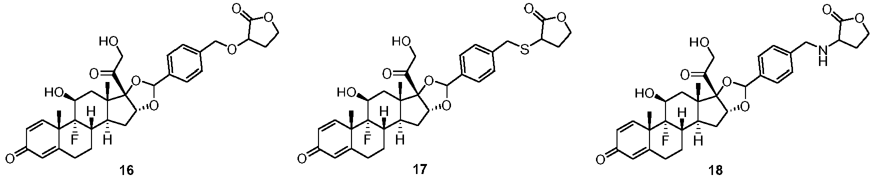

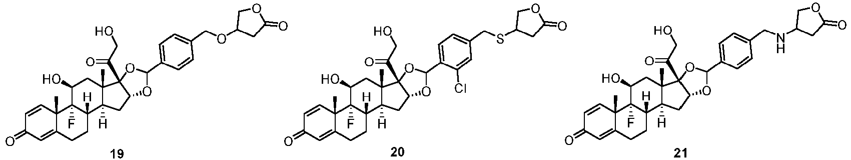

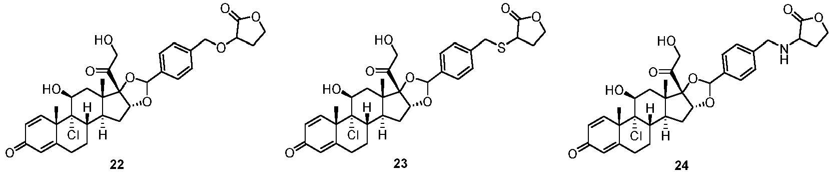

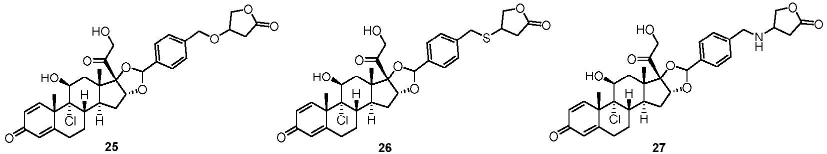

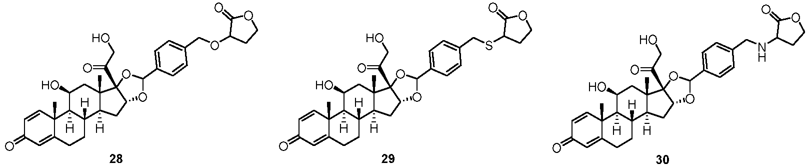

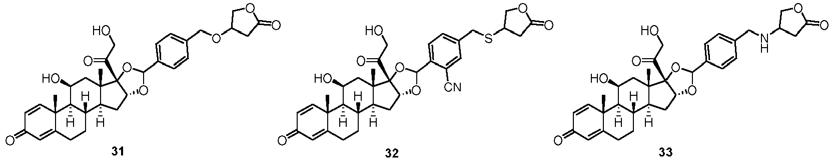

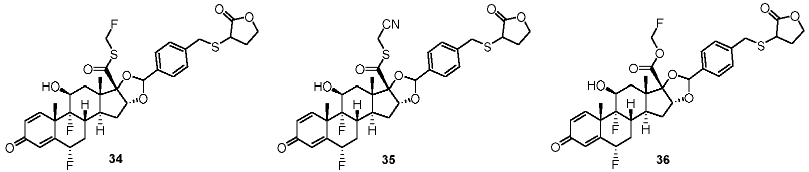

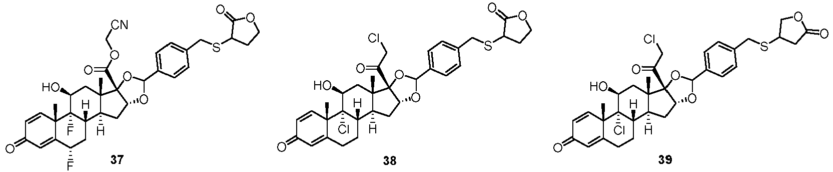

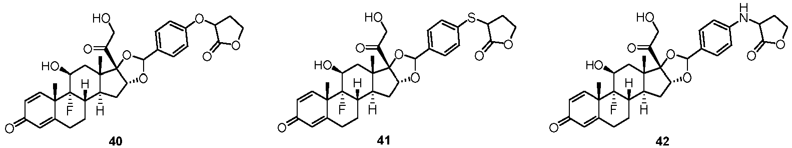

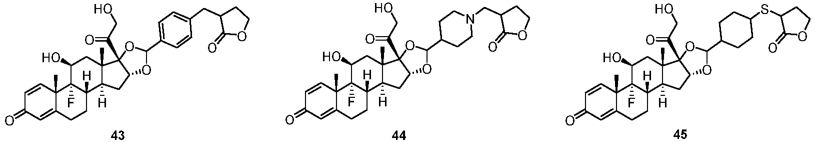

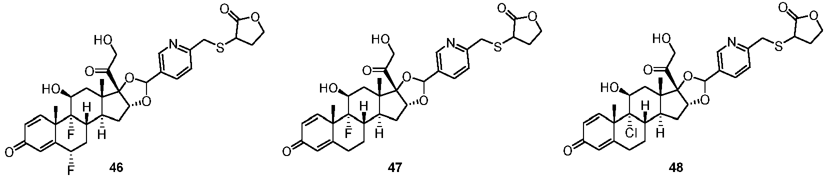

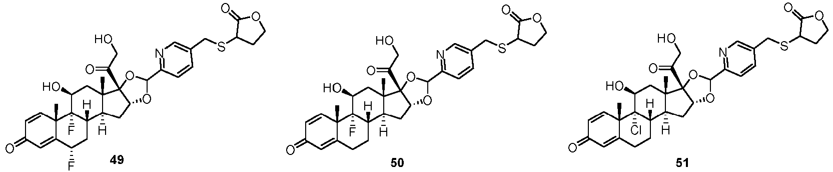

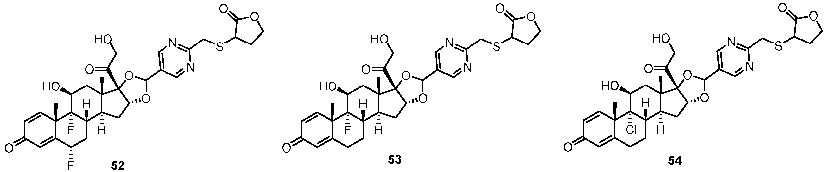

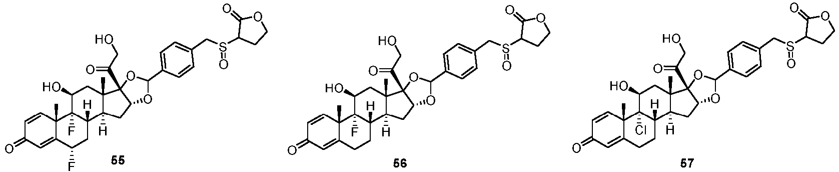

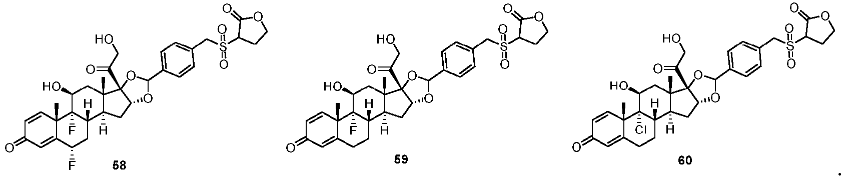

Further, the compound represented by formula I is selected from the following compounds: compound 5, compound 7, compound 8, compound 9, compound 10, compound 11, compound 12, compound 13, compound 14, compound 15, compound 16, compound 17, compound 18, compound 19, compound 20, compound 21, compound 22, compound 23, compound 24, compound 25, compound 26, compound 27, compound 28, compound 29, compound 30, compound 32, compound 33, compound 34, compound 35, compound 36, compound 37, compound 38, compound 39, compound 41, compound 43, compound 45, compound 46, compound 47, compound 48, compound 49, compound 50, compound 51, compound 52, compound 54, compound 55, compound 56, compound 57, compound 58, compound 59, compound 60.

Further, the compound represented by formula I is selected from the following compounds: compound 7, compound 8, compound 10, compound 11, compound 16, compound 17, compound 18, compound 19, compound 20, compound 21, compound 22, compound 23, compound 24, compound 25, compound 26, compound 27, compound 34, compound 35, compound 36, compound 37, compound 38, compound 45, compound 46, compound 47, compound 48, compound 49, compound 50, compound 51, compound 57.

Further, the compound represented by formula I is selected from the following compounds: compound 16, compound 17, compound 20, compound 21, compound 22, compound 23, compound 26, compound 34, compound 35, compound 36, compound 37, compound 46, compound 47, compound 48, compound 51.

Further, the pharmaceutically acceptable salt of the compound represented by formula I is a conventional non-toxic salt formed, for example, from inorganic or organic acids, including, but not limited to, acetate, adipate, alginate, aspartate, benzoate, besylate, bisulfate, butyrate, citrate, camphorate, camphorsulfonate, cyclopentanepropionate, digluconate, dodecyl sulfate, ethanesulfonate, fumarate, glucoheptonate, glycerophosphate, hemisulfate, heptanoate, hexanoate, hydrochloride, hydrobromide, hydroiodide, 2-hydroxyethanesulfonate, lactate, maleate, methanesulfonate, 2-naphthalene-sulfonate, nicotinate, oxalate, palmoate, pectinate, persulfate, 3-phenylpropionate, picrate, pivalate, propionate, succinate, tartrate, thiocyanate, tosylate, or undecanoate.

As a second aspect of the present disclosure, provided is a method for preparing the compound as described above, wherein the preparation is implemented by Method I or Method II:
Method I:
   reacting a compound represented by formula II with a compound represented by formula III under acidic conditions to obtain a compound represented by formula I: 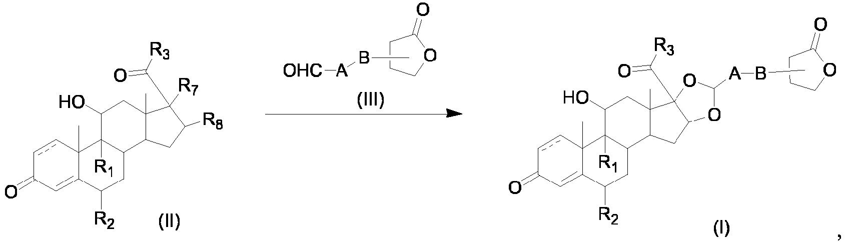
   wherein A, B, R₁, R₂, and R₃ in the compound represented by formula I are as defined in the first aspect above;
   R₁, R₂, and R₃ in the compound represented by formula II are as defined in the compound represented by formula I, R₇ and R₈ are each selected from OH, or R₇ and R₈ together form 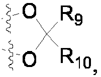 i.e., the positions C₁₆ and C₁₇ are linked via an oxygen bridge, and R₉ and R₁₀ are each independently selected from H or alkyl having 1 to 6 carbon atoms;
   A and B in the compound represented by formula III are as defined in the compound represented by formula I.
   It should be noted that the compound of formula III represents an aldehyde containing an A moiety and a B moiety.
Alternatively, Method II: to obtain a compound represented by formula I wherein B is selected from CH₂NH, the following processes are used:
   (a) reacting a compound represented by formula II with a compound represented by formula V under acidic conditions to obtain a compound represented by formula IV: 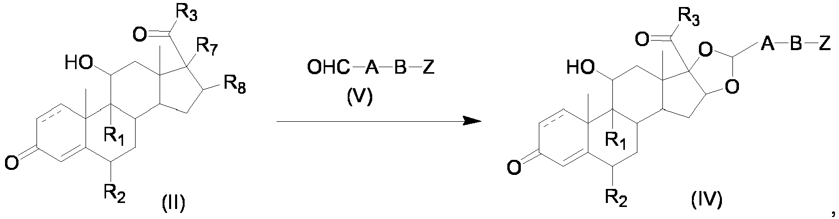
   (b) reacting the compound represented by formula IV with a compound represented by formula VI or a compound represented by formula VII to obtain a compound represented by formula I: 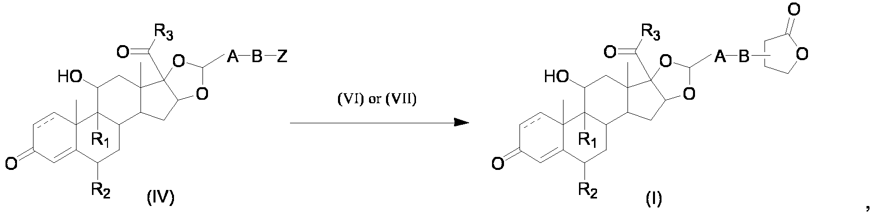
      wherein A, R₁, R₂, and R₃ in the compound represented by formula I are as defined in the first aspect above, and B is selected from CH₂NH;
      R₁, R₂, and R₃ in the compound represented by formula II are as defined in the compound represented by formula I, R₇ and R₈ are each selected from OH, or R₇ and R₈ together form 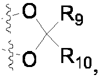 i.e., the positions C₁₆ and C₁₇ are linked via an oxygen bridge, and R₉ and R₁₀ are each independently selected from H or alkyl having 1 to 6 carbon atoms; A, B, R₁, R₂, and R₃ in the compound represented by formula IV are as defined in the compound represented by formula I;
      A in the compound represented by formula V is as defined in the compound represented by formula I, B is selected from CH₂NH, and Z is selected from a Boc protecting group;
      the compound represented by formula VI is selected from 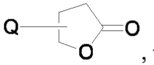 wherein Q is a leaving group selected from Cl or Br;
      the formula VII is selected from

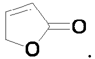

As a third aspect of the present disclosure, provided is a pharmaceutical composition comprising the compound as described above or a compound prepared by the method as described above.

Further, the composition of the present disclosure is in a dosage form including, but not limited to, tablets, pills, granules, powders, lozenges, suspensions, emulsions, solutions, syrups, aerosols, ointments, creams, gels, lotions, soft and hard gelatin capsules, suppositories, eye drops, transdermal patches, injections, implants, sprays, inhalants, and preparations for oral or rectal administration for local gastrointestinal action.

Further, the pharmaceutical composition is in a dosage form selected from creams, ointments, gels, transdermal patches, intradermal injections, eye drops, intraocular injections, ophthalmic implants, nasal sprays, inhalation powders, inhalation aerosols, inhalation sprays, inhalation liquid preparations, vaginal suppositories, vaginal tablets, vaginal gels, and preparations for oral or rectal administration for local gastrointestinal action.

The composition of the present disclosure may optionally comprise other pharmaceutically active substances (which may or may not synergize with the compound of the present disclosure) and suitable carriers, excipients, and diluents, such as lubricants, wetting agents, emulsifiers and suspending agents, dispersants, disintegrants, bulking agents, fillers, preservatives, sweeteners, flavoring agents, flow conditioners, release agents, and the like. Examples include lactose, glucose, sucrose, sorbitol, mannitol, starch, gum acacia, calcium phosphate, alginate, tragacanth, gelatin, calcium silicate, microcrystalline cellulose, polyvinylpyrrolidone, polyethylene glycol, cellulose, (sterile) water, methylcellulose, talc, magnesium stearate, edible oils, vegetable oils, mineral oils, or suitable mixtures thereof. Additionally, the release of the active compound can be modulated by the addition of other substances, such as liposomes or hydrophilic polymer matrices based on natural gels or synthetic polymers. To enhance the solubility and/or the stability of the compound, α-, β-, or γ-cyclodextrin or their derivatives may be used. Co-solvents such as alcohols may also be used to improve the solubility and/or stability of the compound in the preparation of an aqueous composition.

For the treatment of skin diseases, the compound of the present disclosure may be used locally. For example, sprays, creams, ointments, gels, transdermal patches, or other forms of the compound suitable for topical, transdermal, and/or intradermal administration may be beneficial.

For the treatment of ophthalmic diseases, the compound of the present disclosure may be in the form of eye drops, gels, or implants, and a normal saline solution or excipient is typically used as the main vehicle. Ophthalmic formulations are preferably prepared using a suitable buffer system at a comfortable pH.

Further, the administration routes of the pharmaceutical composition include, but are not limited to, topical dermal administration, local ophthalmic administration, otic administration, nasal administration, inhalation administration, intravaginal administration, and oral or rectal administration for local gastrointestinal action.

The dosage forms as described above may be solid, semi-solid, or liquid depending on the administration routes, and the methods, carriers, diluents, and excipients used in their preparation are well-known to those skilled in the art.

For oral administration, the composition of the present disclosure may be admixed with suitable additives, such as excipients, stabilizers, or inert carriers, and converted into suitable administration forms by conventional methods, such as tablets, capsules, aqueous solutions, or alcoholic solutions. Suitable inert carriers may be gum acacia, magnesium oxide, magnesium carbonate, potassium phosphate, lactose, glucose, or starch, particularly corn starch. Suitable excipients or solvents may be vegetable or animal oils, such as sunflower oil or cod liver oil. Suitable solvents for aqueous or alcoholic solutions are water, ethanol, sugar solutions, or mixtures thereof.

When administered by nasal spray or inhalation, the composition of the present disclosure may be prepared according to techniques known in the art. For example, liquid preparations may use benzyl alcohol or other suitable preservatives and absorption promoters to enhance bioavailability, and may also use fluorocarbons known in the art and/or other solubilizers or dispersants. Suitable pharmaceutical formulations for administration in the form of aerosols or sprays may be solutions, suspensions, or emulsions. Solvents used include ethanol, water, or mixtures thereof. If desired, other adjuvants, such as surfactants, emulsifiers, stabilizers, and propellants, may also be included.

For subcutaneous injection, the composition of the present disclosure may be formulated into solutions, suspensions, or emulsions using conventional substances (such as solubilizers, emulsifiers, or other adjuvants). Suitable solvents are, for example, water, normal saline solutions, or alcoholic solvents (e.g., ethanol, propanol, or glycerol), as well as sugar solutions such as glucose solutions or mannitol solutions, or mixtures of the various solvents mentioned above. Such dosage forms may be prepared according to techniques known in the art, for example, using suitable non-toxic, parenterally acceptable diluents or solvents, such as mannitol, 1,3-butanediol, water, Ringer's solution, or isotonic sodium chloride solution, or using suitable dispersants or wetting agents and suspending agents, such as sterile, non-irritating, and non-volatile oils, including synthetic monoglycerides or diglycerides and fatty acids (e.g., oleic acid).

When administered rectally in the form of suppositories, the composition of the present disclosure may be prepared by mixing the compound of the present disclosure with suitable non-irritating excipients, such as cocoa butter, synthetic glycerides, or polyethylene glycols, which are solid at ordinary temperatures but will liquefy and/or dissolve in the rectal lumen to release the drug.

As a fourth aspect of the present disclosure, provided is the use of the compound as described above, a compound prepared by the method as described above, or the pharmaceutical composition as described above, in the manufacture of medicaments for the treatment of glucocorticoid receptor-mediated diseases.

Preferably, the diseases are selected from blepharitis, conjunctivitis, keratitis, iritis, iridocyclitis, uveitis, dry eye syndrome, diabetic retinopathy, wet age-related macular degeneration, choroidal neovascularisation, posterior uveitis, cataracts, glaucoma, retinal detachment, post-strabismus surgery inflammation, eczema, psoriasis, atopic dermatitis, allergic dermatitis, pruritus, hypersensitivity, rheumatoid arthritis, multiple sclerosis and lupus erythematosus disseminated, rhinitis, sinusitis, asthma, nasal polyps, asthma, chronic obstructive pulmonary disease, inflammatory bowel disease, Crohn's disease, ulcerative colitis, or chronic glomerulonephritis;
further preferably, the diseases are selected from conjunctivitis, keratitis, uveitis, dry eye syndrome, asthma, chronic obstructive pulmonary disease, allergic rhinitis, nasal polyps, Crohn's disease, eczema, and psoriasis.

Further, the diseases include, but are not limited to, skin diseases, respiratory diseases, inflammatory bowel diseases, autoimmune diseases, ocular diseases, or renal diseases.

Further, the skin diseases include, but are not limited to, eczema, psoriasis, atopic dermatitis, allergic dermatitis, pruritus, and hypersensitivity.

Further, the respiratory diseases include, but are not limited to, rhinitis, sinusitis, asthma, nasal polyps, and chronic obstructive pulmonary disease;
more specifically, rhinitis of any type, etiology, or pathogenesis, particularly rhinitis selected from the group consisting of: seasonal allergic rhinitis or perennial allergic rhinitis;
sinusitis of any type, etiology, or pathogenesis, particularly sinusitis selected from the group consisting of: suppurative or non-suppurative sinusitis, acute or chronic sinusitis, and ethmoidal sinusitis, frontal sinusitis, maxillary sinusitis, or sphenoid sinusitis;
asthma of any type, etiology, or pathogenesis, particularly asthma selected from the group consisting of: atopic asthma, non-atopic asthma, allergic asthma, atopic bronchial IgE-mediated asthma, bronchial asthma, intrinsic asthma caused by pathophysiological disorders, extrinsic asthma caused by environmental factors, idiopathic asthma of unknown or cryptogenic cause, exercise-induced asthma, allergen-induced asthma, cold air-induced asthma, occupational asthma, infectious asthma caused by bacterial, fungal, protozoal, or viral infections, non-allergic asthma, initial-stage asthma, wheezy infant syndrome, and bronchitis;
obstructive or inflammatory airway diseases of any type, etiology, or pathogenesis, particularly obstructive or inflammatory airway diseases selected from the group consisting of: chronic eosinophilic pneumonia, chronic obstructive pulmonary disease (COPD), COPD including chronic bronchitis, emphysema, and dyspnea associated or not with COPD, COPD characterized by irreversible progressive airway obstruction, adult respiratory distress syndrome (ARDS), exacerbation of airway hyperresponsiveness following other drug therapies, and airway diseases associated with pulmonary hypertension.

Further, the inflammatory bowel diseases include, but are not limited to, inflammatory bowel disease, Crohn's disease, and ulcerative colitis.

Further, the autoimmune diseases include, but are not limited to, rheumatoid arthritis, multiple sclerosis, and lupus erythematosus disseminatus.

Further, the ocular diseases include, but are not limited to, external ocular diseases such as blepharitis, conjunctivitis, and keratitis; anterior ocular diseases such as iritis, iridocyclitis, and uveitis; posterior ocular diseases such as dry eye syndrome, diabetic retinopathy, wet age-related macular degeneration, choroidal neovascularization, and posterior uveitis; postoperative inflammation following cataract, glaucoma, retinal detachment, and strabismus correction surgery.

Further, the renal diseases include, but are not limited to, chronic glomerulonephritis and the like.

The compound of the present disclosure also has the following uses: cancer (e.g., glioma and prostate cancer), acquired immune deficiency syndrome, osteoarthritis, septic shock, graft rejection, emphysema (especially in patients with COPD), post-ischemic injury, pulmonary hypertension, pulmonary fibrosis, interstitial lung disease, acute respiratory distress syndrome, prevention of restenosis after coronary angioplasty, Stevens-Johnson syndrome, HELLP syndrome (a variant of severe preeclampsia), chronic active hepatitis, blood diseases, and acute spinal cord injury.

Further, the diseases are selected from conjunctivitis, keratitis, uveitis, dry eye syndrome, asthma, COPD, allergic rhinitis, nasal polyps, Crohn's disease, eczema, and psoriasis.

Compared with the prior art, the present disclosure has the following beneficial effects:
The compound provided by the present disclosure is a glucocorticoid based on lactone ring modification, exhibiting a high plasma clearance rate, a short half-life (Cl_obs are all greater than 400 mL/min/kg each, T1/2 are all less than 0.5 h), and reduced systemic exposure. Compared with mineralocorticoid receptor (MR) and progesterone receptor, the compound provided by the present disclosure exhibits enhanced glucocorticoid receptor (GR) selectivity (both MR/GR and PR/GR are more than 1000 times), thereby significantly reducing the adverse effects associated with systemic absorption of the glucocorticoid drug. In the pharmacodynamic experiment on anti-asthma, the administration of the compound of the present disclosure can significantly or extremely significantly decrease the Penh values in mice. Moreover, this can significantly inhibit airway hyperresponsiveness in asthma model mice, suppress inflammatory cytokines in alveolar lavage fluid, and has significant anti-inflammatory activity. The present disclosure has significant anti-inflammatory activity in animals with uveitis, dry eye syndrome, inflammatory bowel disease, and psoriasis. The compound provided by the present disclosure demonstrates good safety, with no abnormal intraocular pressure observed after continuous ocular administrations in guinea pigs and no significant changes in the thickness of the epidermal layer after continuous dermal administrations in rats.

### Detailed Description of the Embodiments

In the present disclosure, unless explicitly indicated otherwise, the expression "selected independently of each other" used throughout herein may mean that the specific options expressed by the same or different symbols in different groups do not affect each other, or that the specific options expressed by the same or different symbols in the same group do not affect each other.

The substituents of the compound of the present disclosure are disclosed according to group types or ranges. It is particularly noted that the present disclosure encompasses every independent sub-combination of each member of such group types and ranges. For example, the term "alkyl having 1 to 6 carbons" specifically refers to methyl, ethyl, C3 alkyl, C4 alkyl, C5 alkyl, and C6 alkyl groups, which are independently disclosed.

Unless otherwise specified, the groups and substituents as described above have common meanings in the field of medicinal chemistry.

The term "alkyl having 1 to 6 carbons" refers to any linear or branched group having 1 to 6 carbon atoms, such as methyl, ethyl, n-propyl, isopropyl, n-butyl, isobutyl, t-butyl, sec-butyl, n-pentyl, t-pentyl, and n-hexyl. Similarly, "C1-C4 alkyl" refers to any linear or branched group having 1 to 4 carbon atoms, such as methyl, ethyl, n-propyl, isopropyl, n-butyl, isobutyl, tert-butyl, and sec-butyl.

As used herein, the term "alkenyl having 2 to 6 carbons" refers to a linear or branched unsaturated hydrocarbon group containing at least one carbon-carbon double bond and having 2 to 6 (e.g., 2, 3, 4, 5, or 6) carbon atoms. Specific examples include, but are not limited to, ethenyl, 1-propenyl, 2-propenyl, 1-butenyl, 2-butenyl, 1,3-butadienyl, 1-pentenyl, 2-pentenyl, 3-pentenyl, 1,3-pentadienyl, 1,4-pentadienyl, 1-hexenyl, 2-hexenyl, 3-hexenyl, and 1,4-hexadienyl.

As used herein, the term "alkynyl having 2 to 6 carbons" refers to a linear or branched unsaturated hydrocarbon group containing at least one triple bond and having 2 to 6 carbon atoms. Specific examples include, but are not limited to, ethynyl, 1-propynyl, 2-propynyl, 1-butynyl, 2-butynyl, 1,3-butadiynyl, 1-pentynyl, 2-pentynyl, 3-pentynyl, 1,3-pentadiynyl, 1,4-pentadiynyl, 1-hexynyl, 2-hexynyl, 3-hexynyl, and 1,4-hexadiynyl.

The term "alkoxy having 1 to 10 carbons" refers to any of the above alkyl groups (e.g., alkyl having 1 to 10 carbons, etc.) attached to the remainder of the molecule through an oxygen atom, -O-. Examples include alkoxy having 1 to 10 carbons, such as methoxy, ethoxy, propoxy, and butoxy.

The term "alkylamino having 1 to 10 carbons" refers to any of the above alkyl groups (e.g., alkyl having 1 to 10 carbons, etc.) attached to the remainder of the molecule through a nitrogen atom, -NR- (wherein R may be H or alkyl having 1 to 10 carbons), such as methylamino ((CH₃)NH-), ethylmethylamino ((C₂H₃)N(CH₃)-), propylamino ((C₃H₇)NH-), and butylethylamino ((C₄H₉)N(C₂H₅)-).

As used herein, the term "alkyl ester having 1 to 10 carbons" refers to -COO(alkyl having 1 to 10 carbons). Suitable ester groups include, but are not limited to, -COO(CH₃), -COO(C₂H₅), -COO(C₃H₇), -COO(C₄H₉), and the like.

The term "cycloalkyl having 3 to 10 carbons" refers to a monovalent saturated hydrocarbon ring having 3 to 10 ring carbon atoms, which may be in the form of a single ring, fused ring, bridged ring, and the like. Exemplary examples of cycloalkyl groups include, but are not limited to, the following moieties: 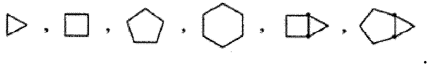 Examples include 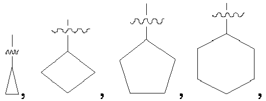 and the like.

Halogen refers to fluorine, chlorine, bromine, or iodine.

The term "aryl having 6 to 10 carbons" refers to an aromatic monocyclic or bicyclic group having 6 to 10 carbons. Specific examples include phenyl and naphthyl, preferably phenyl.

The term "heteroaryl having 4 to 10 carbons" refers to an unsaturated group with a conjugated π-electron system having 4 to 10 carbons and at least one (e.g., 1, 2, 3, or 4) ring atom as a heteroatom selected from N, O, and S, wherein the nitrogen atom is optionally quaternized and the nitrogen and sulfur heteroatoms are optionally oxidized. For example, it is composed of 4 to 12 (e.g., 4, 5, 6, 7, 8, 9, 10, 11, or 12) ring atoms, including 4- to 10-membered, 4- to 9-membered, 6-to 9-membered, 5- to 6-membered heteroaryl, and the like. The heteroaryl groups include monocyclic and polycyclic rings. Specific examples include, but are not limited to, furyl, pyrrolyl, thienyl, pyrazolyl, imidazolyl, thiazolyl, isothiazolyl, thiadiazolyl, oxazolyl, isoxazolyl, oxadiazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, benzofuryl, benzothienyl, benzoxazolyl, benzisoxazolyl, benzimidazolyl, benzotriazolyl, indazolyl, indolyl, quinolinyl, isoquinolinyl, and the like.

As used herein, the term "heterocyclyl having 4 to 10 carbons" refers to a saturated or partially unsaturated cyclic group having 4 to 10 carbons and at least one (e.g., 1, 2, 3, 4, or 5 heteroatoms) ring atom as a heteroatom selected from N, O, and S, wherein the nitrogen atom is optionally quaternized, the nitrogen and sulfur heteroatoms are optionally oxidized, and the carbon atom is optionally oxo. For example, it is composed of 4, 5, 6, 7, 8, 9, 10, 11, or 12 ring atoms, including 4- to 10-membered heterocyclyl, 4- to 9-membered heterocyclyl, 6- to 9-membered heterocyclyl, and the like. The heterocyclyl groups include monocyclic, bicyclic, or polycyclic rings, including spiro rings, fused rings, or bridged rings. Specific examples include, but are not limited to, pyrrolidinyl, piperidinyl, piperazinyl, morpholinyl, imidazolinyl, 2,3-dihydrobenzofuryl, 1,2,3,4-tetrahydroquinolinyl, 3,4-dihydro-1H-benzopyranyl, 1,4-benzodioxanyl, benzomorpholinyl, 1,2,3,4-tetrahydroquinoxaline, and the like.

The term "substituted" means optionally substituted with one or more (e.g., 2 to 9, such as 2, 3, 4, 5, 6, 7, 8, and 9) substituents selected from: halogen, hydroxyl, carbonyl, amino, cyano, carboxyl, aryl having 6 to 10 carbons, heteroaryl having 4 to 10 carbons, cycloalkyl having 3 to 6 carbons, alkoxy having 1 to 10 carbons, alkylamino having 1 to 10 carbons, or alkyl ester having 1 to 10 carbons, and other various groups.

In the present disclosure, "treatment" generally means obtaining a desired pharmacological and/or physiological effect. The effect may be prophylactic in that it completely or partially prevents the disease or its symptoms; and/or therapeutic in that it partially or completely stabilizes or cures the disease and/or side effects arising from the disease. As used herein, "treatment" encompasses any treatment of a disease in a patient, including: (a) preventing the disease or symptoms from occurring in a patient who may be predisposed to the disease or symptoms but has not yet been diagnosed as having it; (b) inhibiting the symptoms of the disease, i.e., arresting their development; or (c) alleviating the symptoms of the disease, i.e., causing regression of the disease or symptoms.

In the present disclosure, "effective amount" refers to an amount that is effective in achieving the desired therapeutic or prophylactic effect at the required dosage and time. The "therapeutically effective amount" of a substance/molecule of the present disclosure may vary depending on factors such as the individual's disease state, age, sex, body weight, and the ability of the substance/molecule to elicit the desired response in the individual. A therapeutically effective amount also encompasses an amount at which the therapeutic benefits of the substance/molecule outweigh any toxic or harmful consequences. A "prophylactically effective amount" refers to an amount that is effective to achieve the desired prophylactic effect at the required dosage and time. Generally, but not necessarily, since a prophylactic dose is administered to a subject before the onset of a disease or at an early stage of a disease, a prophylactically effective amount is lower than a therapeutically effective amount. In the case of cancer, the therapeutically effective amount of a drug may reduce the number of cancer cells; reduce tumor volume; inhibit (i.e., slow to some extent, preferably stop) cancer cell infiltration into surrounding organs; inhibit (i.e., slow to some extent, preferably stop) tumor metastasis; inhibit tumor growth to some extent; and/or alleviate to some extent one or more symptoms associated with the cancer.

In the present disclosure, "subject" refers to a vertebrate. In certain embodiments, the vertebrate refers to a mammal. Mammals include, but are not limited to, livestock (such as cattle), pets (such as cats, dogs, and horses), primates, mice, and rats. In certain embodiments, the mammal is a human.

The compound described in the present disclosure may optionally also be used in combination with one or more other active ingredients, and their respective dosages and proportions can be adjusted by those skilled in the art according to the particular conditions and patient conditions, as well as the clinical needs, and the like.

As used herein, the term "optical isomer" refers to a substance whose structure can rotate the plane of polarization of polarized light by a certain angle, hence termed an optically active substance. These structures are mutually referred to as optical isomers, including enantiomers and diastereomers.

As used herein, the term "solvate" refers to a compound that exists in combination with a certain solvent molecule. The combination may include a stoichiometric amount of a certain solvent, for example, when the solvent is water, a "hydrate" is formed, such as a monohydrate or dihydrate, or may include any amount of water; as another example, when the solvent is an alcohol, such as methanol or ethanol, an "alcohol solvate" may be formed, which may also be stoichiometric or non-stoichiometric.

Hereinafter, the embodiments of the present disclosure will be described in detail with reference to examples. However, those skilled in the art will understand that the following examples are only intended to illustrate the present disclosure and should not be construed as limiting the scope of the present disclosure. Examples, wherein specific conditions are not specified, are implemented in accordance with general conditions. All of the used reagents or instruments, whose manufacturers are not specified, are conventional commercially available products.

In the present disclosure, liquid chromatography-mass spectrometry (LC-MS) is performed using an AB Sciex TripleTOF 4600 mass spectrometer; nuclear magnetic resonance is performed using a BRUKER AVANCE NEO 400MHz nuclear magnetic resonance spectrometer; liquid chromatography is performed using a Shimadzu LC-20AD XR high-performance liquid chromatography system, with a chromatographic column: Agela Venusil MP C18 (2.1x50, 3um).

### Synthetic Example 1

### (2S,6aS,6bR,7S,8aS,8bS,11aR,12aS,12bS)-2,6b-Difluoro-7-hydroxy-8b-(2-hydroxyacetyl)-6a,8a-dimethyl-10-(1-(5-oxotetrahydrofuran-3-carbonyl)piperidin-4-yl)-1,2,6a,6b,7,8,8a,8b,11a,12,12a,1 2b-dodecahydro-4H-naphtho[2',1':4,5]indeno[1,2-d][1,3]dioxol-4-one (1)

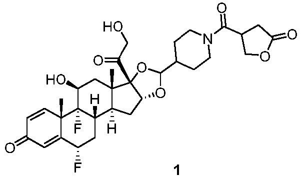

### 1-(5-Oxotetrahydrofuran-3-carbonyl)piperidine-4-carbaldehyde

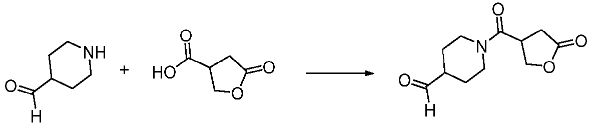

Piperidine-4-carbaldehyde (0.5 g, 4.42 mmol), 5-oxotetrahydrofuran-3-carboxylic acid (0.63 g, 4.86 mmol), 2-(7-azabenzotriazol-1-yl)-*N,N,N',N'*-tetramethyluronium hexafluorophosphate (1.85 g, 4.86 mmol), *N*,*N*-diisopropylethyl amine (1.54 mL, 8.84 mmol), and 30 mL of dichloromethane were added to a reaction flask and allowed to react at room temperature for 5 hours. After completion of the reaction, the mixture was concentrated under reduced pressure. The crude product was separated and purified by silica gel column chromatography (petroleum ether/ethyl acetate = 4/1) and concentrated under reduced pressure to obtain 1-(5-oxotetrahydrofuran-3-carbonyl)piperidine-4-carbaldehyde (0.55 g, yield: 55%). MS m/z (ESI): 226.22 [M+H]⁺

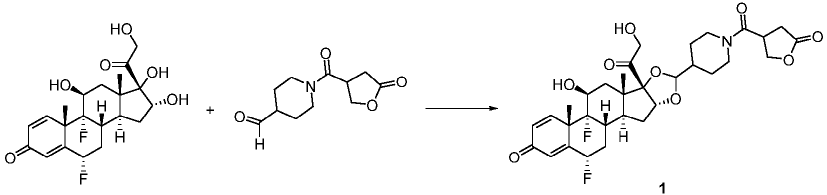

(6α,9α,11β,16α)-6,9-Difluoro-11,16,17,21-tetrahydroxy-pregna-1,4-diene-3,20-dione (0.5 g, 1.21 mmol), 1-(5-oxotetrahydrofuran-3-carbonyl)piperidine-4-carbaldehyde (0.33 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 1 (0.35 g, yield: 46%). ¹H NMR (400 MHz, d₆-DMSO): δ 7.27 (m, 1H), 6.32 (m, 1H), 6.15 (s, 1H), 5.55-5.53 (m, 1H), 5.52 (s, 1H), 5.50 (s, 1H), 5.22-5.15 (m, 2H), 5.02-4.85 (m, 2H), 4.35-4.18 (m, 3H), 3.94-3.44 (m, 4H), 3.12-3.05 (m, 1H), 2.62-2.58 (m, 2H), 2.12-2.01 (m, 5H), 1.83-1.45 (m, 7H), 1.40-1.33 (m, 4H), 0.90-0.86 (m, 3H). MS m/z (ESI): 620.28 [M+H]⁺

### Synthetic Example 2

### N-(4-((2S,6aS,6bR,7S,8aS,8bS,11aR,12aS,12bS)-2,6b-Difluoro-7-hydroxy-8b-(2-hydroxyacetyl)-6a,8a-dimethyl-4-oxo-2,4,6a,6b,7,8,8a,8b,11a,12,12a,12b-dodecahydro-1H-naphtho[2',1':4,5]inde no[1,2-d][1,3]dioxol-10-yl)phenyl)-5-oxotetrahydrofuran-3-carboxamide (2)

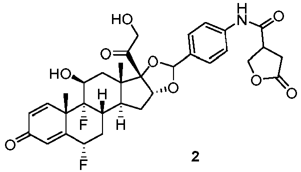

### N-(4-Formylphenyl)-5-oxotetrahydrofuran-3-carboxamide

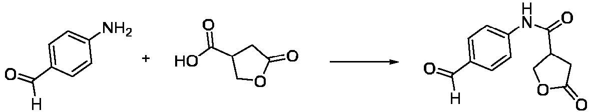

4-Aminobenzaldehyde (0.5 g, 4.13 mmol), 5-oxotetrahydrofuran-3-carboxylic acid (0.6 g, 4.54 mmol), 2-(7-azabenzotriazol-1-yl)-*N,N,N',N'*-tetramethyluronium hexafluorophosphate (1.73 g, 4.54 mmol), *N,N*-diisopropylethyl amine (1.44 mL, 8.25 mmol), and 30 mL of dichloromethane were added to a reaction flask and allowed to react at room temperature for 5 hours. After completion of the reaction, the mixture was concentrated under reduced pressure. The crude product was separated and purified by silica gel column chromatography (petroleum ether/ethyl acetate = 4/1) and concentrated under reduced pressure to obtain *N*-(4-formylphenyl)-5-oxotetrahydrofuran-3-carboxamide (0.6 g, yield: 62%). MS m/z (ESI): 234.10 [M+H]⁺

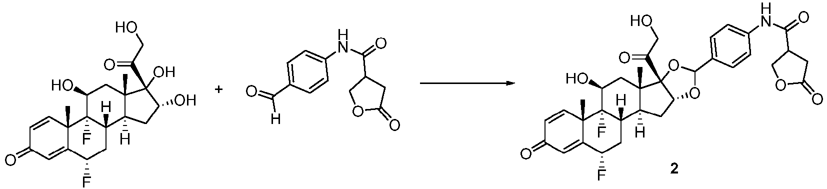

(6α,9α,11β,16α)-6,9-Difluoro-11,16,17,21-tetrahydroxy-pregna-1,4-diene-3,20-dione (0.5 g, 1.21 mmol), *N*-(4-formylphenyl)-5-oxotetrahydrofuran-3-carboxamide (0.34g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **2** (0.33 g, yield: 43%). ¹H NMR (400 MHz, d₆-DMSO): δ 8.33 (s, 1H), 7.45-7.37 (m, 4H), 7.26 (m, 1H), 6.44 (m, 1H), 6.22 (s, 1H), 5.55-5.53 (m, 1H), 5.48 (s, 1H), 5.45 (s, 1H), 5.08-4.95 (m, 2H), 4.65-4.59 (m, 2H), 4.22-3.94 (m, 3H), 3.43-3.22 (m, 1H), 2.82-2.73 (m, 2H), 2.42-2.29 (m, 6H), 2.05-1.96 (m, 1H), 1.70-1.65 (m, 3H), 1.60-1.55 (m, 1H), 0.88-0.86 (s, 3H). MS m/z (ESI): 628.25 [M+H]⁺

### Synthetic Example 3

### N-(4-((2S,6aS,6bR,7S,8aS,8bS,11aR,12aS,12bS)-2,6b-Difluoro-7-hydroxy-8b-(2-hydroxyacetyl)-6a,8a-dimethyl-4-oxo-2,4,6a,6b,7,8,8a,8b,11a,12,12a,12b-dodecahydro-1H-naphtho[2',1':4,5]inde no[1,2-d][1,3]dioxol-10-yl)cyclohexyl)-5-oxotetrahydrofuran-3-carboxamide (3)

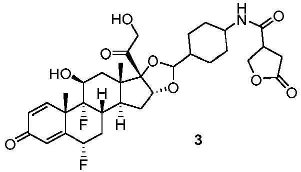

### N-(4-Formylcyclohexyl)-5-oxotetrahydrofuran-3-carboxamide

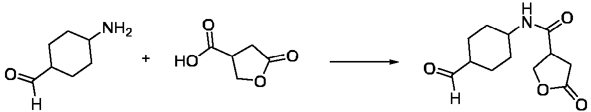

4-Aminocyclohexane-1-carbaldehyde (0.5 g, 3.93 mmol), 5-oxotetrahydrofuran-3-carboxylic acid (0.56 g, 4.32 mmol), 2-(7-azabenzotriazol-1-yl)-*N,N,N',N'*-tetramethyluronium hexafluorophosphate (1.64 g, 4.32 mmol), *N,N*-diisopropylethyl amine (1.37 mL, 7.86 mmol), and 30 mL of dichloromethane were added to a reaction flask and allowed to react at room temperature for 5 hours. After completion of the reaction, the mixture was concentrated under reduced pressure. The crude product was separated and purified by silica gel column chromatography (petroleum ether/ethyl acetate = 4/1) and concentrated under reduced pressure to obtain *N*-(4-formylcyclohexyl)-5-oxotetrahydrofuran-3-carboxamide (0.45 g, yield: 48%). MS m/z (ESI): 240.23 [M+H]⁺

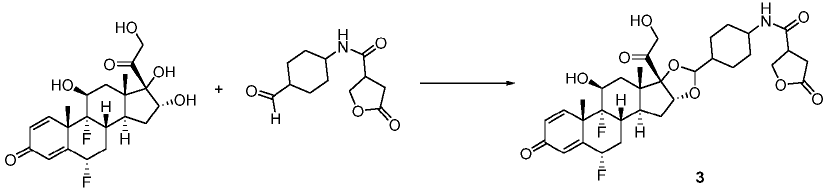

(6α,9α,11β,16α)-6,9-Difluoro-11,16,17,21-tetrahydroxy-pregna-1,4-diene-3,20-dione (0.5 g, 1.21 mmol), *N*-(4-formylcyclohexyl)-5-oxotetrahydrofuran-3-carboxamide (0.35 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 3 (0.4 g, yield: 54%). ¹H NMR (400 MHz, d₆-DMSO): δ 8.35 (s, 1H), 7.26 (m, 1H), 6.33 (m, 1H), 6.11 (s, 1H), 5.57-5.53 (m, 1H), 5.50 (s, 1H), 5.48 (s, 1H), 5.32-5.20 (m, 2H), 4.92-4.85 (m, 2H), 4.32-4.20 (m, 3H), 3.54-3.47 (m, 1H), 3.04-2.98 (m, 1H), 2.67-2.58 (m, 2H), 2.15-2.01 (m, 9H), 1.86-1.48 (m, 7H), 1.38-1.30 (m, 4H), 0.86-0.83 (m, 3H). MS m/z (ESI): 634.25 [M+H]⁺

### Synthetic Example 4

### S-(Fluoromethyl)

### (2S,6aS,6bR,7S,8aS,8bS,11aR,12aS,12bS)-2,6b-difluoro-7-hydroxy-6a,8a-dimethyl-4-oxo-10-(1-( 5-oxotetrahydrofuran-3-carbonyl)piperidin-4-yl)-1,2,4,6a,6b,7,8,8a,11a,12,12a,12b-dodecahydro-8bH-naphtho[2',1':4,5]indeno[1,2-d][1,3]dioxole-8b-carbothioate (4)

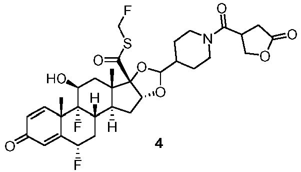

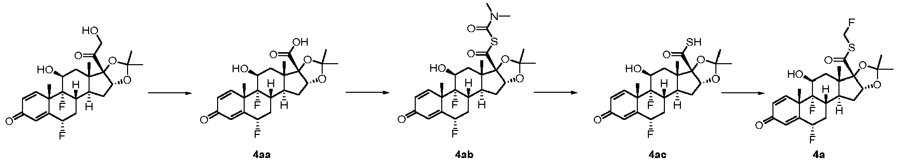

### Step 1

Fluocinolone acetonide (10 g, 0.022 mol) and 100 mL of tetrahydrofuran were added to a reaction flask. Sodium periodate (5.1 g, 0.024 mol) was added in portions, and the mixture was allowed to react at room temperature. TLC monitoring was performed until no fluocinolone acetonide was detected. After completion of the reaction, the mixture was concentrated under reduced pressure.

Water was added, and the concentration was continued until no solvent was distilled off. The pH of the system was adjusted to 9-10 with 1% sodium hydroxide solution, and all the solids were dissolved. The aqueous phase was washed with ethyl acetate and adjusted to pH 1-2 with concentrated hydrochloric acid. The solids precipitated. The mixture was filtered under reduced pressure. The filter cake was washed with water until neutral and dried to obtain compound **4aa** (8.4 g, yield: 87%).

### Step 2

Compound **4aa** (8 g, 0.018 mol), triethylamine (2.8 mL, 0.02 mol), sodium iodide (3 g, 0.02 mol), and 80 mL of tetrahydrofuran were added to a reaction flask. Dimethylthiocarbamoyl chloride (11.1 g, 0.09 mol) was slowly added, and the mixture was allowed to react at room temperature. TLC monitoring was performed until no compound **4aa** was detected. After completion of the reaction, the mixture was concentrated under reduced pressure. Water was added, and the concentration was continued until no solvent was distilled off. The mixture was filtered under reduced pressure. The filter cake was dried to obtain compound **4ab** (7.6 g, yield: 80%).

### Step 3

Compound **4ab** (7.5 g, 0.014 mol), potassium carbonate (2.9 g, 0.021 mol), and 80 mL of methanol were added to a reaction flask and heated to reflux for reaction. TLC monitoring was performed until no compound **4ab** was detected. After completion of the reaction, the mixture was concentrated under reduced pressure. Water was added, and the concentration was continued until no solvent was distilled off. The mixture was filtered under reduced pressure. The filter cake was dried to obtain compound **4ac** (5.4 g, yield: 85%).

### Step 4

Compound **4ac** (5 g, 0.011 mol), sodium sulfite (2.1 g, 0.017 mol), and 20 mL of *N,N*-dimethylacetamide were added to a reaction flask. Fluoroiodomethane (2.7 g, 0.017 mol) was slowly added, and the mixture was allowed to react at room temperature. TLC monitoring was performed until no compound **4ac** was detected. After completion of the reaction, the reaction solution was poured into water and stirred for 30 minutes. The mixture was filtered under reduced pressure. The filter cake was dried. The crude product was separated and purified by silica gel column chromatography (dichloromethane/acetone = 4/1) and concentrated under reduced pressure to obtain compound **4a** (1.9 g, yield: 35%). MS m/z (ESI): 487.20 [M+H]⁺

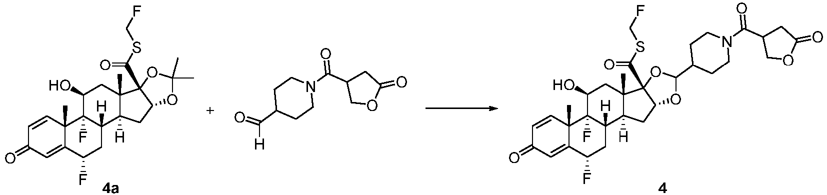

Compound **4a** (0.5 g, 1 mmol), 1-(5-oxotetrahydrofuran-3-carbonyl)piperidine-4-carbaldehyde (0.28 g, 1.23 mmol), and 30 mL of acetonitrile were added to a reaction flask. Perchloric acid (0.26 mL, 3.1 mmol) was slowly added dropwise, and the mixture was allowed to react at room temperature. TLC monitoring was performed until no compound **4a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **4** (0.32 g, yield: 48%). ¹H NMR (400 MHz, d₆-DMSO): δ 7.28 (m, 1H), 6.30 (m, 1H), 6.15 (s, 1H), 5.76 (s, 2H), 5.52-5.49 (m, 1H), 5.42 (s, 1H), 5.35 (s, 1H),5.15-4.95 (m, 2H), 4.34-4.14 (m, 3H), 3.94-3.42 (m, 4H), 3.04-2.95 (m, 1H), 2.82-2.78 (m, 2H), 2.05-1.94 (m, 5H), 1.73-1.40 (m, 7H), 1.30-1.23 (m, 3H), 0.86-0.83 (m, 3H). MS m/z (ESI): 654.24 [M+H]⁺

### Synthetic Example 5

### S-(Fluoromethyl)

### (2S,6aS,6bR,7S,8aS,8bS,11aR,12aS,12bS)-2,6b-difluoro-7-hydroxy-6a,8a-dimethyl-4-oxo-10-(4-( 5-oxotetrahydrofuran-3-carboxamido)phenyl)-1,2,4,6a,6b,7,8,8a,11a,12,12a,12b-dodecahydro-8b H-naphtho[2',1':4,5]indeno[1,2-d][1,3]dioxole-8b-carbothioate (5)

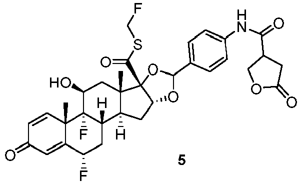

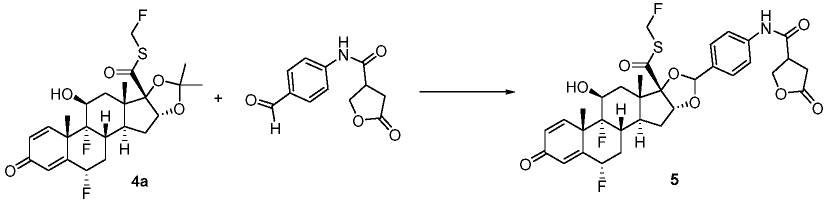

Compound **4a** (0.5 g, 1 mmol), N-(4-formylphenyl)-5-oxotetrahydrofuran-3-carboxamide (0.29 g, 1.23 mmol), and 30 mL of acetonitrile were added to a reaction flask. Perchloric acid (0.26 mL, 3.1 mmol) was slowly added dropwise, and the mixture was allowed to react at room temperature. TLC monitoring was performed until no compound **4a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **5** (0.35 g, yield: 52%). ¹H NMR (400 MHz, d₆-DMSO): δ 8.35 (s, 1H), 7.47-7.38 (m, 4H), 7.28 (m, 1H), 6.44 (m, 1H), 6.16 (s, 1H), 5.80 (s, 1H), 5.54-5.52 (m, 1H), 5.50 (s, 1H), 5.48 (s, 1H), 5.05-4.88 (m, 2H), 4.33-3.98 (m, 3H), 3.53-3.26 (m, 1H), 2.80-2.73 (m, 2H), 2.40-2.25 (m, 6H), 2.03-1.95 (m, 1H), 1.72-1.65 (m, 3H), 1.62-1.57 (m, 1H), 0.90-0.86 (s, 3H). MS m/z (ESI): 662.32 [M+H]⁺

### Synthetic Example 6

### S-(Fluoromethyl)

### (2S,6aS,6bR,7S,8aS,8bS,11aR,12aS,12bS)-2,6b-difluoro-7-hydroxy-6a,8a-dimethyl-4-oxo-10-(4-( 5-oxotetrahydrofuran-3-carboxamido)cyclohexyl)-1,2,4,6a,6b,7,8,8a,11a,12,12a,12b-dodecahydro -8bH-naphtho[2',1':4,5]indeno[1,2-d][1,3]dioxole-8b-carbothioate (6)

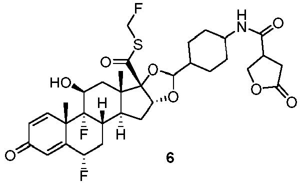

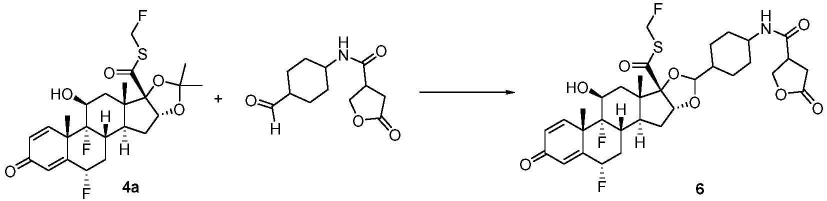

Compound **4a** (0.5 g, 1 mmol), *N*-(4-formylcyclohexyl)-5-oxotetrahydrofuran-3-carboxamide (0.3 g, 1.23 mmol), and 30 mL of acetonitrile were added to a reaction flask. Perchloric acid (0.26 mL, 3.1 mmol) was slowly added dropwise, and the mixture was allowed to react at room temperature. TLC monitoring was performed until no compound **4a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **6** (0.38 g, yield: 55%). ¹H NMR (400 MHz, d₆-DMSO): δ 8.36 (s, 1H), 7.28 (m, 1H), 6.35 (m, 1H), 6.15 (s, 1H), 5.78 (s, 1H), 5.55-5.53 (m, 1H), 5.50 (s, 1H), 5.47 (s, 1H), 5.20-4.90 (m, 2H), 4.52-4.40 (m, 3H), 3.74-3.58 (m, 1H), 3.10-3.01 (m, 1H), 2.88-2.68 (m, 2H), 2.20-2.11 (m, 9H), 1.86-1.48 (m, 7H), 1.40-1.32 (m, 4H), 0.87-0.83 (m, 3H). MS m/z (ESI): 668.35 [M+H]⁺

### Synthetic Example 7

### (2S,6aS,6bR,7S,8aS,8bS,11aR,12aS,12bS)-2,6b-Difluoro-10-(2-fluoro-4-(((2-oxotetrahydrofuran-3-yl)oxy)methyl)phenyl)-7-hydroxy-8b-(2-hydroxyacetyl)-6a,8a-dimethyl-1,2,6a,6b,7,8,8a,8b,11a, 12,12a,12b-dodecahydro-4H-naphtho[2',1':4,5]indeno[1,2-d][1,3]dioxol-4-one(7)

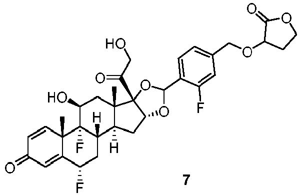

### 2-Fluoro-4-(((2-oxotetrahydrofuran-3-yl)oxy)methyl)benzaldehyde

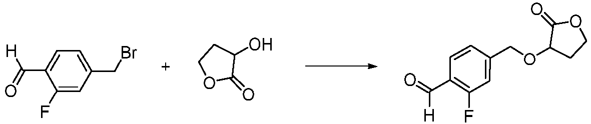

Sodium hydride (60%) (0.54 g, 13.5 mmol), 3-hydroxydihydrofuran-2(3H)-one (1.25 g, 12.3 mmol), and 50 mL of anhydrous tetrahydrofuran were added to a reaction flask, cooled to 0°C, and allowed to react for 30 minutes. 4-(Bromomethyl)-2-fluorobenzaldehyde (3.3 g, 15.3 mmol) and tetrabutylammonium iodide (0.45 g, 1.2 mmol) were added, and the mixture was allowed to react at room temperature for 3 hours. After completion of the reaction, water and dichloromethane were slowly added for extraction of the aqueous phase. The organic phases were combined and concentrated under reduced pressure. The crude product was separated and purified by silica gel column chromatography (petroleum ether/ethyl acetate = 6/4) and concentrated under reduced pressure to obtain 2-fluoro-4-(((2-oxotetrahydrofuran-3-yl)oxy)methyl)benzaldehyde (1.4 g, yield: 49%). MS m/z (ESI): 239.12 [M+H]⁺

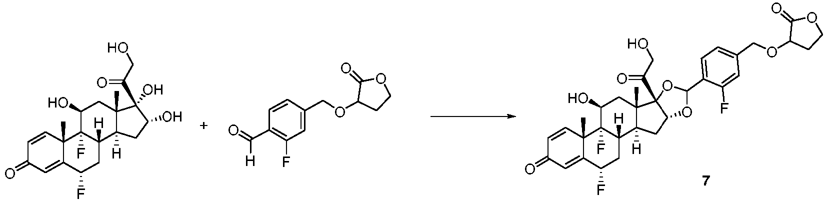

(6α,9α,11β,16α)-6,9-Difluoro-11,16,17,21-tetrahydroxy-pregna-1,4-diene-3,20-dione (0.5 g, 1.21 mmol), 2-fluoro-4-(((2-oxotetrahydrofuran-3-yl)oxy)methyl)benzaldehyde (0.35 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 7 (0.44 g, yield: 57%). ¹H NMR (400 MHz, d₆-DMSO): δ 7.44-7.37 (m, 3H), 7.26 (d, *J* =4.4 Hz, 1H), 6.32 (d, *J* =8.0 Hz, 1H), 6.10 (s, 1H), 5.67-5.54 (m, 1H), 5.50 (s, 2H), 5.45 (s, 1H), 5.40 (s, 1H), 5.15-4.98 (m, 2H), 4.57-4.52 (m, 1H), 4.30-4.18 (m, 4H), 3.67-3.59 (m, 1H), 2.78-2.68 (m, 2H), 2.30-2.26 (m, 2H), 2.09-1.95 (m, 2H), 1.75-1.71 (m, 3H), 1.68-1.51 (m, 4H), 0.88 (s, 3H). MS m/z (ESI): 633.25 [M+H]⁺

### Synthetic Example 8

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((2-oxotetrahydrofuran-3-yl)thio)methyl)phenyl)-1,2,6a,6b,7,8,8a,8b,11a,12,12a, 12b-dodecahydro-4H-naphtho[2',1':4,5]indeno[1,2-d][1,3]dioxol-4-one (8)

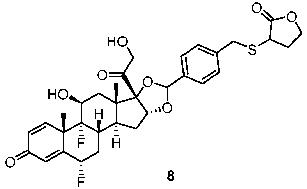

### 4-(((2-Oxotetrahydrofuran-3-yl)thio)methyl)benzaldehyde

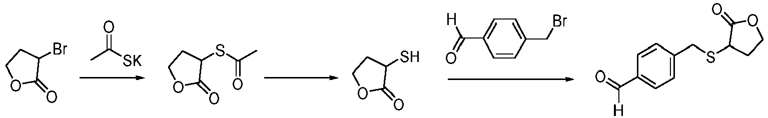

### Step 1

3-Bromodihydrofuran-2(3*H*)-one (20 g, 0.12 mol), potassium thioacetate (16 g, 0.14 mol), and 200 mL of acetone were added to a reaction flask and allowed to react at room temperature for 3 hours. After completion of the reaction, the mixture was filtered under reduced pressure. The filtrate was concentrated under reduced pressure. Dichloromethane was added. The organic phase was washed successively with water and saturated brine and concentrated under reduced pressure to obtain *S*-(2-oxotetrahydrofuran-3-yl) ethanethioate (15.4 g, yield: 82%).

### Step 2

*S*-(2-Oxotetrahydrofuran-3-yl) ethanethioate (15 g, 0.09 mol), hydrazine hydrate (80%) (7 mL, 0.11 mol), and 100 mL of acetonitrile were added to a reaction flask and allowed to react at room temperature for 4 hours. After completion of the reaction, the reaction solution was concentrated under reduced pressure. Dichloromethane was added for liquid-liquid separation and extraction. The organic phases were combined and concentrated under reduced pressure. The crude product was separated and purified by silica gel column chromatography (petroleum ether/ethyl acetate = 2/1) to obtain 3-mercaptodihydrofuran-2(3*H*)-one (8 g, yield: 75%).

### Step 3

3-Mercaptodihydrofuran-2(3*H*)-one (8 g, 0.07 mol), 4-(bromomethyl)benzaldehyde (15.3 g, 0.077 mol), potassium carbonate (11 g, 0.08 mol), and 60 mL of *N,N-*dimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 4-(((2-oxotetrahydrofuran-3-yl)thio)methyl)benzaldehyde (10.8 g, yield: 65%). MS m/z (ESI): 237.14 [M+H]⁺

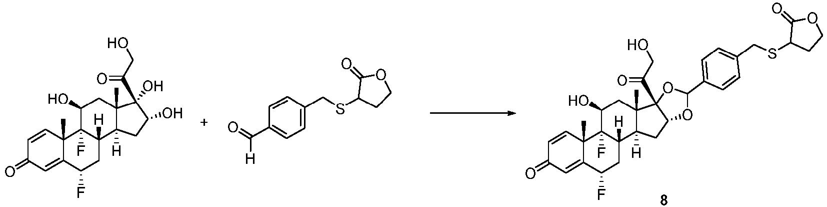

(6α,9α,11β,16α)-6,9-Difluoro-11,16,17,21-tetrahydroxy-pregna-1,4-diene-3,20-dione (0.5 g, 1.21 mmol), 4-(((2-oxotetrahydrofuran-3-yl)thio)methyl)benzaldehyde (0.34 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **8** (0.41 g, yield: 54%). ¹H NMR (400 MHz, d₆-DMSO): δ 7.41-7.34 (m, 4H), 7.26 (d, *J* =4.4 Hz, 1H), 6.30 (d, *J* =8.0 Hz, 1H), 6.12 (s, 1H), 5.52-5.51 (m, 1H), 5.50 (s, 1H), 5.48 (s, 1H), 5.11-4.95 (m, 2H), 4.55-4.49 (m, 1H), 4.28-4.18 (m, 4H), 4.02-3.94 (m, 2H), 3.56-3.53 (m, 1H), 2.62-2.58 (m, 2H), 2.32-2.29 (m, 2H), 2.05-1.96 (m, 2H), 1.74-1.71 (m, 3H), 1.68-1.49 (m, 4H), 0.87-0.86 (m, 3H). MS m/z (ESI): 631.31 [M+H]⁺

### Synthetic Example 9

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((2-oxotetrahydrofuran-3-yl)amino)methyl)phenyl)-1,2,6a,6b,7,8,8a,8b,11a,12,12 a,12b-dodecahydro-4H-naphtho[2',1':4,5]indeno[1,2-d][1,3]dioxol-4-one(9)

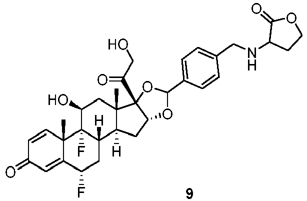

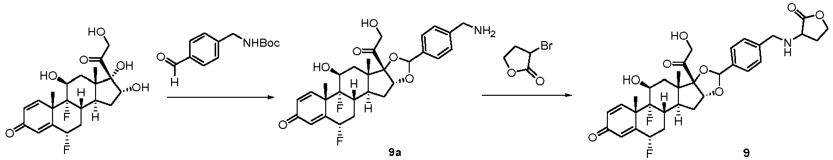

### Step 1

(6α,9α,11β,16α)-6,9-Difluoro-11,16,17,21-tetrahydroxy-pregna-1,4-diene-3,20-dione (0.8 g, 1.94 mmol), tert-butyl (4-formylbenzyl)carbamate (0.68 g, 2.91 mmol), and 30 mL of acetonitrile were added to a reaction flask. Perchloric acid (0.5 mL, 5.82 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **9a** (0.65 g, yield: 63%).

### Step 2

3-Bromodihydrofuran-2(3*H*)-one (0.15 g, 0.94 mmol), compound **9a** (0.5 g, 0.94 mmol), potassium phosphate (0.6 g, 2.82 mmol), and 50 mL of acetonitrile were added to a reaction flask and allowed to react at room temperature. TLC monitoring was performed until no compound **9a** was detected. After completion of the reaction, the mixture was filtered under reduced pressure. The filtrate was concentrated under reduced pressure. Dichloromethane was added. The organic phase was washed successively with water and saturated brine and concentrated under reduced pressure. The crude product was separated and purified by silica gel column chromatography (dichloromethane/acetone = 3/1) and concentrated under reduced pressure to obtain compound **9** (0.18 g, yield: 32%). ¹H NMR (400 MHz, d₆-DMSO): δ 7.40-7.34 (m, 4H), 7.26 (d, *J* =4.4 Hz, 1H), 6.29 (d, *J* =8.0 Hz, 1H), 6.10 (s, 1H), 5.55-5.53 (m, 1H), 5.50 (s, 1H), 5.46 (s, 1H), 5.10-4.95 (m, 2H), 4.66-4.52 (m, 2H), 4.28-4.18 (m, 4H), 3.98-3.85 (m, 2H), 3.43-3.38 (m, 1H), 2.32-2.24 (m, 2H), 2.12-1.86 (m, 7H), 1.78-1.71 (m, 3H), 1.64-1.50 (m, 1H), 0.89 (m, 3H). MS m/z (ESI): 614.33 [M+H]⁺

### Synthetic Example 10

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((5-oxotetrahydrofuran-3-yl)oxy)methyl)phenyl)-1,2,6a,6b,7,8,8a,8b,11a,12,12a,1 2b-dodecahydro-4H-naphtho[2',1':4,5]indeno[1,2-d][1,3]dioxol-4-one (10)

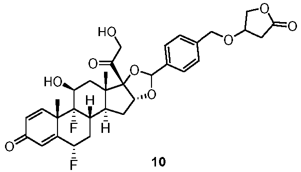

### 4-(((5-Oxotetrahydrofuran-3-yl)oxy)methyl)benzaldehyde

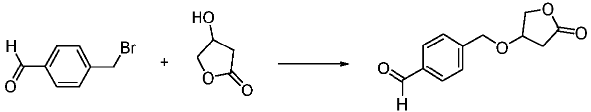

Sodium hydride (60%) (0.4 g, 10 mmol), 4-hydroxydihydrofuran-2(3H)-one (0.93 g, 9.11 mmol), and 50 mL of anhydrous tetrahydrofuran were added to a reaction flask, cooled to 0°C, and allowed to react for 30 minutes. 4-(Bromomethyl)benzaldehyde (2.2 g, 11.1 mmol) and tetrabutylammonium iodide (0.3 g, 0.81 mmol) were added, and the mixture was allowed to react at room temperature for 3 hours. After completion of the reaction, water and dichloromethane were slowly added for extraction of the aqueous phase. The organic phases were combined and concentrated under reduced pressure. The crude product was separated and purified by silica gel column chromatography (petroleum ether/ethyl acetate = 6/4) and concentrated under reduced pressure to obtain 4-(((5-oxotetrahydrofuran-3-yl)oxy)methyl)benzaldehyde (1.24 g, yield: 62%). MS m/z (ESI): 221.10 [M+H]⁺

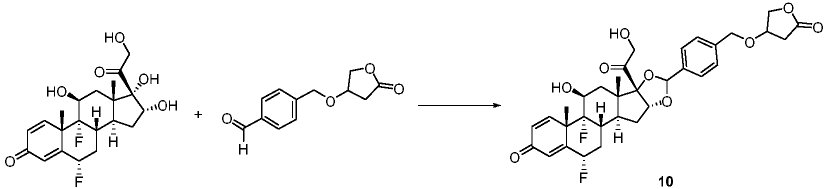

(6α,9α,11β,16α)-6,9-Difluoro-11,16,17,21-tetrahydroxy-pregna-1,4-diene-3,20-dione (0.5 g, 1.21 mmol), 4-(((5-oxotetrahydrofuran-3-yl)oxy)methyl)benzaldehyde (0.32 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **10** (0.36 g, yield: 48%). ¹H NMR (400 MHz, d₆-DMSO): δ 7.39-7.35 (m, 4H), 7.27 (d, *J* =4.4 Hz, 1H), 6.30 (d, *J*=8.0 Hz, 1H), 6.15 (s, 1H), 5.60-5.56 (m, 1H), 5.52 (s, 2H), 5.48 (s, 1H), 4.98-4.85 (m, 2H), 4.78-4.66 (m, 2H), 4.53-4.47 (m, 3H), 4.18-4.10 (m, 1H), 3.95-3.88 (m, 2H), 2.66-2.56 (m, 2H), 2.22-2.10 (m, 2H), 2.01-1.92 (m, 2H), 1.88-1.75 (m, 3H), 1.69-1.53 (m, 3H), 0.90 (s, 3H). MS m/z (ESI): 615.34 [M+H]⁺

### Synthetic Example 11

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((5-oxotetrahydrofuran-3-yl)thio)methyl)phenyl)-1,2,6a,6b,7,8,8a,8b,11a,12,12a, 12b-dodecahydro-4H-naphtho[2',1':4,5]indeno[1,2-d][1,3]dioxol-4-one (11)

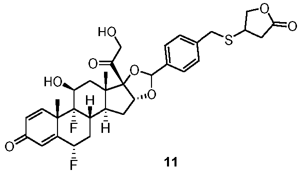

### 4-(((5-Oxotetrahydrofuran-3-yl)thio)methyl)benzaldehyde

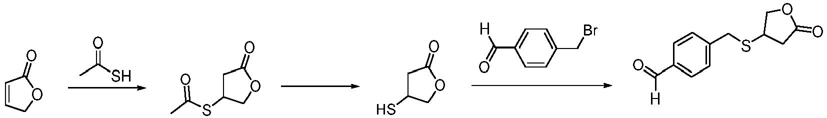

### Step 1

Furan-2(5*H*)-one (5 g, 60 mmol), thioacetic acid (4.5 g, 60 mmol), and 100 mL of dichloromethane were added to a reaction flask. Triethylamine (0.8 mL, 6 mmol) was added, and the mixture was allowed to react at room temperature for 20 hours. After completion of the reaction, the organic phase was washed successively with water and saturated brine and concentrated under reduced pressure to obtain S-(5-oxotetrahydrofuran-3-yl) ethanethioate (8.2 g, yield: 85%).

### Step 2

*S*-(5-Oxotetrahydrofuran-3-yl) ethanethioate (8 g, 0.05 mol), hydrazine hydrate (80%) (3.73 mL, 0.06 mol), and 150 mL of acetonitrile were added to a reaction flask and allowed to react at room temperature for 4 hours. After completion of the reaction, the reaction solution was concentrated under reduced pressure. Dichloromethane was added for liquid-liquid separation and extraction. The organic phases were combined and concentrated under reduced pressure. The crude product was separated and purified by silica gel column chromatography (petroleum ether/ethyl acetate = 2/1) to obtain 4-mercaptodihydrofuran-2(3*H*)-one (3.84 g, yield: 65%).

### Step 3

4-Mercaptodihydrofuran-2(3*H*)-one (3.5 g, 0.03 mol), 4-(bromomethyl)benzaldehyde (6.7 g, 0.033 mol), potassium carbonate (4.8 g, 0.035 mol), and 60 mL of *N,N-*dimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 4-(((5-oxotetrahydrofuran-3-yl)thio)methyl)benzaldehyde (5 g, yield: 71%). MS m/z (ESI): 237.11 [M+H]⁺

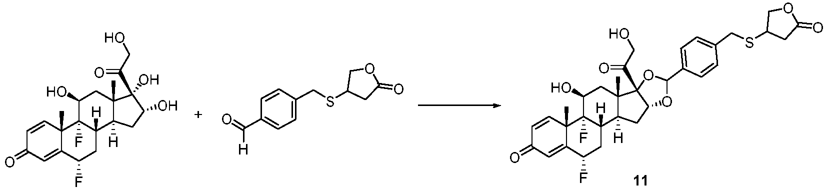

(6α,9α,11β,16α)-6,9-Difluoro-11,16,17,21-tetrahydroxy-pregna-1,4-diene-3,20-dione (0.5 g, 1.21 mmol), 4-(((5-oxotetrahydrofuran-3-yl)thio)methyl)benzaldehyde (0.34 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 11 (0.47 g, yield: 62%). ¹H NMR (400 MHz, d₆-DMSO): δ 7.43-7.36 (m, 4H), 7.26 (d, *J* =4.4 Hz, 1H), 6.29 (d, *J* =8.0 Hz, 1H), 6.10 (s, 1H), 5.49-5.45 (m, 1H), 5.42 (s, 1H), 5.40 (s, 1H), 5.08-4.93 (m, 2H), 4.77-4.69 (m, 2H), 4.20-3.98 (m, 3H), 3.75 (s, 2H), 3.22 (m, 1H), 2.75-2.52 (m, 2H), 2.21-2.03 (m, 4H), 2.01-1.88 (m, 2H), 1.72-1.68 (m, 4H), 1.58-1.43 (m, 1H), 0.87-0.86 (m, 3H). MS m/z (ESI): 631.21 [M+H]⁺

### Synthetic Example 12

### (2S,6aS,6bR,7S,8aS,8bS,11aR,12aS,12bS)-2,6b-Difluoro-10-(2-fluoro-4-(((5-oxotetrahydrofuran-3-yl)amino)methyl)phenyl)-7-hydroxy-8b-(2-hydroxyacetyl)-6a,8a-dimethyl-1,2,6a,6b,7,8,8a,8b,1 1a,12,12a,12b-dodecahydro-4H-naphtho[2',1':4,5]indeno[1,2-d][1,3]dioxol-4-one (12)

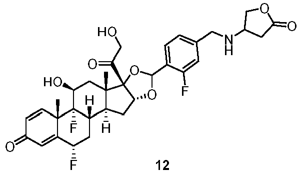

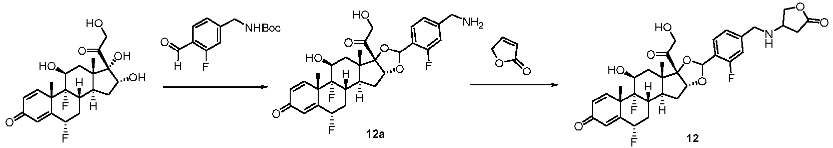

### Step 1

(6α,9α,11β,16α)-6,9-Difluoro-11,16,17,21-tetrahydroxy-pregna-1,4-diene-3,20-dione (0.8 g, 1.94 mol), tert-butyl (3-fluoro-4-formylbenzyl)carbamate (0.74 g, 2.91 mmol), and 30 mL of acetonitrile were added to a reaction flask. Perchloric acid (0.5 mL, 5.82 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene -3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) to obtain compound **12a** (0.65 g, yield: 61%).

### Step 2

Furan-2(5*H*)-one (0.12 g, 1.42 mmol), compound **12a** (0.5 g, 0.94 mmol), and 30 mL of methanol were added to a reaction flask and allowed to react at room temperature. TLC monitoring was performed until no compound **12a** was detected. After completion of the reaction, the mixture was concentrated under reduced pressure. The crude product was separated and purified by silica gel column chromatography (dichloromethane/acetone = 3/1) to obtain compound **12** (0.21 g, yield: 35%). ¹H NMR (400 MHz, d₆-DMSO): δ 7.42-7.34 (m, 3H), 7.27 (d, *J* =4.4 Hz, 1H), 6.28 (d, *J* =8.0 Hz, 1H), 6.12 (s, 1H), 5.54-5.52 (m, 1H), 5.48 (s, 1H), 5.45 (s, 1H), 5.02-4.99 (m, 2H), 4.65-4.50 (m, 2H), 4.25-4.16 (m, 3H), 3.82-3.77 (m, 2H), 3.23-3.18 (m, 1H), 2.50-2.25 (m, 3H), 2.10-1.83 (m, 7H), 1.78-1.68 (m, 3H), 1.62-1.50 (m, 1H), 0.92 (m, 3H). MS m/z (ESI): 632.33 [M+H]⁺

### Synthetic Example 13

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-((2-oxotetrahydrofuran-3-yl)methoxy)phenyl)-1,2,6a,6b,7,8,8a,8b,11a,12,12a,12b-dodecahydro-4H-naphtho[2',1':4,5]indeno[1,2-d][1,3]dioxol-4-one (13)

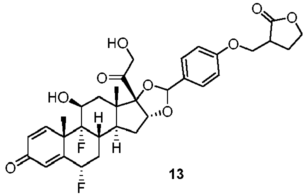

### 4-((2-Oxotetrahydrofuran-3-yl)methoxy)benzaldehyde

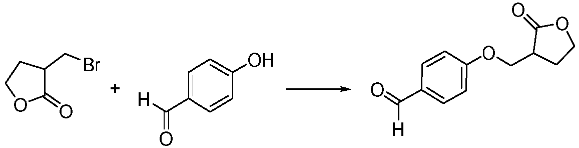

3-(Bromomethyl)dihydrofuran-2(3*H*)-one (7.88 g, 0.044 mol), 4-hydroxybenzaldehyde (5 g, 0.04 mol), potassium carbonate (6.9 g, 0.05 mol), and 150 mL of acetonitrile amine were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) to obtain 4-((2-oxotetrahydrofuran-3-yl)methoxy)benzaldehyde (4 g, yield: 45%). MS m/z (ESI): 221.10 [M+H]⁺

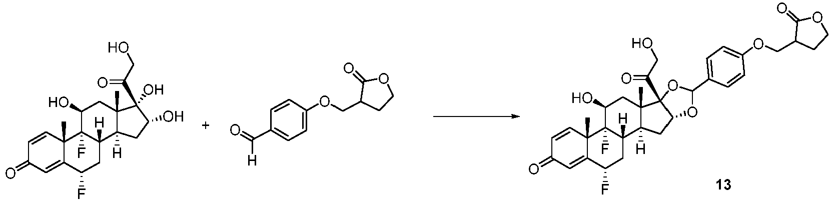

(6α,9α,11β,16α)-6,9-Difluoro-11,16,17,21-tetrahydroxy-pregna-1,4-diene-3,20-dione (0.5 g, 1.21 mmol), compound (0.32 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **13** (0.45 g, yield: 60%). ¹H NMR (400 MHz, d₆-DMSO): δ 7.43 (m, 2H), 7.07 (d, *J* =4.4 Hz, 1H), 6.89 (m, 2H), 6.40 (d, *J*=8.0 Hz, 1H), 6.33 (s, 1H), 5.79-5.75 (m, 1H), 5.22 (s, 1H), 5.15 (s, 1H), 4.92-4.85 (m, 2H), 4.56-4.44 (m, 2H), 4.27-3.96 (m, 3H), 3.77-3,69 (m, 2H), 2.60 (m, 1H), 2.37-2.12 (m, 2H), 2.02-1.92 (m, 4H), 1.90-1.78 (m, 3H), 1.74-1.58 (m, 3H), 1.55-1.48 (m, 1H), 0.91 (s, 3H). MS m/z (ESI): 615.35 [M+H]⁺

### Synthetic Example 14

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((2-oxotetrahydrofuran-3-yl)methyl)thio)phenyl)-1,2,6a,6b,7,8,8a,8b,11a,12,12a, 12b-dodecahydro-4H-naphtho[2',1':4,5]indeno[1,2-d][1,3]dioxol-4-one (14)

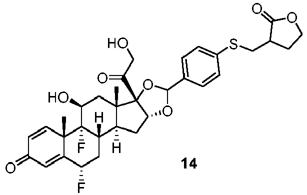

### 4-(((2-Oxotetrahydrofuran-3-yl)methyl)thio)benzaldehyde

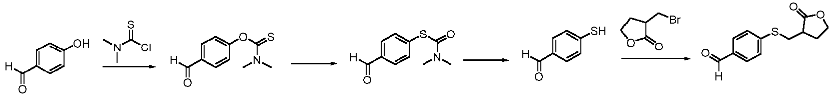

### Step 1

4-Hydroxybenzaldehyde (5 g, 40.94 mmol), 1,4-diazabicyclo[2.2.2]octane (9.2 g, 81.88 mmol), and 50 mL of *N,N*-dimethylformamide were added to a reaction flask. *N,N*-Dimethylthiocarbamoyl chloride (10.1 g, 81.88 mmol) was slowly added, and the mixture was allowed to react at room temperature for 12 hours. After completion of the reaction, the reaction solution was poured into ice water and stirred for 30 minutes. The mixture was filtered under reduced pressure. The filter cake was washed with water and dried to obtain *O*-(4-formylphenyl)dimethylthiocarbamate (5.2 g, yield: 60.6%).

### Step 2

*O*-(4-Formylphenyl)dimethylthiocarbamate (5 g, 23.9 mmol) was added to a reaction flask, heated to 200 °C, and allowed to react for 3 hours. After completion of the reaction, the crude product was separated and purified by silica gel column chromatography (petroleum ether/ethyl acetate = 3/1) to obtain *S*-(4-formylphenyl)dimethylthiocarbamate (4.2 g, yield: 84%).

### Step 3

*S*-(4-Formylphenyl)dimethylthiocarbamate (2 g, 9.6 mmol), potassium hydroxide (5 g, 89.1 mmol), and 60 mL of methanol were added to a reaction flask, heated to reflux, and allowed to react for 3 hours. After completion of the reaction, the mixture was concentrated under reduced pressure and adjusted to pH 6-7 by the addition of hydrochloric acid solution. Ethyl acetate was added for liquid-liquid separation and extraction. The organic phases were combined and concentrated under reduced pressure. The crude product was separated and purified by silica gel column chromatography (petroleum ether/ethyl acetate = 3/1) to obtain 4-mercaptobenzaldehyde (1.1 g, yield: 83.3%).

### Step 4

3-(Bromomethyl)dihydrofuran-2(3*H*)-one (1.43 g, 8 mmol), 4-mercaptobenzaldehyde (1 g, 7.24 mmol), potassium carbonate (1.2 g, 8.69 mmol), and 60 mL of acetonitrile amine were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) to obtain 4-(((2-oxotetrahydrofuran-3-yl)methyl)thio)benzaldehyde (1.1 g, yield: 65%). MS m/z (ESI): 237.10 [M+H]⁺

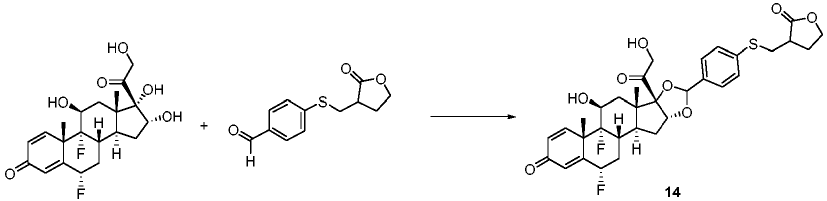

(6α,9α,11β,16α)-6,9-Difluoro-11,16,17,21-tetrahydroxy-pregna-1,4-diene-3,20-dione (0.5 g, 1.21 mmol), 4-(((2-oxotetrahydrofuran-3-yl)methyl)thio)benzaldehyde (0.34 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **14** (0.49 g, yield: 64%). ¹H NMR (400 MHz, d₆-DMSO): δ 7.33-7.31 (m, 4H), 7.06 (m, 1H), 6.40 (m, 1H), 6.33 (s, 1H), 5.79-5.75 (m, 1H), 5.25 (s, 1H), 5.17 (s, 1H), 4.85-4.69 (m, 2H), 4.47-4.22 (m, 2H), 4.07-3.83 (m, 3H), 2.84-2.53 (s, 2H), 2.40-2.12 (m, 3H), 2.01-1.76 (m, 6H), 1.62-1.58 (m, 1H), 1.55-1.43 (m, 4H), 0.87-0.86 (m, 3H). MS m/z (ESI): 631.31 [M+H]⁺

### Synthetic Example 15

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((2-oxotetrahydrofuran-3-yl)methyl)amino)phenyl)-1,2,6a,6b,7,8,8a,8b,11a,12,12 a,12b-dodecahydro-4H-naphtho[2',1':4,5]indeno[1,2-d][1,3]dioxol-4-one(15)

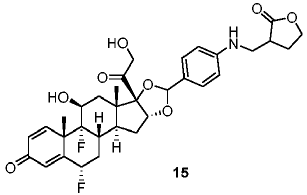

### 4-(((2-Oxotetrahydrofuran-3-yl)methyl)amino)benzaldehyde

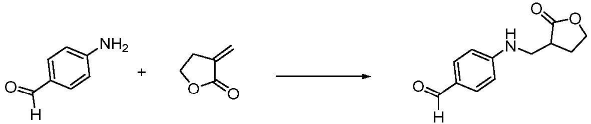

4-Aminobenzaldehyde (5 g, 0.04 mol), 3-methylenedihydrofuran-2(3*H*)-one (5 g, 0.05 mol), lithium tetrafluoroborate (0.75 g, 8 mmol), and 60 mL of methanol were added to a reaction flask, heated to reflux, and allowed to react for 12 hours. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (petroleum ether/ethyl acetate = 4/1) and concentrated under reduced pressure to obtain 4-(((2-oxotetrahydrofuran-3-yl)methyl)amino)benzaldehyde (2.8 g, yield: 32%). MS m/z (ESI): 220.11 [M+H]⁺

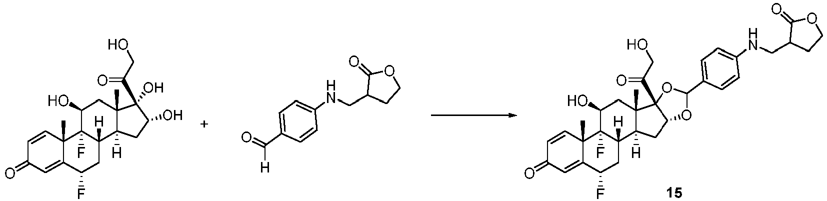

(6α,9α,11β,16α)-6,9-Difluoro-11,16,17,21-tetrahydroxy-pregna-1,4-diene-3,20-dione (0.5 g, 1.21 mmol), 4-(((2-oxotetrahydrofuran-3-yl)methyl)amino)benzaldehyde (0.32 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) to obtain compound **15** (0.36 g, yield: 48%). ¹H NMR (400 MHz, d₆-DMSO): δ 7.33 (m, 2H), 7.30 (s, 1H), 7.03 (m, 1H), 6.45 (m, 2H), 6.40 (m, 1H), 6.30 (s, 1H), 5.57-5.54 (m, 1H), 5.22 (s, 1H), 5.14 (s, 1H), 4.88-4.72 (m, 2H), 4.66-4.12 (m, 5H), 3.22-3.10 (m, 2H), 2.47 (m, 2H), 2.22 (m, 1H), 2.02-1.88 (m, 4H), 1.85-1.73 (m, 3H), 1.70-1.62 (m, 3H), 1.58-1.49 (m, 1H), 0.89-0.87 (m, 3H). MS m/z (ESI): 614.32 [M+H]⁺

### Synthetic Example 16

### (6aS,6bR,7S,8aS,8bS,11aR,12aS,12bS)-6b-Fluoro-7-hydroxy-8b-(2-hydroxyacetyl)-6a,8a-dimeth yl-10-(4-(((2-oxotetrahydrofuran-3-yl)oxy)methyl)phenyl)-1,2,6a,6b,7,8,8a,8b,11a,12,12a,12b-do decahydro-4H-naphtho[2',1':4,5]indeno[1,2-d][1,3]dioxol-4-one(16)

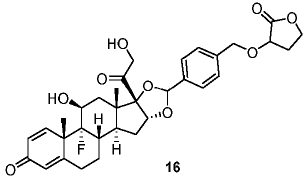

### 4-(((2-Oxotetrahydrofuran-3-yl)oxy)methyl)benzaldehyde

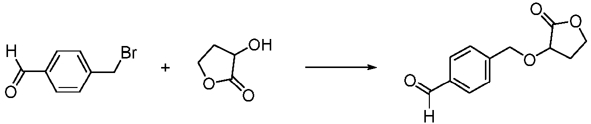

Sodium hydride (60%) (0.54 g, 13.5 mmol), 3-hydroxydihydrofuran-2(3*H*)-one (1.25 g, 12.3 mmol), and 50 mL of anhydrous tetrahydrofuran were added to a reaction flask, cooled to 0 °C, and allowed to react for 30 minutes. 4-(Bromomethyl)benzaldehyde (3 g, 15.3 mmol) and tetrabutylammonium iodide (0.45 g, 1.2 mmol) were added, and the mixture was allowed to react at room temperature for 3 hours. After completion of the reaction, water and dichloromethane were slowly added for extraction of the aqueous phase. The organic phases were combined and concentrated under reduced pressure. The crude product was separated and purified by silica gel column chromatography (petroleum ether/ethyl acetate = 6/4) and concentrated under reduced pressure to obtain 4-(((2-oxotetrahydrofuran-3-yl)oxy)methyl)benzaldehyde (1.5 g, yield: 55.3%). MS m/z (ESI): 221.18 [M+H]⁺

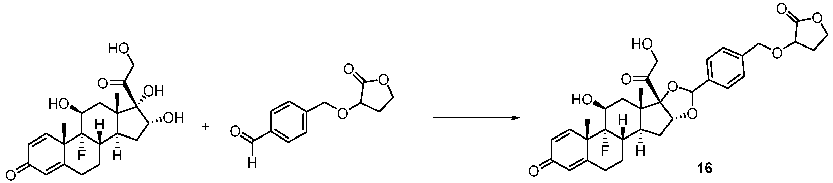

(9α,11β,16α)-9-Fluoro-11,16,17,21-tetrahydroxy-pregna-1,4-diene-3,20-dione (0.5 g, 1.27 mmol), 4-(((2-oxotetrahydrofuran-3-yl)oxy)methyl)benzaldehyde (0.33 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **16** (0.42 g, yield: 55%). ¹H NMR (400 MHz, d₆-DMSO): δ 7.45-7.21 (m, 4H), 7.25 (m 1H), 6.25-6.22 (m, 1H), 6.05 (s, 1H), 5.48-5.43 (m, 1H), 5.12-5.09 (m, 1H), 4.95-4.93 (m, 1H), 4.69 (m, 2H), 4.63 (m, 2H), 4.35-4.25 (m, 3H), 4.14-4.10 (m, 2H), 2.42-2.11 (m, 4H), 2.08-1.72 (m, 6H), 1.65 (m, 1H), 1.55-1.40 (m, 3H), 1.38-1.33 (m, 1H), 0.87 (m, 3H). MS m/z (ESI): 597.35 [M+H]⁺

### Synthetic Example 17

### (6aS,6bR,7S,8aS,8bS,11aR,12aS,12bS)-6b-Fluoro-7-hydroxy-8b-(2-hydroxyacetyl)-6a,8a-dimeth yl-10-(4-(((2-oxotetrahydrofuran-3-yl)thio)methyl)phenyl)-1,2,6a,6b,7,8,8a,8b,11a,12,12a,12b-do decahydro-4H-naphtho[2',1':4,5]indeno[1,2-d][1,3]dioxol-4-one (17)

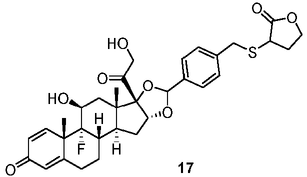

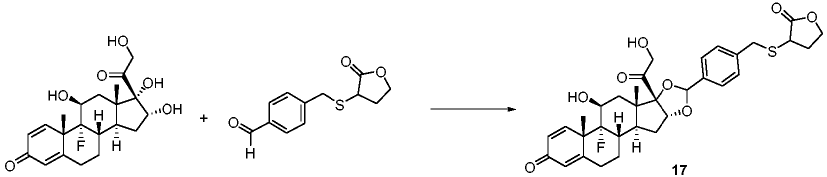

(9α,11β,16α)-9-Fluoro-11,16,17,21-tetrahydroxy-pregna-1,4-diene-3,20-dione (0.5 g, 1.27 mmol), 4-(((2-oxotetrahydrofuran-3-yl)thio)methyl)benzaldehyde (0.36 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **17** (0.45 g, yield: 58%). ¹H NMR (400 MHz, d₆-DMSO): δ 7.41-7.35 (m, 4H), 7.28 (d, *J*=10.0 Hz, 1H), 6.24-6.21 (m, 1H), 6.02 (s, 1H), 5.47-5.43 (m, 2H), 5.10-5.07 (m, 1H), 4.94-4.93 (m, 1H), 4.55-4.48 (m, 1H), 4.17-4.16 (m, 4H), 4.03-3.84 (m, 2H), 3.56-3.53 (m, 1H), 2.65-2.56 (m, 2H), 2.50-2.49 (m, 1H), 2.34-2.32 (m, 1H), 2.18-1.96 (m, 3H), 1.83 (m, 1H), 1.69-1.64 (m, 3H), 1.49-1.40 (m, 3H), 1.37-1.36 (m, 1H), 0.86 (m, 3H). MS m/z (ESI): 613.32 [M+H]⁺

### Synthetic Example 18

### (6aS,6bR,7S,8aS,8bS,11aR,12aS,12bS)-6b-Fluoro-7-hydroxy-8b-(2-hydroxyacetyl)-6a,8a-dimeth yl-10-(4-(((2-oxotetrahydrofuran-3-yl)amino)methyl)phenyl)-1,2,6a,6b,7,8,8a,8b,11a,12,12a,12b-dodecahydro-4H-naphtho[2',1':4,5]indeno[1,2-d][1,3]dioxol-4-one (18)

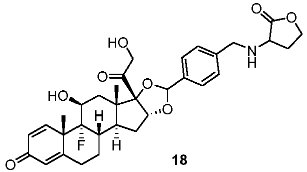

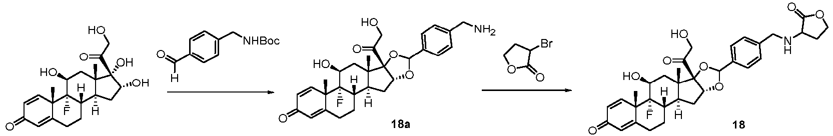

### Step 1

(9α,11β,16α)-9-Fluoro-11,16,17,21-tetrahydroxy-pregna-1,4-diene-3,20-dione (0.8 g, 2.03 mmol), tert-butyl (4-formylbenzyl)carbamate (0.72 g, 3.04 mmol), and 30 mL of acetonitrile were added to a reaction flask. Perchloric acid (0.5 mL, 6.08 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **18a** (0.6 g, yield: 58%).

### Step 2

3-Bromodihydrofuran-2(3H)-one (0.15 g, 0.94 mmol), compound **18a** (0.48 g, 0.94 mmol), potassium phosphate (0.6 g, 2.82 mmol), and 50 mL of acetonitrile were added to a reaction flask and allowed to react at room temperature. TLC monitoring was performed until no compound **18a** was detected. After completion of the reaction, the mixture was filtered under reduced pressure. The filtrate was concentrated under reduced pressure. Dichloromethane was added. The organic phase was washed successively with water and saturated brine and concentrated under reduced pressure. The crude product was separated and purified by silica gel column chromatography (dichloromethane/acetone = 3/1) and concentrated under reduced pressure to obtain compound **18** (0.14 g, yield: 25%). ¹H NMR (400 MHz, d₆-DMSO): δ 7.45-7.27 (m, 4H), 7.23 (m 1H), 6.30-6.25 (m, 1H), 6.10 (s, 1H), 5.50-5.48 (m, 1H), 5.15-5.10 (m, 1H), 4.98-4.95 (m, 1H), 4.72 (m, 2H), 4.23 (m, 1H), 4.17-4.05 (m, 4H), 3.82 (m, 2H), 3.52 (m, 1H), 2.32-2.11 (m, 4H), 2.06-1.72 (m, 6H), 1.70 (m, 1H), 1.65-1.56 (m, 3H), 1.45-1.38 (m, 1H), 0.95 (m, 3H). MS m/z (ESI): 596.37 [M+H]⁺

### Synthetic Example 19

### (6aS,6bR,7S,8aS,8bS,11aR,12aS,12bS)-6b-Fluoro-7-hydroxy-8b-(2-hydroxyacetyl)-6a,8a-dimeth yl-10-(4-(((5-oxotetrahydrofuran-3-yl)oxy)methyl)phenyl)-1,2,6a,6b,7,8,8a,8b,11a,12,12a,12b-do decahydro-4H-naphtho[2',1':4,5]indeno[1,2-d][1,3]dioxol-4-one (19)

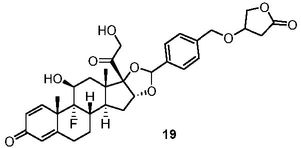

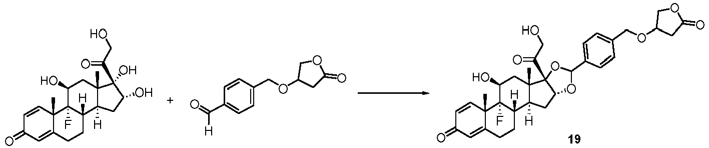

(9α,11β,16α)-9-Fluoro-11,16,17,21-tetrahydroxy-pregna-1,4-diene-3,20-dione (0.5 g, 1.27 mmol), 4-(((5-oxotetrahydrofuran-3-yl)oxy)methyl)benzaldehyde (0.33 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **19** (0.46 g, yield: 61%). ¹H NMR (400 MHz, d₆-DMSO): δ 7.44-7.21 (m, 4H), 7.27 (m 1H), 6.27-6.25 (m, 1H), 6.07 (s, 1H), 5.50-5.45 (m, 1H), 5.11-5.08 (m, 1H), 4.95-4.93 (m, 1H), 4.69 (m, 2H), 4.63 (m, 2H), 4.55-4.30 (m, 3H), 4.14-3.95 (m, 2H), 2.56-2.32 (m, 4H), 2.05-1.72 (m, 6H), 1.69 (m, 1H), 1.60-1.53 (m, 3H), 1.47-1.39 (m, 1H), 092 (m, 3H). MS m/z (ESI): 597.33 [M+H]⁺

### Synthetic Example 20

### (6aS,6bR,7S,8aS,8bS,11aR,12aS,12bS)-10-(2-Chloro-4-(((5-oxotetrahydrofuran-3-yl)thio)methyl) phenyl)-6b-fluoro-7-hydroxy-8b-(2-hydroxyacetyl)-6a,8a-dimethyl-1,2,6a,6b,7,8,8a,8b,11a,12,12a, 12b-dodecahydro-4H-naphtho[2',1':4,5]indeno[1,2-d][1,3]dioxol-4-one (20)

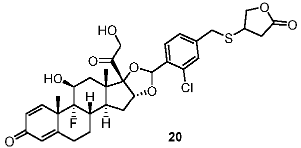

### 2-Chloro-4-(((5-oxotetrahydrofuran-3-yl)thio)methyl)benzaldehyde

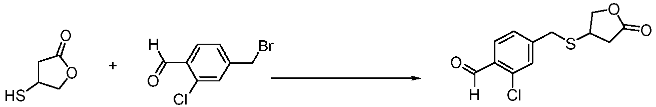

4-Mercaptodihydrofuran-2(3*H*)-one (3.5 g, 0.03 mol), 4-(bromomethyl)-2-chlorobenzaldehyde (7.7 g, 0.033 mol), potassium carbonate (4.8 g, 0.035 mol), and 60 mL of N,Ndimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 2-chloro-4-(((5-oxotetrahydrofuran-3-yl)thio)methyl)benzaldehyde (5.3 g, yield: 65%). MS m/z (ESI): 271.08 [M+H]⁺

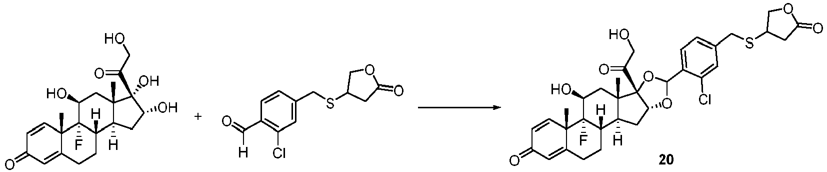

(9α,11β,16α)-9-Fluoro-11,16,17,21-tetrahydroxy-pregna-1,4-diene-3,20-dione (0.5 g, 1.27 mmol), 2-chloro-4-(((5-oxotetrahydrofuran-3-yl)thio)methyl)benzaldehyde (0.41 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **20** (0.47 g, yield: 57%). ¹H NMR (400 MHz, d₆-DMSO): δ 7.44-7.37 (m, 3H), 7.26 (m, 1H), 6.30-6.27 (m, 1H), 6.05 (s, 1H), 5.50-5.47 (m, 1H), 5.12-5.09 (m, 1H), 4.95-4.93 (m, 1H), 4.75-4.50 (m, 2H), 4.48-4.41 (m, 2H), 4.32-4.14 (m, 2H), 3.70 (s, 2H), 3.14 (m, 1H), 2.75-2.52 (m, 2H), 2.42-2.32 (m, 2H), 2.19-1.96 (m, 2H), 1.85 (m, 2H), 1.70-1.66 (m, 3H), 1.52-1.44 (m, 3H), 1.39-1.37 (m, 1H), 0.87 (m, 3H). MS m/z (ESI): 647.22 [M+H]⁺

### Synthetic Example 21

### (6aS,6bR,7S,8aS,8bS,11aR,12aS,12bS)-6b-Fluoro-7-hydroxy-8b-(2-hydroxyacetyl)-6a,8a-dimeth yl-10-(4-(((5-oxotetrahydrofuran-3-yl)amino)methyl)phenyl)-1,2,6a,6b,7,8,8a,8b,11a,12,12a,12b-dodecahydro-4H-naphtho[2',1':4,5]indeno[1,2-d][1,3]dioxol-4-one (21)

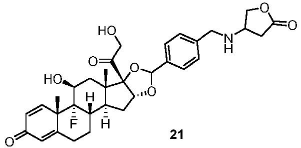

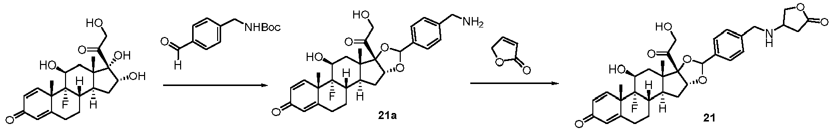

### Step 1

(9α,11β,16α)-9-Fluoro-11,16,17,21-tetrahydroxy-pregna-1,4-diene-3,20-dione (0.8 g, 2.03 mmol), tert-butyl (4-formylbenzyl)carbamate (0.72 g, 3.04 mmol), and 30 mL of acetonitrile were added to a reaction flask. Perchloric acid (0.5 mL, 6.08 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **21a** (0.64 g, yield: 62%).

### Step 2

Furan-2(5*H*)-one (0.12 g, 1.42 mmol), compound **21a** (0.48 g, 0.94 mmol), and 30 mL of methanol were added to a reaction flask and allowed to react at room temperature. TLC monitoring was performed until no compound **21a** was detected. After completion of the reaction, the mixture was concentrated under reduced pressure. The crude product was separated and purified by silica gel column chromatography (dichloromethane/acetone = 3/1) and concentrated under reduced pressure to obtain compound **21** (0.20 g, yield: 35%). ¹H NMR (400 MHz, d₆-DMSO): δ 7.43-7.27 (m, 4H), 7.22 (m 1H), 6.32-6.25 (m, 2H), 6.12 (s, 1H), 5.51-5.49 (m, 1H), 5.15-5.12 (m, 1H), 4.98-4.96 (m, 1H), 4.75 (m, 2H), 4.50-4.35 (m, 2H), 4.32-4.15 (m, 2H), 3.85 (m, 2H), 3.23 (m, 1H), 2.52-2.22 (m, 4H), 2.06-1.75 (m, 6H), 1.68 (m, 1H), 1.63-1.54 (m, 3H), 1.48-1.40 (m, 1H), 0.98 (m, 3H). MS m/z (ESI): 596.32 [M+H]⁺

### Synthetic Example 22

### (6aS,6bR,7S,8aS,8bS,11aR,12aS,12bS)-6b-Chloro-7-hydroxy-8b-(2-hydroxyacetyl)-6a,8a-dimeth yl-10-(4-(((2-oxotetrahydrofuran-3-yl)oxy)methyl)phenyl)-1,2,6a,6b,7,8,8a,8b,11a,12,12a,12b-do decahydro-4H-naphtho[2',1':4,5]indeno[1,2-d][1,3]dioxol-4-one (22)

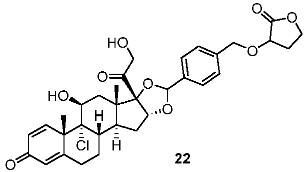

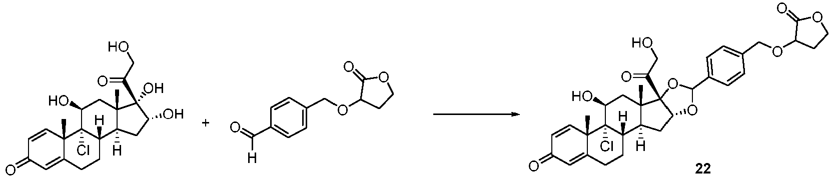

(9α,11β,16α)-9-Chloro-11,16,17,21-tetrahydroxy-pregna-1,4-diene-3,20-dione (0.5 g, 1.22 mmol), 4-(((2-oxotetrahydrofuran-3-yl)oxy)methyl)benzaldehyde (0.32 g, 1.46 mmol), and 30 mL of acetonitrile were added to a reaction flask. Perchloric acid (0.31 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **22** (0.42 g, yield: 56%). ¹H NMR (400 MHz, d₆-DMSO): δ 7.48 (m, 2H), 7.38-7.33 (m, 2H), 7.27 (m, 1H), 6.26-6.24 (m, 1H), 6.01 (s, 1H), 5.60-5.58 (m, 1H), 5.50 (s, 1H), 5.10-5.03 (m, 1H), 4.93-4.92 (m, 1H), 4.47-4.39 (m,2H), 4.30-4.26 (m,2H), 4.21-4.12 (m, 3H), 3.55-3.52 (m, 1H), 2.79-2.32 (m, 5H), 2.01-1.95 (m, 2H), 1.84-1.79 (m, 1H), 1.78-1.56 (m, 7H), 0.85 (s, 3H). MS m/z (ESI): 613.25 [M+H]⁺

### Synthetic Example 23

### (6aS,6bR,7S,8aS,8bS,11aR,12aS,12bS)-6b-Chloro-7-hydroxy-8b-(2-hydroxyacetyl)-6a,8a-dimeth yl-10-(4-(((2-oxotetrahydrofuran-3-yl)thio)methyl)phenyl)-1,2,6a,6b,7,8,8a,8b,11a,12,12a,12b-do decahydro-4H-naphtho[2',1':4,5]indeno[1,2-d][1,3]dioxol-4-one (23)

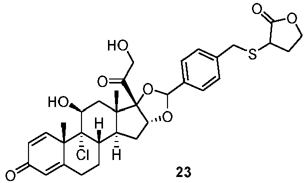

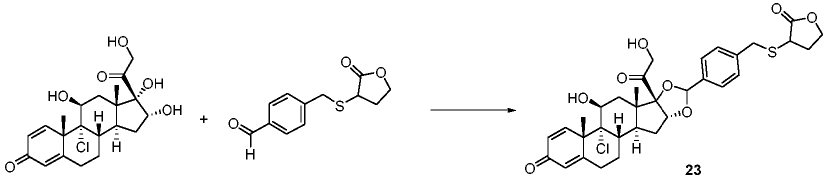

(9α,11β,16α)-9-Chloro-11,16,17,21-tetrahydroxy-pregna-1,4-diene-3,20-dione (0.5 g, 1.22 mmol), 4-(((2-oxotetrahydrofuran-3-yl)thio)methyl)benzaldehyde (0.35 g, 1.46 mmol), and 30 mL of acetonitrile were added to a reaction flask. Perchloric acid (0.31 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **23** (0.52 g, yield: 68%). ¹H NMR (400 MHz, d₆-DMSO): δ 7.46 (d, J =8.4 Hz, 2H), 7.36-7.31 (m, 2H), 7.28 (m, 1H), 6.25-6.21 (m, 1H), 5.99 (s, 1H), 5.61-5.60 (m, 1H), 5.49 (s, 1H), 5.09-5.01 (m, 1H), 4.93-4.92 (m, 1H), 4.47-4.39 (m,2H), 4.28-4.16 (m, 3H), 4.00-3.84 (m, 2H), 3.56-3.53 (m, 1H), 2.75-2.33 (m, 5H), 2.01-1.96 (m, 2H), 1.81-1.79 (m, 1H), 1.76-1.56 (m, 7H), 0.82 (s, 3H). MS m/z (ESI): 629.22 [M+H]⁺

### Synthetic Example 24

### (6aS,6bR,7S,8aS,8bS,11aR,12aS,12bS)-6b-Chloro-7-hydroxy-8b-(2-hydroxyacetyl)-6a,8a-dimeth yl-10-(4-(((2-oxotetrahydrofuran-3-yl)amino)methyl)phenyl)-1,2,6a,6b,7,8,8a,8b,11a,12,12a,12b-dodecahydro-4H-naphtho[2',1':4,5]indeno[1,2-d][1,3]dioxol-4-one (24)

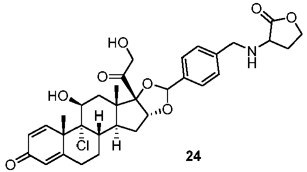

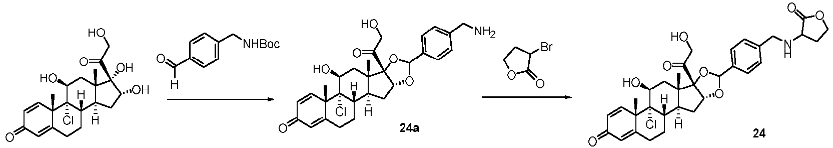

### Step 1

(9α,11β,16α)-9-Chloro-11,16,17,21-tetrahydroxy-pregna-1,4-diene-3,20-dione (0.8 g, 1.95 mmol), tert-butyl (4-formylbenzyl)carbamate (0.69 g, 2.92 mmol), and 30 mL of acetonitrile were added to a reaction flask. Perchloric acid (0.5 mL, 5.84 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **24a** (0.65 g, yield: 63%).

### Step 2

3-Bromodihydrofuran-2(3*H*)-one (0.15 g, 0.94 mmol), compound **24a** (0.5 g, 0.94 mmol), potassium phosphate (0.6 g, 2.82 mmol), and 50 mL acetonitrile were added to a reaction flask and allowed to react at room temperature. TLC monitoring was performed until no compound **24a** was detected. After completion of the reaction, the mixture was filtered under reduced pressure. The filtrate was concentrated under reduced pressure. Dichloromethane was added. The organic phase was washed successively with water and saturated brine and concentrated under reduced pressure. The crude product was separated and purified by silica gel column chromatography (dichloromethane/acetone = 3/1) to obtain compound **24** (0.16 g, yield: 28%). ¹H NMR (400 MHz, d₆-DMSO): δ 7.45 (d, J =8.4 Hz, 2H), 7.36-7.30 (m, 2H), 7.26 (m, 1H), 6.27-6.23 (m, 1H), 6.00 (s, 1H), 5.62-5.60 (m, 1H), 5.51 (s, 1H), 5.10-5.03 (m, 1H), 4.92-4.90 (m, 1H), 4.45-4.39 (m,2H), 4.28-4.15 (m, 4H), 3.84-3.64 (m, 2H), 3.52-3.48 (m, 1H), 2.73-2.37 (m, 5H), 2.00-1.96 (m, 2H), 1.82-1.79 (m, 1H), 1.77-1.56 (m, 7H), 0.86 (s, 3H). MS m/z (ESI): 612.34 [M+H]⁺

### Synthetic Example 25

### (6aS,6bR,7S,8aS,8bS,11aR,12aS,12bS)-6b-Chloro-7-hydroxy-8b-(2-hydroxyacetyl)-6a,8a-dimeth yl-10-(4-(((5-oxotetrahydrofuran-3-yl)oxy)methyl)phenyl)-1,2,6a,6b,7,8,8a,8b,11a,12,12a,12b-do decahydro-4H-naphtho[2',1':4,5]indeno[1,2-d][1,3]dioxol-4-one (25)

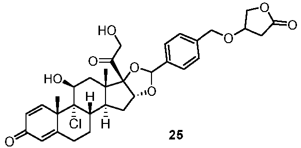

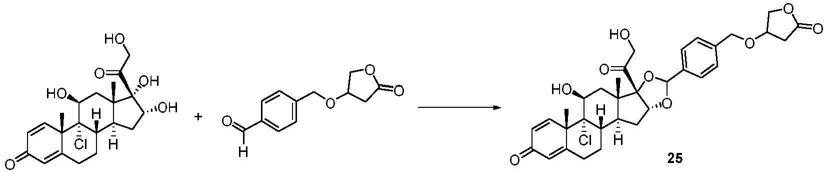

(9α,11β,16α)-9-Chloro-11,16,17,21-tetrahydroxy-pregna-1,4-diene-3,20-dione (0.5 g, 1.22 mmol), 4-(((5-oxotetrahydrofuran-3-yl)oxy)methyl)benzaldehyde (0.32 g, 1.46 mmol), and 30 mL of acetonitrile were added to a reaction flask. Perchloric acid (0.31 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **25** (0.35 g, yield: 47%). ¹H NMR (400 MHz, d₆-DMSO): δ 7.45 (m, 2H), 7.35-7.30 (m, 2H), 7.26 (m, 1H), 6.28-6.24 (m, 1H), 6.00 (s, 1H), 5.59-5.57 (m, 1H), 5.52 (s, 1H), 5.15-5.09 (m, 1H), 4.73-4.55 (m, 2H), 4.52-4.30 (m,3H), 4.27-4.15 (m,2H), 4.11-4.05 (m,2H), 2.56-2.31 (m, 4H), 2.01-1.96 (m, 2H), 1.84-1.73 (m, 5H), 1.62-1.43 (m, 4H), 0.87 (s, 3H). MS m/z (ESI): 613.33 [M+H]⁺

### Synthetic Example 26

### (6aS,6bR,7S,8aS,8bS,11aR,12aS,12bS)-6b-Chloro-7-hydroxy-8b-(2-hydroxyacetyl)-6a,8a-dimeth yl-10-(4-(((5-oxotetrahydrofuran-3-yl)thio)methyl)phenyl)-1,2,6a,6b,7,8,8a,8b,11a,12,12a,12b-do decahydro-4H-naphtho[2',1':4,5]indeno[1,2-d][1,3]dioxol-4-one (26)

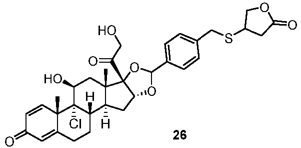

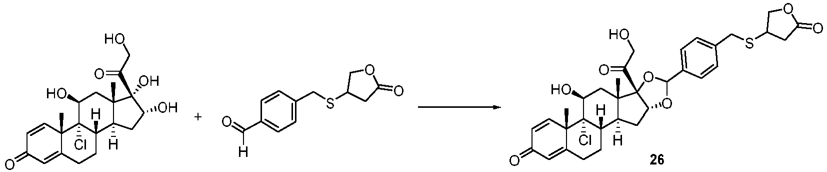

(9α,11β,16α)-9-Chloro-11,16,17,21-tetrahydroxy-pregna-1,4-diene-3,20-dione (0.5 g, 1.22 mmol), 4-(((5-oxotetrahydrofuran-3-yl)thio)methyl)benzaldehyde (0.35 g, 1.46 mmol), and 30 mL of acetonitrile were added to a reaction flask. Perchloric acid (0.31 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **26** (0.42 g, yield: 55%). ¹H NMR (400 MHz, d₆-DMSO): δ 7.45 (d, J =8.4 Hz, 2H), 7.36-7.33 (m, 2H), 7.27 (m, 1H), 6.23-6.25 (m, 1H), 6.02 (s, 1H), 5.60-5.58 (m, 1H), 5.50 (s, 1H), 5.12-5.05 (m, 1H), 4.72 (s,2H), 4.70-4.50 (m, 2H), 4.32-4,21 (m, 1H), 3.85-3.70 (m, 3H), 3.24-3.14 (m, 1H), 2.75-2.50 (m, 2H), 2.42-2.30 (m, 2H), 1.97-1.72 (m, 2H), 1.70-1.46 (m, 4H), 1.43-1.39 (m, 4H), 1.07-1.04 (m,1H), 0.89 (s, 3H). MS m/z (ESI): 629.23 [M+H]⁺

### Synthetic Example 27

### (6aS,6bR,7S,8aS,8bS,11aR,12aS,12bS)-6b-Chloro-7-hydroxy-8b-(2-hydroxyacetyl)-6a,8a-dimeth yl-10-(4-(((5-oxotetrahydrofuran-3-yl)amino)methyl)phenyl)-1,2,6a,6b,7,8,8a,8b,11a,12,12a,12b-dodecahydro-4H-naphtho[2',1':4,5]indeno[1,2-d][1,3]dioxol-4-one (27)

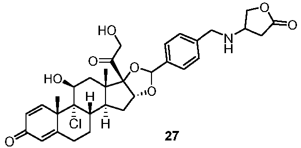

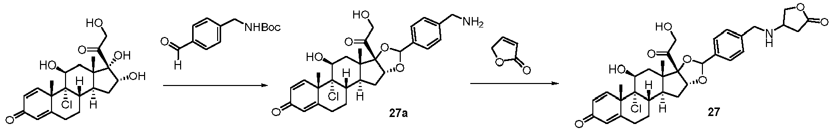

### Step 1

(9α,11β,16α)-9-Chloro-11,16,17,21-tetrahydroxy-pregna-1,4-diene-3,20-dione (0.8 g, 1.95 mmol), tert-butyl (4-formylbenzyl)carbamate (0.69 g, 2.92 mmol), and 30 mL of acetonitrile were added to a reaction flask. Perchloric acid (0.5 mL, 5.84 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **27a** (0.64 g, yield: 62%).

### Step 2

Furan-2(5*H*)-one (0.12 g, 1.42 mmol), compound **27a** (0.5 g, 0.94 mmol), and 30 mL of methanol were added to a reaction flask and allowed to react at room temperature. TLC monitoring was performed until no compound **27a** was detected. After completion of the reaction, the mixture was concentrated under reduced pressure. The crude product was separated and purified by silica gel column chromatography (dichloromethane/acetone = 3/1) and concentrated under reduced pressure to obtain compound **27** (0.2 g, yield: 34%). ¹H NMR (400 MHz, d₆-DMSO): δ 7.44 (d, J =8.4 Hz, 2H), 7.35-7.30 (m, 2H), 7.28 (m, 1H), 6.36 (m, 1H), 6.25-6.23 (m, 1H), 6.02 (s, 1H), 5.60-5.58 (m, 1H), 5.51 (s, 1H), 5.10-5.03 (m, 1H), 4.72-4.70 (m, 2H), 4.47-4.39 (m,3H), 3.82-3.65 (m, 3H), 3.25 (m, 1H), 2.53-2.27 (m, 4H), 1.97-1.76 (m, 2H), 1.70-1.45 (m, 5H), 1.43 (m, 3H), 1.04 (m, 1H), 0.86 (s, 3H). MS m/z (ESI): 612.35 [M+H]⁺

### Synthetic Example 28

### (6aR,6bS, 7S,8aS,8bS,11aR,12aS,12bS)-7-Hydroxy-8b-(2-hydroxyacetyl)-6a,8a-dimethyl-10-(4-(( (2-oxotetrahydrofuran-3-yl)oxy)methyl)phenyl)-1,2,6a,6b,7,8,8a,8b,11a,12,12a,12b-dodecahydro-4H-naphtho[2',1':4,5]indeno[1,2-d][1,3]dioxol-4-one (28)

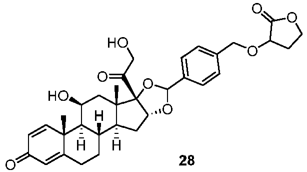

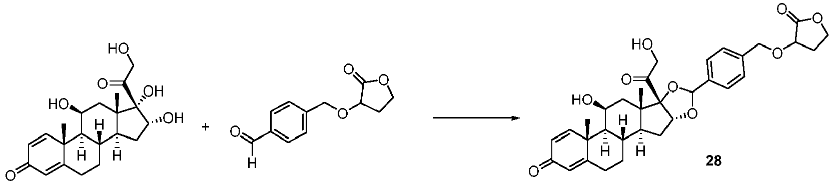

(11β,16α)-11,16,17,21-Tetrahydroxy-pregna-1,4-diene-3,20-dione (0.5 g, 1.33 mmol), 4-(((2-oxotetrahydrofuran-3-yl)oxy)methyl)benzaldehyde (0.35 g, 1.59 mmol), and 30 mL of acetonitrile were added to a reaction flask. Perchloric acid (0.34 mL, 4 mmol) was slowly added dropwise, and the mixture was allowed to react at room temperature. TLC monitoring was performed until no (11β,16α)-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **28** (0.37 g, yield: 48%). ¹H NMR (400 MHz, d₆-DMSO): δ 7.45 (m, 2H), 7.40-7.36 (m, 2H), 7.27 (m, 1H), 6.25 (m, 1H), 6.02 (s, 1H), 5.58-5.56 (m, 1H), 5.50 (s, 1H), 5.15 (m, 1H), 4.72-4.69 (m,2H), 4.63 (s, 2H), 4.35-4.25 (m,3H), 4.18-4.12 (m, 1H), 3.44-3.42 (m, 1H), 2.42-2.12 (m, 4H), 2.00-1.96 (m, 2H), 1.88-1.79 (m, 4H), 1.78-1.56 (m, 4H), 1.38-1.14 (m. 2H), 0.83 (s, 3H). MS m/z (ESI): 579.34 [M+H]⁺

### Synthetic Example 29

### (6aR,6bS, 7S,8aS,8bS,11aR,12aS,12bS)-7-Hydroxy-8b-(2-hydroxyacetyl)-6a,8a-dimethyl-10-(4-(( (2-oxotetrahydrofuran-3-yl)thio)methyl)phenyl)-1,2,6a,6b,7,8,8a,8b,11a,12,12a,12b-dodecahydro-4H-naphtho[2',1':4,5]indeno[1,2-d][1,3]dioxol-4-one (29)

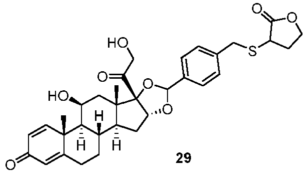

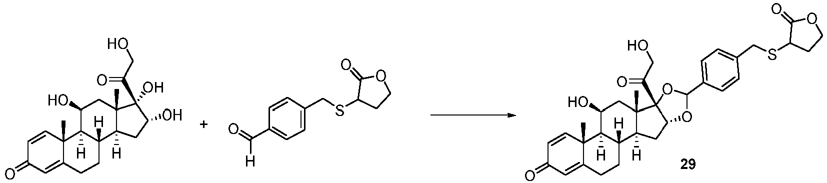

(11β,16α)-11,16,17,21-Tetrahydroxy-pregna-1,4-diene-3,20-dione (0.5 g, 1.33 mmol), 4-(((2-oxotetrahydrofuran-3-yl)thio)methyl)benzaldehyde (0.38 g, 1.59 mmol), and 30 mL of acetonitrile were added to a reaction flask. Perchloric acid (0.34 mL, 4 mmol) was slowly added dropwise, and the mixture was allowed to react at room temperature. TLC monitoring was performed until no (11β,16α)-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **29** (0.4 g, yield: 51%). ¹H NMR (400 MHz, d₆-DMSO): δ 7.45 (d, J =8.4 Hz, 2H), 7.36-7.30 (m, 2H), 7.27 (m, 1H), 6.25-6.22 (m, 1H), 6.01 (s, 1H), 5.60-5.59 (m, 1H), 5.52 (s, 1H), 5.10-5.01 (m, 1H), 4.45-4.24 (m,4H), 3.56-3.34 (m, 4H), 2.75-2.32 (m, 4H), 2.01-1.79 (m, 7H), 1.70-1.56 (m, 4H), 1.32-1.04 (m, 2H), 0.85 (s, 3H). MS m/z (ESI): 595.35 [M+H]⁺

### Synthetic Example 30

### (6aR,6bS, 7S,8aS,8bS,11aR,12aS,12bS)-7-Hydroxy-8b-(2-hydroxyacetyl)-6a,8a-dimethyl-10-(4-(( (2-oxotetrahydrofuran-3-yl)amino)methyl)phenyl)-1,2,6a,6b,7,8,8a,8b,11a,12,12a,12b-dodecahydr o-4H-naphtho[2',1':4,5]indeno[1,2-d][1,3]dioxol-4-one (30)

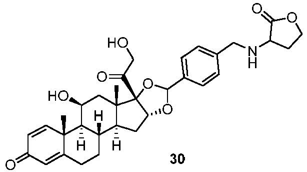

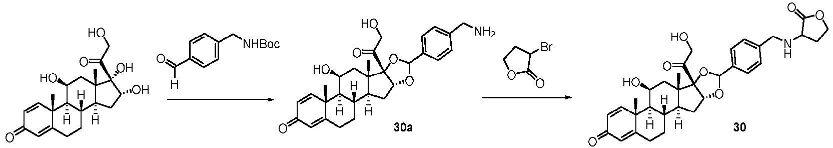

### Step 1

(11β,16α)-11,16,17,21-Tetrahydroxy-pregna-1,4-diene-3,20-dione (0.8 g, 2.13 mmol), tert-butyl (4-formylbenzyl)carbamate (0.75 g, 3.19 mmol), and 30 mL of acetonitrile were added to a reaction flask. Perchloric acid (0.54 mL, 6.38 mmol) was slowly added dropwise, and the mixture was allowed to react at room temperature. TLC monitoring was performed until no (11β,16α)-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **30a** (0.71 g, yield: 68%).

### Step 2

3-Bromodihydrofuran-2(3*H*)-one (0.15 g, 0.94 mmol), compound **30a** (0.46 g, 0.94 mmol), potassium phosphate (0.6 g, 2.82 mmol), and 50 mL acetonitrile were added to a reaction flask and allowed to react at room temperature. TLC monitoring was performed until no compound **30a** was detected. After completion of the reaction, the mixture was filtered under reduced pressure. The filtrate was concentrated under reduced pressure. Dichloromethane was added. The organic phase was washed successively with water and saturated brine and concentrated under reduced pressure. The crude product was separated and purified by silica gel column chromatography (dichloromethane/acetone = 3/1) and concentrated under reduced pressure to obtain compound **30** (0.19 g, yield: 35%). ¹H NMR (400 MHz, d₆-DMSO): δ 7.46 (d, J =8.4 Hz, 2H), 7.36-7.32 (m, 2H), 7.26 (m, 1H), 6.25-6.21 (m, 1H), 6.00 (s, 1H), 5.61-5.99 (m, 1H), 5.50 (s, 1H), 5.10-5.02 (m, 1H), 4.75-4.69 (m,2H), 4.35-4.21 (m, 3H), 4.16-4.12 (m,1H), 3.84-3.82 (m, 2H), 3.44-3.43 (m, 2H), 2.43-2.07 (m, 4H), 2.00-1.97 (m, 2H), 1.82-1.78 (m, 5H), 1.75-1.35 (m, 3H), 1.12-1.04 (m, 2H), 0.86 (s, 3H). MS m/z (ESI): 578.34 [M+H]⁺

### Synthetic Example 31

### (6aR,6bS, 7S,8aS,8bS,11aR,12aS,12bS)-7-Hydroxy-8b-(2-hydroxyacetyl)-6a,8a-dimethyl-10-(4-(( (5-oxotetrahydrofuran-3-yl)oxy)methyl)phenyl)-1,2,6a,6b,7,8,8a,8b,11a,12,12a,12b-dodecahydro-4H-naphtho[2',1':4,5]indeno[1,2-d][1,3]dioxol-4-one (31)

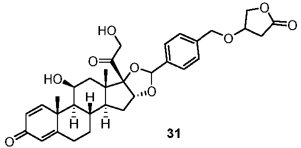

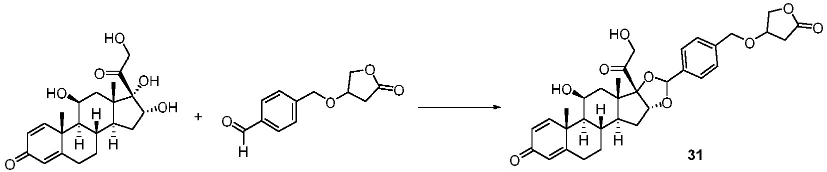

(11β,16α)-11,16,17,21-Tetrahydroxy-pregna-1,4-diene-3,20-dione (0.5 g, 1.33 mmol), 4-(((5-oxotetrahydrofuran-3-yl)oxy)methyl)benzaldehyde (0.35 g, 1.59 mmol), and 30 mL of acetonitrile were added to a reaction flask. Perchloric acid (0.34 mL, 4 mmol) was slowly added dropwise, and the mixture was allowed to react at room temperature. TLC monitoring was performed until no (11β,16α)-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **31** (0.48 g, yield: 62%). ¹H NMR (400 MHz, d₆-DMSO): δ 7.45 (m, 2H), 7.38-7.36 (m, 2H), 7.26 (m, 1H), 6.27 (m, 1H), 6.00 (s, 1H), 5.60-5.58 (m, 1H), 5.51 (s, 1H), 5.15 (m, 1H), 4.70-4.67 (m,2H), 4.63-4.60 (s, 2H), 4.55-4.30 (m,3H), 4.14-4.12 (m, 1H), 3.90 (m, 1H), 3.44-3.42 (m, 1H), 2.40-2.11 (m, 4H), 2.00-1.95 (m, 2H), 1.89-1.78 (m, 4H), 1.76-1.58 (m, 3H), 1.42-1.16 (m. 2H), 0.86 (s, 3H). MS m/z (ESI): 579.33 [M+H]⁺

### Synthetic Example 32

### 2-((6aR,6bS,7S,8aS,8bS,11aR,12aS,12bS)-7-Hydroxy-8b-(2-hydroxyacetyl)-6a,8a-dimethyl-4-ox o-2,4,6a,6b,7,8,8a,8b,11a,12,12a,12b-dodecahydro-1H-naphtho[2',1':4,5]indeno[1,2-d][1,3]dioxol-10-yl)-5-(((5-oxotetrahydrofuran-3-yl)thio)methyl)benzonitrile (32)

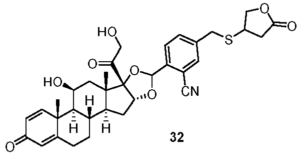

### 2-Formyl-5-(((5-oxotetrahydrofuran-3-yl)thio)methyl)benzonitrile

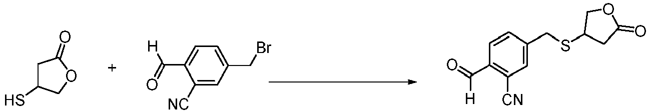

4-Mercaptodihydrofuran-2(3*H*)-one (3.5 g, 0.03 mol), 5-(bromomethyl)-2-formylbenzonitrile (7.4 g, 0.033 mol), potassium carbonate (4.8 g, 0.035 mol), and 60 mL of *N,N*-dimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 2-formyl-5-(((5-oxotetrahydrofuran-3-yl)thio)methyl)benzonitrile (4.3 g, yield: 55%). MS m/z (ESI): 262.12 [M+H]⁺

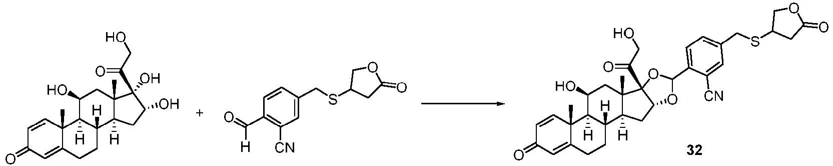

(11β,16α)-11,16,17,21-Tetrahydroxy-pregna-1,4-diene-3,20-dione (0.5 g, 1.33 mmol), 2-formyl-5-(((5-oxotetrahydrofuran-3-yl)thio)methyl)benzonitrile (0.42 g, 1.59 mmol), and 30 mL of acetonitrile were added to a reaction flask. Perchloric acid (0.34 mL, 4 mmol) was slowly added dropwise, and the mixture was allowed to react at room temperature. TLC monitoring was performed until no (11β,16α)-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **32** (0.49 g, yield: 60%). ¹H NMR (400 MHz, d₆-DMSO): δ 7.46 (s, 1H), 7.36-7.32 (m, 2H), 7.27 (m, 1H), 6.25-6.21 (m, 1H), 6.00 (s, 1H), 5.62-5.60 (m, 1H), 5.53 (s, 1H), 5.10-5.00 (m, 1H), 4.75-4.50 (m,4H), 4.32 (m, 1H), 3.70 (s, 2H), 3.44 (m, 1H), 3.14 (s, 1H), 2.74-2.30 (m, 4H), 1.98-1.75 (m, 7H), 1.67-1.56 (m, 3H), 1.30-1.05 (m, 2H), 0.87 (s, 3H). MS m/z (ESI): 620.33 [M+H]⁺

### Synthetic Example 33

### (6aR,6bS, 7S,8aS,8bS,11aR,12aS,12bS)-7-Hydroxy-8b-(2-hydroxyacetyl)-6a,8a-dimethyl-10-(4-(( (5-oxotetrahydrofuran-3-yl)amino)methyl)phenyl)-1,2,6a,6b,7,8,8a,8b,11a,12,12a,12b-dodecahydr o-4H-naphtho[2',1':4,5]indeno[1,2-d][1,3]dioxol-4-one (33)

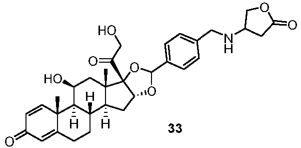

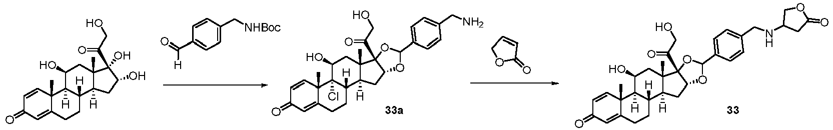

### Step 1

(11β,16α)-11,16,17,21-Tetrahydroxy-pregna-1,4-diene-3,20-dione (0.8 g, 2.13 mmol), tert-butyl (4-formylbenzyl)carbamate (0.75 g, 3.19 mmol), and 30 mL of acetonitrile were added to a reaction flask. Perchloric acid (0.54 mL, 6.38 mmol) was slowly added dropwise, and the mixture was allowed to react at room temperature. TLC monitoring was performed until no (11β,16α)-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **33a** (0.61 g, yield: 58%).

### Step 2

Furan-2(5*H*)-one (0.12 g, 1.42 mmol), compound **33a** (0.46 g, 0.94 mmol), and 30 mL of methanol were added to a reaction flask and allowed to react at room temperature. TLC monitoring was performed until no compound **33a** was detected. After completion of the reaction, the mixture was concentrated under reduced pressure. The crude product was separated and purified by silica gel column chromatography (dichloromethane/acetone = 3/1) and concentrated under reduced pressure to obtain compound **33** (0.17 g, yield: 32%). ¹H NMR (400 MHz, d₆-DMSO): δ 7.47 (d, J =8.4 Hz, 2H), 7.35-7.32 (m, 2H), 7.28 (m, 1H), 6.36 (m, 1H), 6.25-6.22 (m, 1H), 6.02 (s, 1H), 5.60-5.98 (m, 1H), 5.52 (s, 1H), 5.13-5.05 (m, 1H), 4.74-4.69 (m,2H), 4.50-4.25 (m, 3H), 4.06-3.98 (m,2H), 3.44 (m, 1H), 3.23 (m, 1H), 2.43-2.06 (m, 4H), 1.99-172 (m, 2H), 1.72-1.35 (m, 4H), 1.35-1.15 (m, 4H), 1.07-1.02 (m, 2H), 0.86 (s, 3H). MS m/z (ESI): 578.33 [M+H]⁺

### Synthetic Example 34

### S-(Fluoromethyl)

### (2S,6aS,6bR,7S,8aS,8bS,11aR,12aS,12bS)-2,6b-difluoro-7-hydroxy-6a,8a-dimethyl-4-oxo-10-(4-( ((2-oxotetrahydrofuran-3-yl)thio)methyl)phenyl)-1,2,4,6a,6b,7,8,8a,11a,12,12a,12b-dodecahydro-8bH-naphtho[2',1':4,5]indeno[1,2-d][1,3]dioxole-8b-carbothioate (34)

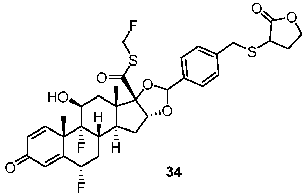

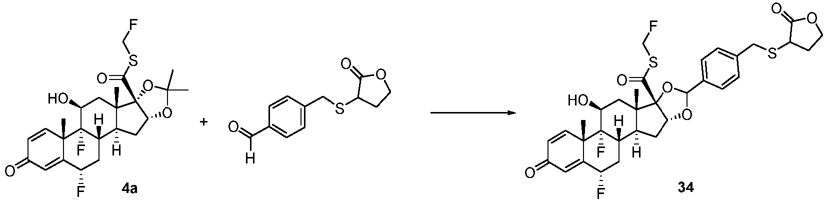

Compound **4a** (0.5 g, 1 mmol), 4-(((2-oxotetrahydrofuran-3-yl)thio)methyl)benzaldehyde (0.3 g, 1.23 mmol), and 30 mL of acetonitrile were added to a reaction flask. Perchloric acid (0.26 mL, 3.1 mmol) was slowly added dropwise, and the mixture was allowed to react at room temperature. TLC monitoring was performed until no compound **4a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **34** (0.31 g, yield: 45%). ¹H NMR (400 MHz, d₆-DMSO): δ 7.43-7.35 (m, 4H), 7.27 (d, *J*=4.4 Hz, 1H), 6.31 (d, *J*=8.0 Hz, 1H), 6.11 (s, 1H), 5.72-5.67 (m, 1H), 5.62-5.58 (s, 2H), 5.52 (s, 1H), 5.49 (s, 1H), 4.35-4.25 (m, 4H), 3.94-3.91 (m, 1H), 3.70 (s, 2H), 3.34 (m, 1H), 2.56-2.31 (m, 2H), 2.22-1.29 (m, 10H), 0.89 (m, 3H). MS m/z (ESI): 665.22 [M+H]⁺

### Synthetic Example 35

### S-(Cyanomethyl)

### (2S,6aS,6bR,7S,8aS,8bS,11aR,12aS,12bS)-2,6b-difluoro-7-hydroxy-6a,8a-dimethyl-4-oxo-10-(4-( ((2-oxotetrahydrofuran-3-yl)thio)methyl)phenyl)-1,2,4,6a,6b,7,8,8a,11a,12,12a,12b-dodecahydro-8bH-naphtho[2',1':4,5]indeno[1,2-d][1,3]dioxole-8b-carbothioate (35)

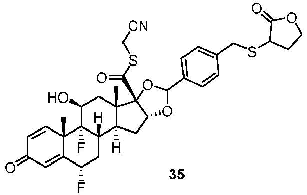

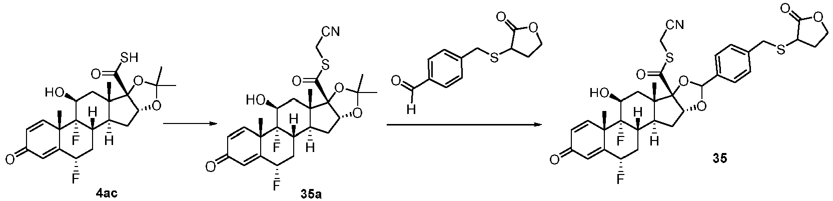

### Step 1

Compound **4ac** (2 g, 4.4 mmol), sodium sulfite (0.84 g, 6.8 mmol), and 10 mL of N,N-dimethylacetamide were added to a reaction flask. 2-Iodoacetonitrile (1.1 g, 6.8 mmol) was slowly added, and the mixture was allowed to react at room temperature. TLC monitoring was performed until no compound **4ac** was detected. After completion of the reaction, the reaction solution was poured into water and stirred for 30 minutes. The mixture was filtered under reduced pressure. The filter cake was dried. The crude product was separated and purified by silica gel column chromatography (dichloromethane/acetone = 4/1) and concentrated under reduced pressure to obtain compound **35a** (0.9 g, yield: 42%).

### Step 2

Compound **35a** (0.5 g, 1 mmol), 4-(((2-oxotetrahydrofuran-3-yl)thio)methyl)benzaldehyde (0.29 g, 1.22 mmol), and 30 mL of acetonitrile were added to a reaction flask. Perchloric acid (0.26 mL, 3 mmol) was slowly added dropwise, and the mixture was allowed to react at room temperature. TLC monitoring was performed until no compound **35a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **35** (0.38 g, yield: 56%). ¹H NMR (400 MHz, d₆-DMSO): δ 7.44-7.37 (m, 4H), 7.27 (d, *J* =4.4 Hz, 1H), 6.30 (d, *J* =8.0 Hz, 1H), 6.10 (s, 1H), 5.60-5.58 (m, 1H), 5.52 (s, 1H), 5.50 (s, 1H), 4.37-4.25 (m, 4H), 3.99 (s, 2H), 3.91 (m, 1H), 3.72 (s, 2H), 3.36 (m, 1H), 2.55-2.31 (m, 2H), 2.11-1.24 (m, 10H), 0.87 (m, 3H). MS m/z (ESI): 672.28 [M+H]⁺

### Synthetic Example 36

### Fluoromethyl

### (2S,6aS,6bR,7S,8aS,8bS,11aR,12aS,12bS)-2,6b-difluoro-7-hydroxy-6a,8a-dimethyl-4-oxo-10-(4-( ((2-oxotetrahydrofuran-3-yl)thio)methyl)phenyl)-1,2,4,6a,6b,7,8,8a,11a,12,12a,12b-dodecahydro-8bH-naphtho[2',1':4,5]indeno[1,2-d][1,3]dioxole-8b-carboxylate (36)

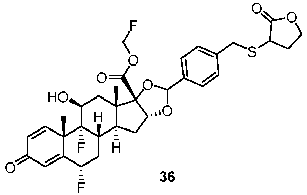

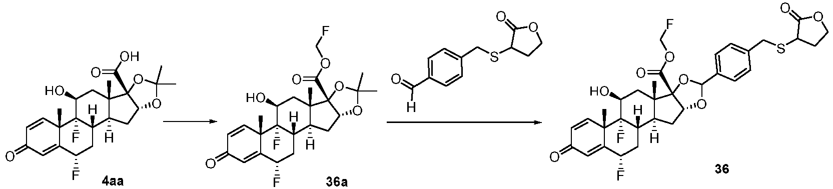

### Step 1

Compound **4aa** (1.9 g, 4.4 mmol), sodium sulfite (0.84 g, 6.8 mmol), and 10 mL of N,N-dimethylacetamide were added to a reaction flask. Fluoroiodomethane (1.1 g, 6.8 mmol) was slowly added, and the mixture was allowed to react at room temperature. TLC monitoring was performed until no compound **4aa** was detected. After completion of the reaction, the reaction solution was poured into water and stirred for 30 minutes. The mixture was filtered under reduced pressure. The filter cake was dried. The crude product was separated and purified by silica gel column chromatography (dichloromethane/acetone = 4/1) and concentrated under reduced pressure to obtain compound **36a** (0.9 g, yield: 45%).

### Step 2

Compound **36a** (0.5 g, 1 mmol), 4-(((2-oxotetrahydrofuran-3-yl)thio)methyl)benzaldehyde (0.3 g, 1.28 mmol), and 30 mL of acetonitrile were added to a reaction flask. Perchloric acid (0.27 mL, 3.2 mmol) was slowly added dropwise, and the mixture was allowed to react at room temperature. TLC monitoring was performed until no compound **36a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **36** (0.38 g, yield: 55%). ¹H NMR (400 MHz, d₆-DMSO): δ 7.45-7.37 (m, 4H), 7.26 (d, *J* =4.4 Hz, 1H), 6.88 (s, 2H), 6.32 (d, *J* =8.0 Hz, 1H), 6.13 (s, 1H), 5.65-5.63 (m, 1H), 5.50 (s, 1H), 5.46 (s, 1H), 4.37-4.27 (m, 3H), 3.91 (m, 1H), 3.70 (s, 2H), 3.35 (m, 2H), 2.57-2.35 (m, 2H), 2.03-1.18 (m, 10H), 0.85 (m, 3H). MS m/z (ESI): 649.31 [M+H]⁺

### Synthetic Example 37

### Cyanomethyl

### (2S,6aS,6bR,7S,8aS,8bS,11aR,12aS,12bS)-2,6b-difluoro-7-hydroxy-6a,8a-dimethyl-4-oxo-10-(4-( ((2-oxotetrahydrofuran-3-yl)thio)methyl)phenyl)-1,2,4,6a,6b,7,8,8a,11a,12,12a,12b-dodecahydro-8bH-naphtho[2',1':4,5]indeno[1,2-d][1,3]dioxole-8b-carboxylate (37)

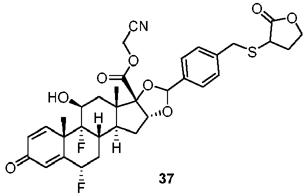

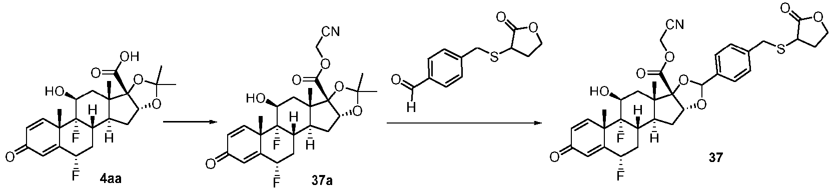

### Step 1

Compound **4aa** (1.9 g, 4.4 mmol), sodium sulfite (0.84 g, 6.8 mmol), and 10 mL of N,N-dimethylacetamide were added to a reaction flask. 2-Iodoacetonitrile (1.1 g, 6.8 mmol) was slowly added, and the mixture was allowed to react at room temperature. TLC monitoring was performed until no compound **4aa** was detected. After completion of the reaction, the reaction solution was poured into water and stirred for 30 minutes. The mixture was filtered under reduced pressure. The filter cake was dried. The crude product was separated and purified by silica gel column chromatography (dichloromethane/acetone = 4/1) and concentrated under reduced pressure to obtain compound **37a** (0.8 g, yield: 38%).

### Step 2

Compound **37a** (0.5 g, 1 mmol), 4-(((2-oxotetrahydrofuran-3-yl)thio)methyl)benzaldehyde (0.3 g, 1.26 mmol), and 30 mL of acetonitrile were added to a reaction flask. Perchloric acid (0.27 mL, 3.14 mmol) was slowly added dropwise, and the mixture was allowed to react at room temperature. TLC monitoring was performed until no compound **37a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 37 (0.42 g, yield: 61%). ¹H NMR (400 MHz, d₆-DMSO): δ 7.45-7.38 (m, 4H), 7.27 (d, *J* =4.4 Hz, 1H), 6.35 (d, *J* =8.0 Hz, 1H), 6.15 (s, 1H), 5.63-5.60 (m, 1H), 5.55 (s, 1H), 5.53 (s, 1H), 5.20 (s, 2H), 4.73 (m, 1H), 4.35-4.25 (m, 3H), 3.91 (m, 1H), 3.70 (s, 2H), 3.37 (m, 1H), 2.57-2.33 (m, 2H), 2.04-1.11 (m, 10H), 0.88 (m, 3H). MS m/z (ESI): 656.32 [M+H]⁺

### Synthetic Example 38

### (6aS,6bR,7S,8aS,8bS,11aR,12aS,12bS)-6b-chloro-8b-(2-chloroacetyl)-7-hydroxy-6a,8a-dimethyl -10-(4-(((2-oxotetrahydrofuran-3-yl)thio)methyl)phenyl)-1,2,6a,6b,7,8,8a,8b,11a,12,12a,12b-dode cahydro-4H-naphtho[2',1':4,5]indeno[1,2-d][1,3]dioxol-4-one (38)

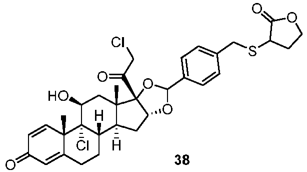

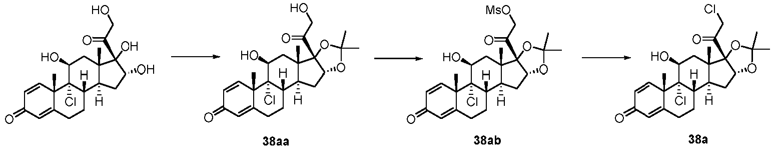

### Step 1

(9α,11β,16α)-9-Chloro-11,16,17,21-tetrahydroxy-pregna-1,4-diene-3,20-dione (5 g, 0.012 mol) and 50 mL of acetone were added to a reaction flask. Perchloric acid (5 mL, 0.06 mol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water was added, and the concentration was continued until no solvent was distilled off. The concentrate was stirred for 30 minutes. The mixture was filtered under reduced pressure. The filter cake was dried to obtain compound **38aa** (3.8 g, yield: 71%).

### Step 2

Compound **38aa** (3.5 g, 7.76 mmol) and 30 mL of pyridine were added to a reaction flask and cooled to 0 °C. Methanesulfonyl chloride (1.2 mL, 15.5 mmol) was slowly added dropwise, and the mixture was allowed to react at room temperature. TLC monitoring was performed until no compound **38aa** was detected. After completion of the reaction, the reaction solution was poured into 1 N dilute hydrochloric acid and stirred for 30 minutes. The mixture was filtered under reduced pressure. The filter cake was washed with water until neutral and dried to obtain compound **38ab** (2.7 g, yield: 65%).

### Step 3

Compound **38ab** (2.5 g, 4.73 mmol), lithium chloride (0.4 g, 9.46 mmol), and 25 mL of *N,N*-dimethylformamide were added to a reaction flask and allowed to react at room temperature. TLC monitoring was performed until no compound **38ab** was detected. After completion of the reaction, the reaction solution was poured into water and stirred for 30 minutes. The mixture was filtered under reduced pressure. The filter cake was dried. The crude product was separated and purified by silica gel column chromatography (dichloromethane/acetone = 4/1) to obtain compound **38a** (0.93 g, yield: 42%). MS m/z (ESI): 469.21 [M+H]⁺

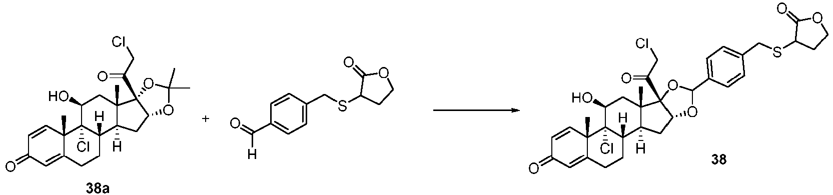

Compound **38a** (0.5 g, 1.16 mmol), 4-(((2-oxotetrahydrofuran-3-yl)thio)methyl)benzaldehyde (0.33 g, 1.4 mmol), and 30 mL of acetonitrile were added to a reaction flask. Perchloric acid (0.3 mL, 3.5 mmol) was slowly added dropwise, and the mixture was allowed to react at room temperature. TLC monitoring was performed until no compound **38a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **38** (0.49 g, yield: 65%). ¹H NMR (400 MHz, d₆-DMSO): δ 7.45 (m, 2H), 7.36-7.32 (m, 2H), 7.26 (m, 1H), 6.24-6.22 (m, 1H), 6.00 (s, 1H), 5.60-5.58 (m, 1H), 5.52 (s, 1H), 4.54 (s, 2H), 4.35-4.25 (m,3H), 3.75-3.70 (m, 3H), 3.40-3.34 (m, 1H), 2.55-2.38 (m, 4H), 2.01-1.96 (m, 2H), 1.85-1.82 (m, 2H), 1.79-1.58 (m, 7H), 0.85 (s, 3H). MS m/z (ESI): 647.22 [M+H]⁺

### Synthetic Example 39

### (6aS,6bR,7S,8aS,8bS,11aR,12aS,12bS)-6b-Chloro-8b-(2-chloroacetyl)-7-hydroxy-6a,8a-dimethyl -10-(4-(((5-oxotetrahydrofuran-3-yl)thio)methyl)phenyl)-l,2,6a,6b,7,8,8a,8b,11a,12,12a,12b-dode cahydro-4H-naphtho[2',1':4,5]indeno[1,2-d][1,3]dioxol-4-one (39)

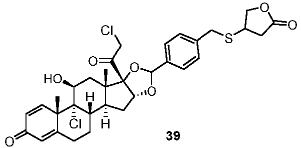

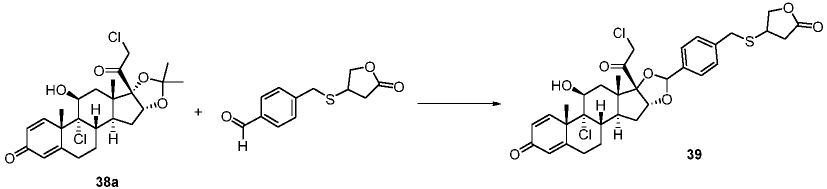

Compound **38a** (0.5 g, 1.16 mmol), 4-(((5-oxotetrahydrofuran-3-yl)thio)methyl)benzaldehyde (0.33 g, 1.4 mmol), and 30 mL of acetonitrile were added to a reaction flask. Perchloric acid (0.3 mL, 3.5 mmol) was slowly added dropwise, and the mixture was allowed to react at room temperature. TLC monitoring was performed until no compound **38a** was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **39** (0.44 g, yield: 58%). ¹H NMR (400 MHz, d₆-DMSO): δ 7.47 (m, 2H), 7.38-7.34 (m, 2H), 7.26 (m, 1H), 6.25-6.22 (m, 1H), 6.01 (s, 1H), 5.61-5.59 (m, 1H), 5.51 (s, 1H), 4.75-4.55 (m, 2H), 4.51 (s, 2H), 4.32 (m,1H), 3.75-3.70 (m, 3H), 3.14 (m, 1H), 2.75-2.41 (m, 4H), 2.00-1.96 (m, 2H), 1.72-1.45 (m, 5H), 1.44-1.42 (m, 3H), 1.06-1.04 (m, 1H), 0.86 (s, 3H). MS m/z (ESI): 647.24 [M+H]⁺

### Synthetic Example 40

### (6aS,6bR,7S,8aS,8bS,11aR,12aS,12bS)-6b-Fluoro-7-hydroxy-8b-(2-hydroxyacetyl)-6a,8a-dimeth yl-10-(4-((2-oxotetrahydrofuran-3-yl)oxy)phenyl)-1,2,6a,6b,7,8,8a,8b,11a,12,12a,12b-dodecahydr o-4H-naphtho[2',1':4,5]indeno[1,2-d][1,3]dioxol-4-one (40)

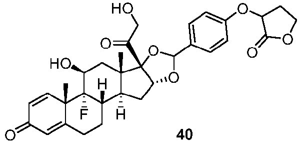

### 4-((2-Oxotetrahydrofuran-3-yl)oxy)benzaldehyde

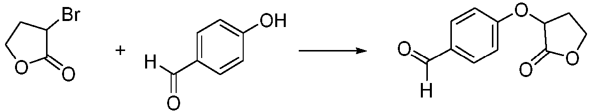

3-Bromodihydrofuran-2(3H)-one (7.26 g, 0.044 mol), 4-hydroxybenzaldehyde (5 g, 0.04 mol), potassium carbonate (6.9 g, 0.05 mol), and 150 mL of acetonitrile amine were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 4-((2-oxotetrahydrofuran-3-yl)oxy)benzaldehyde (3.5 g, yield: 43%). MS m/z (ESI): 207.12 [M+H]⁺

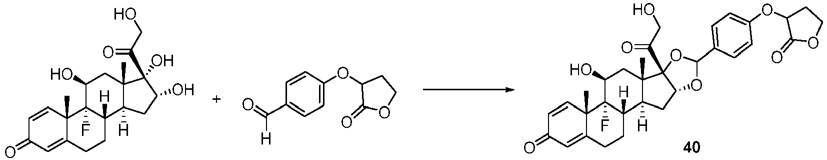

(9α,11β,16α)-9-Fluoro-11,16,17,21-tetrahydroxy-pregna-1,4-diene-3,20-dione (0.5 g, 1.27 mmol), 4-((2-oxotetrahydrofuran-3-yl)oxy)benzaldehyde (0.31 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **40** (0.32 g, yield: 43%). ¹H NMR (400 MHz, d₆-DMSO): δ 7.42-7.37 (m, 4H), 7.28 (m, 1H), 6.25-6.21 (m, 1H), 6.00 (s, 1H), 5.50-5.47 (m, 1H), 5.10 (m, 1H), 4.94 (m, 1H), 4.58-4.49 (m, 2H), 4.45 (m, 1H), 4.37-4.16 (m, 4H), 2.65-2.39 (m, 2H), 2.10-1.83 (m, 2H), 1.80-1.46 (m, 3H), 1.44-1.02 (m, 4H), 0.98 (m, 1H), 0.93 (m, 3H), 0.86 (m, 3H). MS m/z (ESI): 583.31 [M+H]⁺

### Synthetic Example 41

### (6aS,6bR,7S,8aS,8bS,11aR,12aS,12bS)-6b-Fluoro-7-hydroxy-8b-(2-hydroxyacetyl)-6a,8a-dimeth yl-10-(4-((2-oxotetrahydrofuran-3-yl)thio)phenyl)-1,2,6a,6b,7,8,8a,8b,11a,12,12a,12b-dodecahydr o-4H-naphtho[2',1':4,5]indeno[1,2-d][1,3]dioxol-4-one (41)

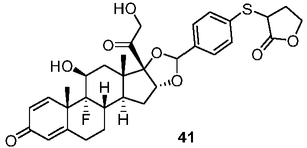

### 4-((2-Oxotetrahydrofuran-3-yl)thio)benzaldehyde

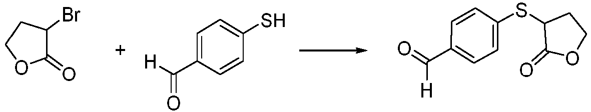

3-Bromodihydrofuran-2(3H)-one (7.26 g, 0.044 mol), 4-mercaptobenzaldehyde (5.5 g, 0.04 mol), potassium carbonate (6.9 g, 0.05 mol), and 150 mL of acetonitrile amine were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 4-((2-oxotetrahydrofuran-3-yl)thio)benzaldehyde (3.6 g, yield: 40%). MS m/z (ESI): 223.15 [M+H]⁺

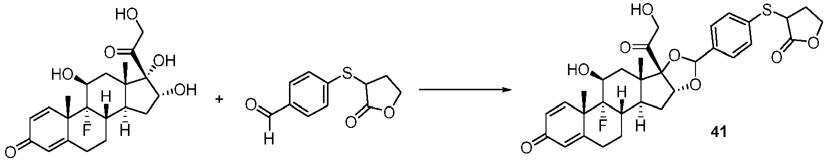

(9α,11β,16α)-9-Fluoro-11,16,17,21-tetrahydroxy-pregna-1,4-diene-3,20-dione (0.5 g, 1.27 mmol), 4-((2-oxotetrahydrofuran-3-yl)thio)benzaldehyde (0.34 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected.

After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **41** (0.34 g, yield: 45%). ¹H NMR (400 MHz, d₆-DMSO): δ 7.45-7.37 (m, 4H), 7.26 (m, 1H), 6.27-6.23 (m, 1H), 6.02 (s, 1H), 5.52-5.49 (m, 1H), 5.07 (m, 1H), 4.93 (m, 1H), 4.69-4.65 (m, 2H), 4.35-4.15 (m, 4H), 3.69 (m, 1H), 2.59-2.32 (m, 4H), 2.07-1.85 (m, 3H), 1.80-1.46 (m, 4H), 1.42-1.02 (m, 3H), 0.98 (m, 1H), 0.87 (m, 3H). MS m/z (ESI): 599.30 [M+H]⁺

### Synthetic Example 42

### (6aS,6bR,7S,8aS,8bS,11aR,12aS,12bS)-6b-Fluoro-7-hydroxy-8b-(2-hydroxyacetyl)-6a,8a-dimeth yl-10-(4-((2-oxotetrahydrofuran-3-yl)amino)phenyl)-1,2,6a,6b,7,8,8a,8b,11a,12,12a,12b-dodecahy dro-4H-naphtho[2',1':4,5]indeno[1,2-d][1,3]dioxol-4-one (42)

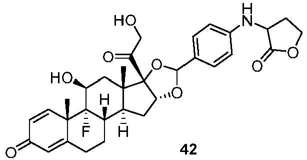

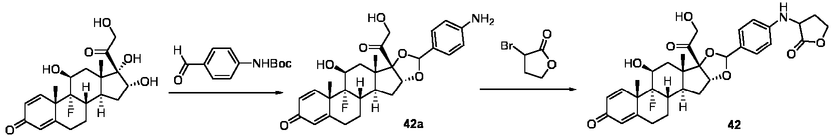

### Step 1

(9α,11β,16α)-9-Fluoro-11,16,17,21-tetrahydroxy-pregna-1,4-diene-3,20-dione (0.8 g, 2.03 mmol), tert-butyl (4-formylphenyl)carbamate (0.67 g, 3.04 mmol), and 30 mL of acetonitrile were added to a reaction flask. Perchloric acid (0.5 mL, 6.08 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **42a** (0.7 g, yield: 68%).

### Step 2

Compound **42a** (0.5 g, 1 mmol), 3-bromodihydrofuran-2(3H)-one (0.18 g, 1.1 mmol), potassium carbonate (0.17 g, 1.2 mmol), and 10 mL of *N,N*-dimethylformamide were added to a reaction flask and heated to 80 °C. TLC monitoring was performed until no compound **42a** was detected. After completion of the reaction, the reaction solution was poured into water and stirred for 30 minutes. The mixture was filtered under reduced pressure. The filter cake was washed with water and dried. The crude product was separated and purified by silica gel column chromatography (dichloromethane/acetone = 4/1) and concentrated under reduced pressure to obtain compound **42** (0.15 g, yield: 25%). ¹H NMR (400 MHz, d₆-DMSO): δ 10.01 (m, 1H), 7.46-7.37 (m, 4H), 7.27 (m, 1H), 6.27-6.23 (m, 1H), 6.00 (s, 1H), 5.50-5.48 (m, 1H), 5.05 (m, 1H), 4.95 (m, 1H), 4.69-4.64 (m, 2H), 4.37-4.12 (m, 4H), 3.52 (m, 1H), 2.61-2.35 (m, 4H), 2.09-1.66 (m, 5H), 1.58-1.22 (m, 5H), 1.03 (m, 1H), 0.86 (m, 3H). MS m/z (ESI): 582.31 [M+H]⁺

### Synthetic Example 43

### (6aS,6bR,7S,8aS,8bS,11aR,12aS,12bS)-6b-Fluoro-7-hydroxy-8b-(2-hydroxyacetyl)-6a,8a-dimeth yl-10-(4-((2-oxotetrahydrofuran-3-yl)methyl)phenyl)-1,2,6a,6b,7,8,8a,8b,11a,12,12a,12b-dodecah ydro-4H-naphtho[2',1':4,5]indeno[1,2-d][1,3]dioxol-4-one (43)

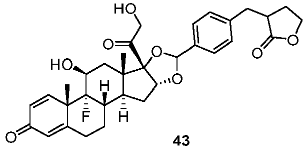

### 4-((2-Oxotetrahydrofuran-3-yl)methyl)benzaldehyde

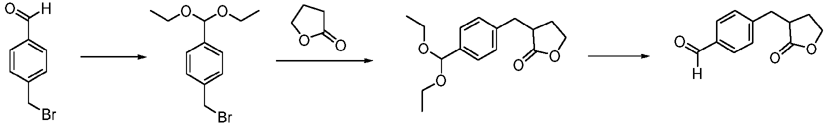

### Step 1

4-Bromomethylbenzaldehyde (5 g, 0.025 mol), triethyl orthoformate (4.6 mL, 0.028 mol), p-toluenesulfonic acid monohydrate (0.5 g, 2.5 mmol), and 20 mL of ethanol were added to a reaction flask and allowed to react at room temperature overnight. After completion of the reaction, the mixture was concentrated under reduced pressure. The crude product was separated and purified by silica gel column chromatography (petroleum ether/ethyl acetate = 10/1) and concentrated under reduced pressure to obtain 1-bromomethyl-4-(diethoxymethyl)benzene (2.87 g, yield: 42%).

### Step 2

Diisopropylamine (1.3 mL, 9 nnol) and 20 mL of dry tetrahydrofuran were added to a reaction flask and cooled to -78°C. A 2.5 M solution of n-butyllithium in n-hexane (3.6 mL, 9 mmol) was slowly added dropwise. Dihydrofuran-2(3*H*)-one (0.77 g, 9 mmol) was added and stirred for 30 minutes. 1-Bromomethyl-4-(diethoxymethyl)benzene (2.7 g, 10 mmol) was added. The mixture was slowly warmed to room temperature and allowed to react for 20 hours. After completion of the reaction, saturated ammonium chloride solution was added to quench the reaction. Dichloromethane was added for extraction and liquid-liquid separation. The organic phases were combined, washed with saturated brine, and concentrated under reduced pressure. The crude product was separated and purified by silica gel column chromatography (petroleum ether/ethyl acetate=5/1) and concentrated under reduced pressure to obtain 3-(4-(diethoxymethyl)benzyl)dihydrofuran-2(3H)-one(1.4 g, yield: 55%).

### Step 3

3-(4-(Diethoxymethyl)benzyl)dihydrofuran-2(3H)-one(1 g, 3.6 mmol), 5 mL of tetrahydrofuran, and 10 mL of 2 M HCl solution were added to a reaction flask and allowed to react at room temperature for 2 hours. After completion of the reaction, water and ethyl acetate were added for extraction and liquid-liquid separation. The organic phases were combined, washed with saturated brine, and concentrated under reduced pressure. The crude product was separated and purified by silica gel column chromatography (petroleum ether/ethyl acetate = 4/1) and concentrated under reduced pressure to obtain 4-((2-oxotetrahydrofuran-3-yl)methyl)benzaldehyde (0.55 g, yield: 75%). MS m/z (ESI): 205.15 [M+H]⁺

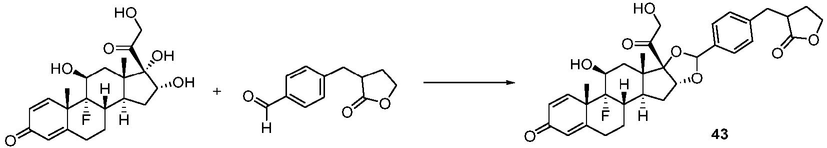

(9α,11β,16α)-9-Fluoro-11,16,17,21-tetrahydroxy-pregna-1,4-diene-3,20-dione (0.5 g, 1.27 mmol), 4-((2-oxotetrahydrofuran-3-yl)methyl)benzaldehyde (0.31 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **43** (0.47 g, yield: 64%). ¹H NMR (400 MHz, d₆-DMSO): δ 7.46-7.38 (m, 4H), 7.27 (m, 1H), 6.25-6.23 (m, 1H), 6.00 (s, 1H), 5.55-5.53 (m, 1H), 5.10 (m, 1H), 4.98 (m, 1H), 4.75-4.72 (m, 2H), 4.44-4.21 (m, 4H), 3.23-3.02 (m, 2H), 2.70 (m, 1H), 2.41-2.32 (m, 2H), 2.17-1.85 (m, 6H), 1.80-1.45 (m, 3H), 1.22-1.03 (m, 3H), 0.99 (m, 1H), 0.85 (m, 3H). MS m/z (ESI): 581.37 [M+H]⁺

### Synthetic Example 44

### (6aS,6bR,7S,8aS,8bS,11aR,12aS,12bS)-6b-Fluoro-7-hydroxy-8b-(2-hydroxyacetyl)-6a,8a-dimeth yl-10-(1-((2-oxotetrahydrofuran-3-yl)methyl)piperidin-4-yl)-1,2,6a,6b,7,8,8a,8b,11a,12,12a,12b-d odecahydro-4H-naphtho[2',1':4,5]indeno[1,2-d][1,3]dioxol-4-one (44)

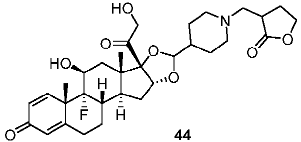

### 1-((2-Oxotetrahydrofuran-3-yl)methyl)piperidine-4-carbaldehyde

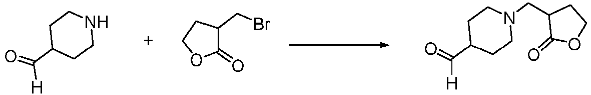

Piperidine-4-carbaldehyde (5 g, 0.04 mol), 3-bromomethyl dihydrofuran-2(3*H*)-one (9 g, 0.05 mol), and 60 mL of dichloromethane were added to a reaction flask and allowed to react at room temperature for 12 hours. After completion of the reaction, the mixture was concentrated under reduced pressure. The crude product was separated and purified by silica gel column chromatography (petroleum ether/ethyl acetate = 4/1) and concentrated under reduced pressure to obtain 1-((2-oxotetrahydrofuran-3-yl)methyl)piperidine-4-carbaldehyde (3 g, yield: 35%). MS m/z (ESI): 212.23 [M+H]⁺

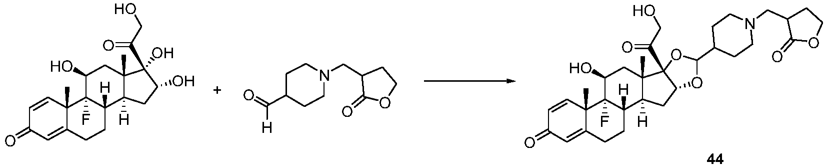

(9α,11β,16α)-9-Fluoro-11,16,17,21-tetrahydroxy-pregna-1,4-diene-3,20-dione (0.5 g, 1.27 mmol), 1-((2-oxotetrahydrofuran-3-yl)methyl)piperidine-4-carbaldehyde (0.32 g, 1.52 mmol), and 20 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **44** (0.31 g, yield: 43%). ¹H NMR (400 MHz, d₆-DMSO): δ 7.27 (m, 1H), 6.23-6.20 (m, 1H), 6.02 (s, 1H), 5.39-5.36 (m, 1H), 5.12 (m, 1H), 4.97 (m, 1H), 4.69-4.67 (m, 2H), 4.35-4.14 (m, 3H), 3.94 (m, 1H), 2.81-2.78 (m, 2H), 2.51-1.98 (m, 9H), 2.17-1.85 (m, 6H), 1.80-1.45 (m, 4H), 1.25-1.05 (m, 5H), 0.99 (m, 1H), 0.86 (m, 3H). MS m/z (ESI): 588.32 [M+H]⁺

### Synthetic Example 45

### (6aS,6bR,7S,8aS,8bS,11aR,12aS,12bS)-6b-Fluoro-7-hydroxy-8b-(2-hydroxyacetyl)-6a,8a-dimeth yl-10-(4-((2-oxotetrahydrofuran-3-yl)thio)cyclohexyl)-1,2,6a,6b,7,8,8a,8b,11a,12,12a,12b-dodeca hydro-4H-naphtho[2',1':4,5]indeno[1,2-d][1,3]dioxol-4-one (45)

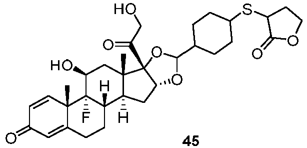

### 4-((2-Oxotetrahydrofuran-3-yl)thio)cyclohexane-1-carbaldehyde

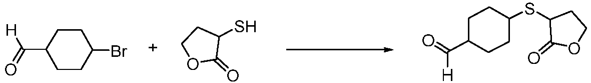

3-Mercaptotetrahydrofuran-2(3H)-one (0.68 g, 5.75 mmol), 4-bromo-1-carbaldehyde (1 g, 5.23 mmol), potassium carbonate (0.87 g, 6.28 mmol), and 10 mL of N,N-dimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 4/1) and concentrated under reduced pressure to obtain 4-((2-oxotetrahydrofuran-3-yl)thio)cyclohexane-1-carbaldehyde (0.72 g, yield: 60%). MS m/z (ESI): 229.11 [M+H]⁺

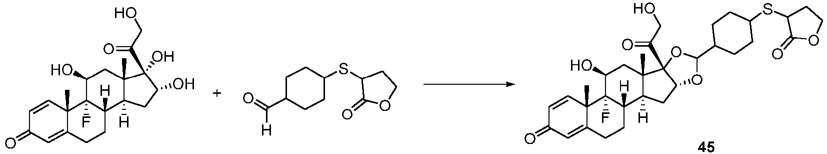

(9α,11β,16α)-9-Fluoro-11,16,17,21-tetrahydroxy-pregna-1,4-diene-3,20-dione (0.5 g, 1.27 mmol), 4-((2-oxotetrahydrofuran-3-yl)thio)cyclohexane-1-carbaldehyde (0.34 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **45** (0.42 g, yield: 55%). ¹H NMR (400 MHz, d₆-DMSO): δ 7.27 (m, 1H), 6.22-6.20 (m, 1H), 6.00 (s, 1H), 5.55-5.52 (m, 1H), 5.10 (m, 1H), 4.97 (m, 1H), 4.72-4.69 (m, 2H), 4.37-4.15 (m, 3H), 3.97 (m, 1H), 3.34 (m, 1H), 2.56-2.31 (m, 5H), 1.93-1.35 (m, 15H), 1.22-1.05 (m, 4H), 1.02 (m, 1H), 0.85 (m, 3H). MS m/z (ESI): 605.37 [M+H]⁺

### Synthetic Example 46

### (25,6aS,6bR,78,8a8,8bS,11aR,12a8,12b8S)-2,6b-Difluoro-7-hydroxy-8b-(2-hydroxyacetyl)-6a,8a-dimethyl-10-(6-(((2-oxotetrahydrofuran-3-yl)thio)methyl)pyridin-3-yl)-1,2,6a,6b,7,8,8a,8b,11a,12, 12a,12b-dodecahydro-4H-naphtho[2',1':4,5]indeno[1,2-d][1,3]dioxol-4-one (46)

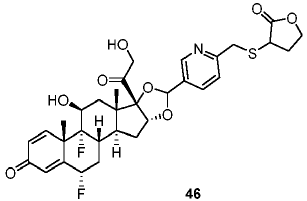

### 6-(((2-Oxotetrahydrofuran-3-yl)thio)methyl)nicotinaldehyde

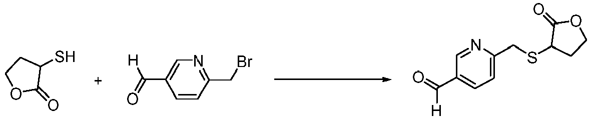

3-Mercaptodihydrofuran-2(3*H*)-one (1 g, 8.46 mmol), 6-(bromomethyl)nicotinaldehyde (1.86 g, 9.31 mmol), potassium carbonate (1.4 g, 10.1 mmol), and 20 mL of *N,N-*dimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 2/1) and concentrated under reduced pressure to obtain 6-(((2-oxotetrahydrofuran-3-yl)thio)methyl)nicotinaldehyde (0.7 g, yield: 35%). MS m/z (ESI): 238.15 [M+H]⁺

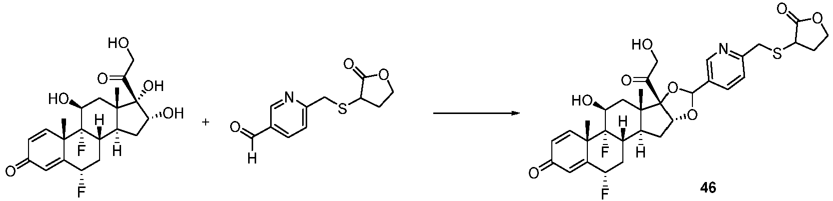

(6α,9α,11β,16α)-6,9-Difluoro-11,16,17,21-tetrahydroxy-pregna-1,4-diene-3,20-dione (0.5 g, 1.21 mmol), 6-(((2-oxotetrahydrofuran-3-yl)thio)methyl)nicotinaldehyde (0.35 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **46** (0.48 g, yield: 62%). ¹H NMR (400 MHz, d₆-DMSO): δ 8.40 (m, 1H), 7.70 (m, 1H), 7.34 (m, 1H), 7.15 (m, 1H), 6.45 (m, 1H), 6.33 (s, 1H), 6.07 (m, 1H), 5.10 (s, 1H), 4.95 (s, 1H), 4.85 (m, 2H), 4.44-4.32 (m, 3H), 4.28-4.20 (m, 2H), 4.02-3.94 (m, 2H), 3.34 (m, 1H), 2.62-2.48 (m, 2H), 2.32-1.96 (m, 4H), 1.75-1.65 (m, 3H), 1.55-1.42 (m, 4H), 0.86 (m, 3H). MS m/z (ESI): 632.32 [M+H]⁺

### Synthetic Example 47

### (6aS,6bR,7S,8aS,8bS,11aR,12aS,12bS)-6b-Fluoro-7-hydroxy-8b-(2-hydroxyacetyl)-6a,8a-dimeth yl-10-(6-(((2-oxotetrahydrofuran-3-yl)thio)methyl)pyridin-3-yl)-1,2,6a,6b,7,8,8a,8b,11a,12,12a,12 b-dodecahydro-4H-naphtho[2',1':4,5]indeno[1,2-d][1,3]dioxol-4-one (47)

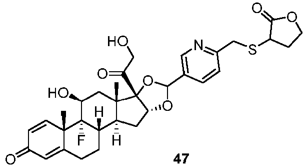

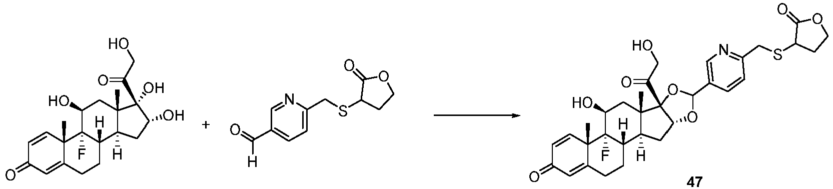

(9α,11β,16α)-9-Fluoro-11,16,17,21-tetrahydroxy-pregna-1,4-diene-3,20-dione (0.5 g, 1.27 mmol), 6-(((2-oxotetrahydrofuran-3-yl)thio)methyl)nicotinaldehyde (0.36 g, 1.52 mmol), and 30 mL of acetonitrile were added to a reaction flask. Perchloric acid (0.3 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **47** (0.53 g, yield: 68%). ¹H NMR (400 MHz, d₆-DMSO): δ 8.42 (m, 1H), 7.75 (m, 1H), 7.36 (m, 1H), 7.10 (m, 1H), 6.47 (m, 1H), 6.30 (s, 1H), 6.05 (m, 1H), 5.13 (s, 1H), 4.96 (s, 1H), 4.90 (m, 2H), 4.45-4.32 (m, 3H), 4.30-4.22 (m, 2H), 4.00-3.95 (m, 2H), 3.35 (m, 1H), 2.62-2.48 (m, 2H), 2.32-1.95 (m, 5H), 1.77-1.68 (m, 3H), 1.58-1.42 (m, 4H), 0.89 (m, 3H). MS m/z (ESI): 614.32 [M+H]⁺

### Synthetic Example 48

### (6aS,6bR,7S,8aS,8bS,11aR,12aS,12bS)-6b-Chloro-7-hydroxy-8b-(2-hydroxyacetyl)-6a,8a-dimeth yl-10-(6-(((2-oxotetrahydrofuran-3-yl)thio)methyl)pyridin-3-yl)-1,2,6a,6b,7,8,8a,8b,11a,12,12a,12 b-dodecahydro-4H-naphtho[2',1':4,5]indeno[1,2-d][1,3]dioxol-4-one (48)

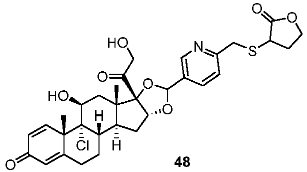

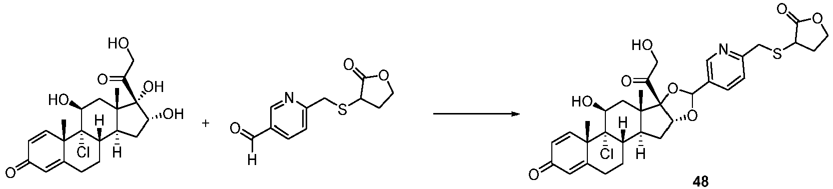

(9α,11β,16α)-9-Chloro-11,16,17,21-tetrahydroxy-pregna-1,4-diene-3,20-dione (0.5 g, 1.22 mmol), 6-(((2-oxotetrahydrofuran-3-yl)thio)methyl)nicotinaldehyde (0.35 g, 1.46 mmol), and 30 mL of acetonitrile were added to a reaction flask. Perchloric acid (0.3 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **48** (0.50 g, yield: 65%). ¹H NMR (400 MHz, d₆-DMSO): δ 8.40 (m, 1H), 7.76 (m, 1H), 7.40 (m, 1H), 7.12 (m, 1H), 6.45 (m, 1H), 6.32 (s, 1H), 6.10 (m, 1H), 5.12 (s, 1H), 4.96 (s, 1H), 4.88 (m, 2H), 4.35-4.25 (m, 2H), 4.30 (s, 2H), 3.96-3.90 (m, 2H), 3.34 (m, 1H), 2.58-2.31 (m, 4H), 2.32-2.02 (m, 3H), 1.78-1.68 (m, 4H), 1.48-1.35 (m, 3H), 1.10 (m, 1H), 0.87 (m, 3H). MS m/z (ESI): 630.25 [M+H]⁺

### Synthetic Example 49

### (2S,6aS,6bR,7S,8aS,8bS,11aR,12aS,12bS)-2,6b-Difluoro-7-hydroxy-8b-(2-hydroxyacetyl)-6a,8a-dimethyl-10-(5-(((2-oxotetrahydrofuran-3-yl)thio)methyl)pyridin-2-yl)-1,2,6a,6b,7,8,8a,8b,11a,12, 12a,12b-dodecahydro-4H-naphtho[2',1':4,5]indeno[1,2-d][1,3]dioxol-4-one (49)

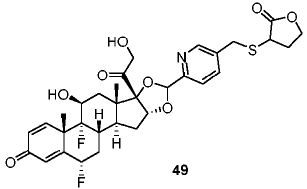

### 5-(((2-Oxotetrahydrofuran-3-yl)thio)methyl)picolinaldehyde

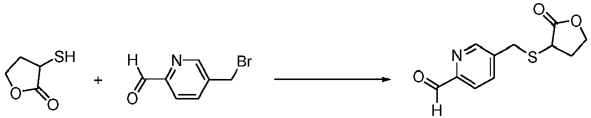

3-Mercaptodihydrofuran-2(3H)-one (1 g, 8.46 mmol), 5-(bromomethyl)picolinaldehyde (1.86 g, 9.31 mmol), potassium carbonate (1.4 g, 10.1 mmol), and 20 mL of N,Ndimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 2/1) and concentrated under reduced pressure to obtain 5-(((2-oxotetrahydrofuran-3-yl)thio)methyl)picolinaldehyde (0.64 g, yield: 32%). MS m/z (ESI): 238.17 [M+H]⁺

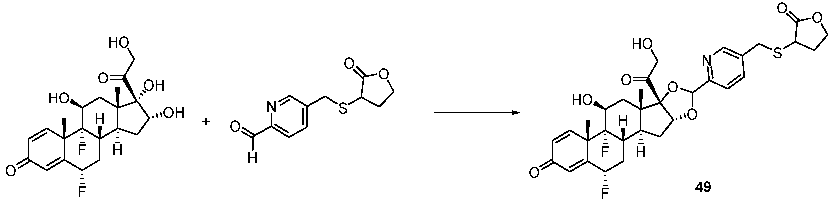

(6α,9α,11β,16α)-6,9-Difluoro-11,16,17,21-tetrahydroxy-pregna-1,4-diene-3,20-dione (0.5 g, 1.21 mmol), 5-(((2-oxotetrahydrofuran-3-yl)thio)methyl)picolinaldehyde (0.35 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **49** (0.44 g, yield: 58%). ¹H NMR (400 MHz, d₆-DMSO): δ 8.40 (m, 1H), 7.78 (m, 1H), 7.54 (m, 1H), 7.01 (m, 1H), 6.44 (m, 1H), 6.35 (s, 1H), 6.05 (m, 1H), 5.15 (s, 1H), 4.96 (s, 1H), 4.87 (m, 2H), 4.45-4.32 (m, 3H), 4.31-4.25 (m, 2H), 3.70 (m, 2H), 3.35 (m, 1H), 2.56-2.31 (m, 2H), 2.30-1.65 (m, 7H), 1.44-1.35 (m, 3H), 1.11 (m, 1H), 0.90 (m, 3H). MS m/z (ESI): 632.35 [M+H]⁺

### Synthetic Example 50

### (6aS,6bR,7S,8aS,8bS,11aR,12aS,12bS)-6b-Fluoro-7-hydroxy-8b-(2-hydroxyacetyl)-6a,8a-dimeth yl-10-(5-(((2-oxotetrahydrofuran-3-yl)thio)methyl)pyridin-2-yl)-1,2,6a,6b,7,8,8a,8b,11a,12,12a,12 b-dodecahydro-4H-naphtho[2',1':4,5]indeno[1,2-d][1,3]dioxol-4-one(50)

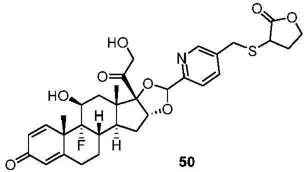

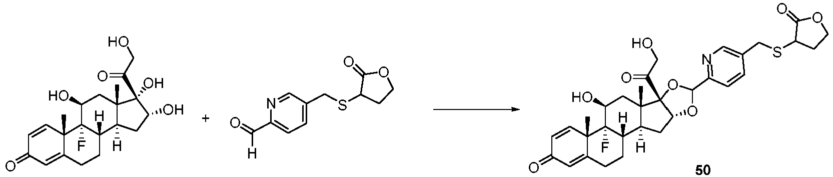

(9α,11β,16α)-9-Fluoro-11,16,17,21-tetrahydroxy-pregna-1,4-diene-3,20-dione (0.5 g, 1.27 mmol), 5-(((2-oxotetrahydrofuran-3-yl)thio)methyl)picolinaldehyde (0.36 g, 1.52 mmol), and 30 mL of acetonitrile were added to a reaction flask. Perchloric acid (0.3 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **50** (0.47 g, 61%). ¹H NMR (400 MHz, d₆-DMSO): δ 8.40 (m, 1H), 7.87 (m, 1H), 7.55 (m, 1H), 7.11 (m, 1H), 6.52 (m, 1H), 6.12 (s, 1H), 6.07 (m, 1H), 5.12 (s, 1H), 4.98 (s, 1H), 4.86 (m, 2H), 4.45-4.15 (m, 3H), 4.30-4.22 (m, 2H), 4.00 (m, 1H), 3.70 (m, 2H), 3.34 (m, 1H), 2.46-2.31 (m, 4H), 2.26-1.92 (m, 4H), 1.77-1.42 (m, 4H), 1.22 (m, 1H), 0.89 (m, 3H). MS m/z (ESI): 614.35 [M+H]⁺

### Synthetic Example 51

### (6aS,6bR,7S,8aS,8bS,11aR,12aS,12bS)-6b-Chloro-7-hydroxy-8b-(2-hydroxyacetyl)-6a,8a-dimeth yl-10-(5-(((2-oxotetrahydrofuran-3-yl)thio)methyl)pyridin-2-yl)-1,2,6a,6b,7,8,8a,8b,11a,12,12a,12 b-dodecahydro-4H-naphtho[2',1':4,5]indeno[1,2-d][1,3]dioxol-4-one (51)

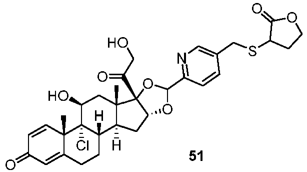

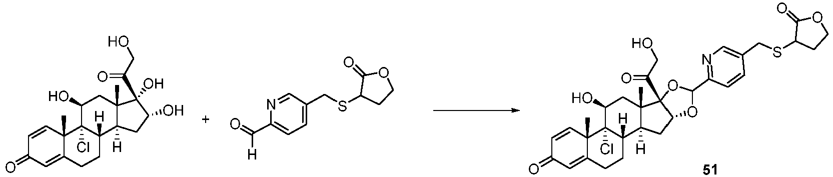

(9α,11β,16α)-9-Chloro-11,16,17,21-tetrahydroxy-pregna-1,4-diene-3,20-dione (0.5 g, 1.22 mmol), 5-(((2-oxotetrahydrofuran-3-yl)thio)methyl)picolinaldehyde (0.35 g, 1.46 mmol), and 30 mL of acetonitrile were added to a reaction flask. Perchloric acid (0.3 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **51** (0.45 g, yield: 59%). ¹H NMR (400 MHz, d₆-DMSO): δ 8.39 (m, 1H), 7.77 (m, 1H), 7.43 (m, 1H), 7.02 (m, 1H), 6.42 (m, 1H), 6.33 (s, 1H), 6.11 (m, 1H), 5.10 (s, 1H), 4.96 (s, 1H), 4.70 (m, 2H), 4.37-4.26 (m, 2H), 3.94-3.92 (m, 1H), 3.70 (s, 2H), 3.35 (m, 1H), 2.55-2.30 (m, 4H), 2.33-1.89 (m, 6H), 1.79-1.68 (m, 2H), 1.50-1.38 (m, 3H), 1.15 (m, 1H), 0.87 (m, 3H). MS m/z (ESI): 630.28 [M+H]⁺

### Synthetic Example 52

### (2S,6aS,6bR,7S,8aS,8bS,11aR,12aS,12bS)-2,6b-Difluoro-7-hydroxy-8b-(2-hydroxyacetyl)-6a,8a-dimethyl-10-(2-(((2-oxotetrahydrofuran-3-yl)thio)methyl)pyrimidin-5-yl)-1,2,6a,6b,7,8,8a,8b,11a, 12,12a,12b-dodecahydro-4H-naphtho[2',1':4,5]indeno[1,2-d][1,3]dioxol-4-one (52)

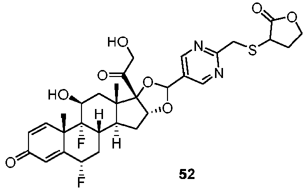

### 2-(((2-Oxotetrahydrofuran-3-yl)thio)methyl)pyrimidine-5-carbaldehyde

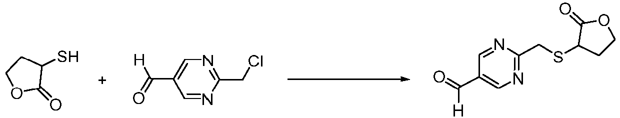

3-Mercaptodihydrofuran-2(3*H*)-one (1 g, 8.46 mmol), 2-(chloromethyl)pyrimidine-5-carbaldehyde (1.46 g, 9.31 mmol), potassium carbonate (1.4 g, 10.1 mmol), and 20 mL of *N,N-*dimethylformamide were added to a reaction flask and allowed to react at room temperature for 6 hours. After completion of the reaction, water and dichloromethane were added for extraction and liquid-liquid separation. The organic phase was washed successively with water and saturated brine and concentrated under reduced pressure. The crude product was separated and purified by silica gel column chromatography (petroleum ether/ethyl acetate = 2/1) and concentrated under reduced pressure to obtain 2-(((2-oxotetrahydrofuran-3-yl)thio)methyl) pyrimidine-5-carbaldehyde (0.83 g, yield: 41%). MS m/z (ESI): 239.15 [M+H]⁺

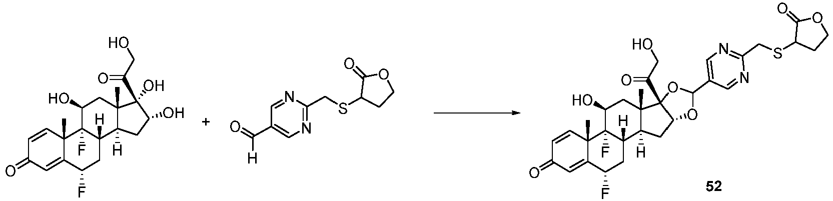

(6α,9α,11β,16α)-6,9-Difluoro-11,16,17,21-tetrahydroxy-pregna-1,4-diene-3,20-dione (0.5 g, 1.21 mmol), 2-(((2-oxotetrahydrofuran-3-yl)thio)methyl) pyrimidine-5-carbaldehyde (0.35 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **52** (0.44 g, yield: 57%). ¹H NMR (400 MHz, d₆-DMSO): δ 8.83 (m, 2H), 7.01 (m, 1H), 6.41 (m, 1H), 6.33 (s, 1H), 6.05 (m, 1H), 5.01 (s, 1H), 4.88 (s, 1H), 4.79 (m, 2H), 4.35-4.25 (m, 3H), 4.02-3.98 (m, 2H), 3.72 (m, 2H), 3.34 (m, 1H), 2.54-2.31 (m, 2H), 2.35-1.65 (m, 7H), 1.48-1.35 (m, 3H), 1.08(m, 1H), 0.87 (m, 3H). MS m/z (ESI): 633.33 [M+H]⁺

### Synthetic Example 53

### (6aS,6bR,7S,8aS,8bS,11aR,12aS,12bS)-6b-Fluoro-7-hydroxy-8b-(2-hydroxyacetyl)-6a,8a-dimeth yl-10-(2-(((2-oxotetrahydrofuran-3-yl)thio)methyl)pyrimidin-5-yl)-1,2,6a,6b,7,8,8a,8b,11a,12,12a, 12b-dodecahydro-4H-naphtho[2',1':4,5]indeno[1,2-d][1,3]dioxol-4-one (53)

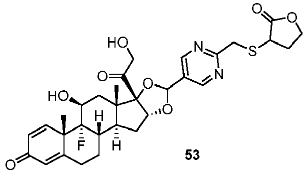

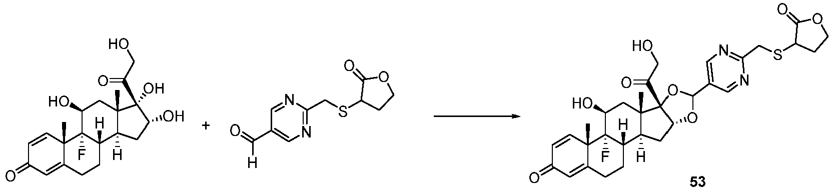

(9α,11β,16α)-9-Fluoro-11,16,17,21-tetrahydroxy-pregna-1,4-diene-3,20-dione (0.5 g, 1.27 mmol), 2-(((2-oxotetrahydrofuran-3-yl)thio)methyl)pyrimidine-5-carbaldehyde (0.36 g, 1.52 mmol), and 30 mL of acetonitrile were added to a reaction flask. Perchloric acid (0.3 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **53** (0.39 g, yield: 50%). ¹H NMR (400 MHz, d₆-DMSO): δ 8.85 (m, 2H), 6.99 (m, 1H), 6.38 (m, 1H), 6.28 (s, 1H), 6.03 (m, 1H), 5.10 (s, 1H), 4.98 (s, 1H), 4.67 (m, 2H), 4.37-4.25 (m, 2H), 3.96 (m, 1H), 3.75 (s, 2H), 3.35 (m, 1H), 2.56-2.37 (m, 4H), 2.31-1.85 (m, 6H), 1.79-1.69 (m, 2H), 1.58-1.38 (m, 3H), 1.02 (m, 1H), 0.85 (m, 3H). MS m/z (ESI): 615.38 [M+H]⁺

### Synthetic Example 54

### (6aS,6bR,7S,8aS,8bS,11aR,12aS,12bS)-6b-Chloro-7-hydroxy-8b-(2-hydroxyacetyl)-6a,8a-dimeth yl-10-(2-(((2-oxotetrahydrofuran-3-yl)thio)methyl)pyrimidin-5-yl)-1,2,6a,6b,7,8,8a,8b,11a,12,12a, 12b-dodecahydro-4H-naphtho[2',1':4,5]indeno[1,2-d][1,3]dioxol-4-one (54)

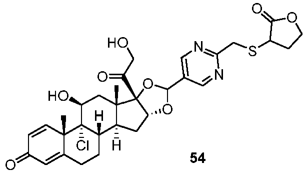

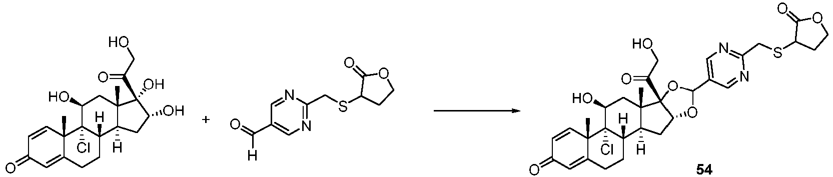

(9α,11β,16α)-9-Chloro-11,16,17,21-tetrahydroxy-pregna-1,4-diene-3,20-dione (0.5 g, 1.22 mmol), 2-(((2-oxotetrahydrofuran-3-yl)thio)methyl)pyrimidine-5-carbaldehyde (0.35 g, 1.46 mmol), and 30 mL of acetonitrile were added to a reaction flask. Perchloric acid (0.3 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **54** (0.41 g, yield: 53%). ¹H NMR (400 MHz, d₆-DMSO): δ 8.79 (m, 2H), 7.02 (m, 1H), 6.35 (m, 1H), 6.31 (s, 1H), 6.02 (m, 1H), 5.04 (s, 1H), 4.94 (s, 1H), 4.69 (m, 2H), 4.35-4.24 (m, 2H), 3.94 (m, 1H), 3.68 (s, 2H), 3.33 (m, 1H), 2.56-2.37 (m, 4H), 2.33-1.87 (m, 6H), 1.74-1.68 (m, 2H), 1.55-1.42 (m, 3H), 1.22 (m, 1H), 0.97 (m, 3H). MS m/z (ESI): 631.25 [M+H]⁺

### Synthetic Example 55

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((2-oxotetrahydrofuran-3-yl)sulfinyl)methyl)phenyl)-1,2,6a,6b,7,8,8a,8b,11a,12,1 2a,12b-dodecahydro-4H-naphtho[2',1':4,5]indeno[1,2-d][1,3]dioxol-4-one (55)

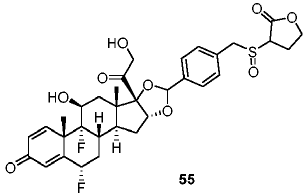

### 4-(((2-Oxotetrahydrofuran-3-yl)sulfinyl)methyl)benzaldehyde

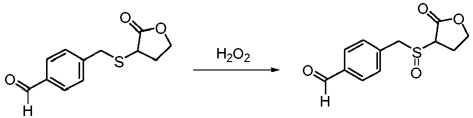

4-(((2-Oxotetrahydrofuran-3-yl)thio)methyl)benzaldehyde (1 g, 4.23 mmol), 10 mL of 30% hydrogen peroxide, and 20 mL of acetonitrile were added to a reaction flask and allowed to react at room temperature for 24 hours. After completion of the reaction, water and ethyl acetate were added for extraction and liquid-liquid separation. The organic phase was washed successively with water and saturated brine and concentrated under reduced pressure. The crude product was separated and purified by silica gel column chromatography (petroleum ether/ethyl acetate = 2/1) and concentrated under reduced pressure to obtain 4-(((2-oxotetrahydrofuran-3-yl)sulfinyl)methyl)benzaldehyde (0.9 g, yield: 83%). MS m/z (ESI): 253.11 [M+H]⁺

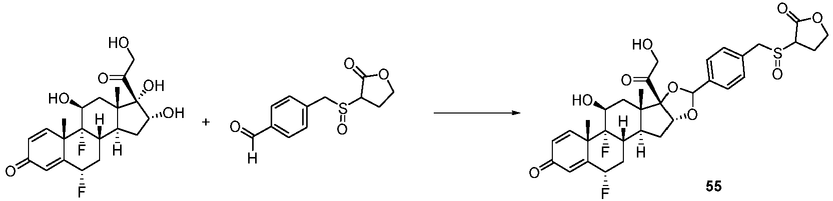

(6α,9α,11β,16α)-6,9-Difluoro-11,16,17,21-tetrahydroxy-pregna-1,4-diene-3,20-dione (0.5 g, 1.21 mmol), 4-(((2-oxotetrahydrofuran-3-yl)sulfinyl)methyl)benzaldehyde (0.37 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 55 (0.43 g, yield: 55%). ¹H NMR (400 MHz, d₆-DMSO): δ 8.83 (m, 2H), 7.01 (m, 1H), 6.41 (m, 1H), 6.33 (s, 1H), 6.05 (m, 1H), 5.01 (s, 1H), 4.88 (s, 1H), 4.79 (m, 2H), 4.35-4.08 (m, 5H), 3.72 (m, 2H), 3.52 (m, 1H), 2.54-2.31 (m, 4H), 2.35-1.65 (m, 7H), 1.48-1.35 (m, 3H), 1.08 (m, 1H), 0.85 (m, 3H). MS m/z (ESI): 647.31 [M+H]⁺

### Synthetic Example 56

### (6aS,6bR,7S,8aS,8bS,11aR,12aS,12bS)-6b-Fluoro-7-hydroxy-8b-(2-hydroxyacetyl)-6a,8a-dimeth yl-10-(4-(((2-oxotetrahydrofuran-3-yl)sulfinyl)methyl)phenyl)-1,2,6a,6b,7,8,8a,8b,11a,12,12a,12b -dodecahydro-4H-naphtho[2',1':4,5]indeno[1,2-d][1,3]dioxol-4-one (56)

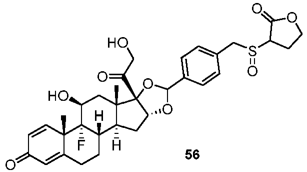

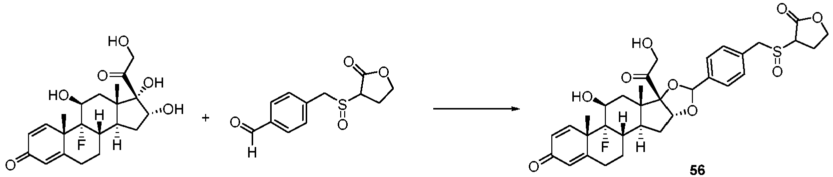

(9α,11β,16α)-9-Fluoro-11,16,17,21-tetrahydroxy-pregna-1,4-diene-3,20-dione (0.5 g, 1.27 mmol), 4-(((2-oxotetrahydrofuran-3-yl)sulfinyl)methyl)benzaldehyde (0.38 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound 56 (0.38 g, yield: 48%). ¹H NMR (400 MHz, d₆-DMSO): δ 8.85 (m, 2H), 6.99 (m, 1H), 6.38 (m, 1H), 6.28 (s, 1H), 6.03 (m, 1H), 5.10 (s, 1H), 4.98 (s, 1H), 4.67-4.35 (m, 4H), 4.05 (m, 1H), 3.75 (s, 2H), 3.35 (m, 1H), 2.56-2.37 (m, 4H), 2.31-1.85 (m, 6H), 1.79-1.69 (m, 4H), 1.58-1.38 (m, 3H), 1.02 (m, 1H), 0.87 (m, 3H). MS m/z (ESI): 629.32 [M+H]⁺

### Synthetic Example 57

### (6aS,6bR,7S,8aS,8bS,11aR,12aS,12bS)-6b-Chloro-7-hydroxy-8b-(2-hydroxyacetyl)-6a,8a-dimeth yl-10-(4-(((2-oxotetrahydrofuran-3-yl)sulfinyl)methyl)phenyl)-1,2,6a,6b,7,8,8a,8b,11a,12,12a,12b -dodecahydro-4H-naphtho[2',1':4,5]indeno[1,2-d][1,3]dioxol-4-one (57)

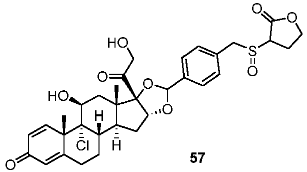

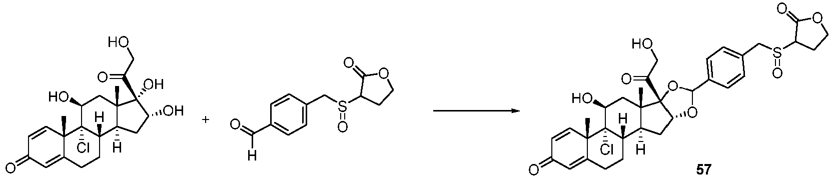

(9α,11β,16α)-9-Chloro-11,16,17,21-tetrahydroxy-pregna-1,4-diene-3,20-dione (0.5 g, 1.22 mmol), 4-(((2-oxotetrahydrofuran-3-yl)sulfinyl)methyl)benzaldehyde (0.37 g, 1.46 mmol), and 30 mL of acetonitrile were added to a reaction flask. Perchloric acid (0.3 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **57** (0.43 g, yield: 55%). ¹H NMR (400 MHz, d₆-DMSO): δ 8.79 (m, 2H), 7.02 (m, 1H), 6.35 (m, 1H), 6.31 (s, 1H), 6.02 (m, 1H), 5.04 (s, 1H), 4.94 (s, 1H), 4.69-4.36 (m, 4H), 4.054 (m, 1H), 3.68 (s, 2H), 3.33 (m, 1H), 2.56-2.37 (m, 4H), 2.33-1.87 (m, 6H), 1.74-1.68 (m, 4H), 1.55-1.42 (m, 3H), 1.22 (m, 1H), 0.86 (m, 3H). MS m/z (ESI): 645.32 [M+H]⁺

### Synthetic Example 58

### (2S,6aS,6bR,7S,8aS,8bS,11aR,12aS,12bS)-2,6b-Difluoro-7-hydroxy-8b-(2-hydroxyacetyl)-6a,8a-dimethyl-10-(4-(((2-oxotetrahydrofuran-3-yl)sulfonyl)methyl)phenyl)-1,2,6a,6b,7,8,8a,8b,11a,12, 12a,12b-dodecahydro-4H-naphtho[2',1':4,5]indeno[1,2-d][1,3]dioxol-4-one (58)

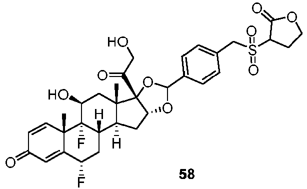

### 4-(((2-Oxotetrahydrofuran-3-yl)sulfonyl)methyl)benzaldehyde

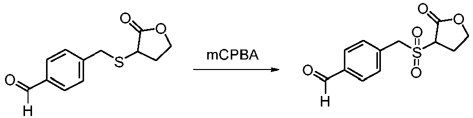

4-(((2-Oxotetrahydrofuran-3-yl)thio)methyl)benzaldehyde (1 g, 4.23 mmol), m-chloroperoxybenzoic acid (3.6 g, 0.02 mol), and 30 mL of acetonitrile were added to a reaction flask and allowed to react at room temperature for 8 hours. After completion of the reaction, water and ethyl acetate were added for extraction and liquid-liquid separation. The organic phase was washed successively with water and saturated brine and concentrated under reduced pressure. The crude product was separated and purified by silica gel column chromatography (petroleum ether/ethyl acetate = 2/1) and concentrated under reduced pressure to obtain 4-(((2-oxotetrahydrofuran-3-yl)sulfonyl)methyl) benzaldehyde (0.84 g, yield: 74%). MS m/z (ESI): 269.15 [M+H]⁺

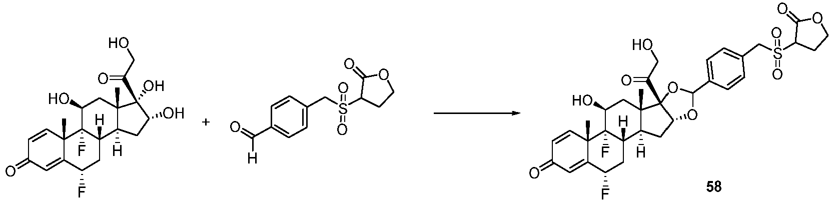

(6α,9α,11β,16α)-6,9-Difluoro-11,16,17,21-tetrahydroxy-pregna-1,4-diene-3,20-dione (0.5 g, 1.21 mmol), 4-(((2-oxotetrahydrofuran-3-yl)sulfonyl)methyl) benzaldehyde (0.39 g, 1.45 mmol), and 30 mL of acetonitrile were added to a reaction flask. Perchloric acid (0.3 mL, 3.64 mmol) was slowly added dropwise, and the mixture was allowed to react at room temperature. TLC monitoring was performed until no (6α,9α,11β,16α)-6,9-difluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **58** (0.42 g, yield: 52%). ¹H NMR (400 MHz, d₆-DMSO): δ 8.83 (m, 2H), 7.01 (m, 1H), 6.41 (m, 1H), 6.33 (s, 1H), 6.05 (m, 1H), 5.01 (s, 1H), 4.88 (s, 1H), 4.79 (m, 2H), 4.42-4.12 (m, 5H), 3.86 (m, 2H), 3.52 (m, 1H), 2.54-2.31 (m, 4H), 2.35-1.65 (m, 7H), 1.48-1.35 (m, 3H), 1.08 (m, 1H), 0.85 (m, 3H). MS m/z (ESI): 663.32 [M+H]⁺

### Synthetic Example 59

### (6aS,6bR,7S,8aS,8bS,11aR,12aS,12bS)-6b-Fluoro-7-hydroxy-8b-(2-hydroxyacetyl)-6a,8a-dimeth yl-10-(4-(((2-oxotetrahydrofuran-3-yl)sulfonyl)methyl)phenyl)-1,2,6a,6b,7,8,8a,8b,11a,12,12a,12b -dodecahydro-4H-naphtho[2',1':4,5]indeno[1,2-d][1,3]dioxol-4-one(59)

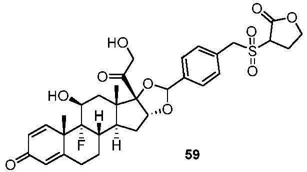

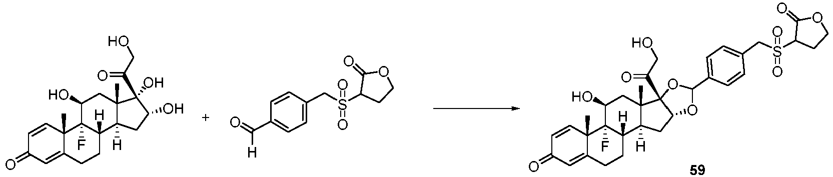

(9α,11β,16α)-9-Fluoro-11,16,17,21-tetrahydroxy-pregna-1,4-diene-3,20-dione (0.5 g, 1.27 mmol), 4-(((2-oxotetrahydrofuran-3-yl)sulfonyl)methyl)benzaldehyde (0.4 g, 1.52 mmol), and 30 mL of acetonitrile were added to a reaction flask. Perchloric acid (0.32 mL, 3.8 mmol) was slowly added dropwise, and the mixture was allowed to react at room temperature. TLC monitoring was performed until no (9α,11β,16α)-9-fluoro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **59** (0.44 g, yield: 52%). ¹H NMR (400 MHz, d₆-DMSO): δ 8.85 (m, 2H), 6.99 (m, 1H), 6.38 (m, 1H), 6.28 (s, 1H), 6.03 (m, 1H), 5.10 (s, 1H), 4.98 (s, 1H), 4.78-4.42 (m, 4H), 4.21 (m, 1H), 3.75 (s, 2H), 3.35 (m, 1H), 2.56-2.37 (m, 4H), 2.31-1.85 (m, 6H), 1.79-1.69 (m, 4H), 1.58-1.38 (m, 3H), 1.02 (m, 1H), 0.87 (m, 3H). MS m/z (ESI): 645.23 [M+H]⁺

### Synthetic Example 60

### (6aS,6bR,7S,8aS,8bS,11aR,12aS,12bS)-6b-Chloro-7-hydroxy-8b-(2-hydroxyacetyl)-6a,8a-dimeth yl-10-(4-(((2-oxotetrahydrofuran-3-yl)sulfonyl)methyl)phenyl)-1,2,6a,6b,7,8,8a,8b,11a,12,12a,12b -dodecahydro-4H-naphtho[2',1':4,5]indeno[1,2-d][1,3]dioxol-4-one (60)

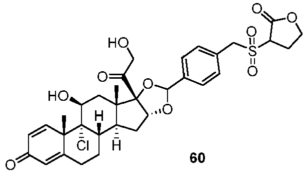

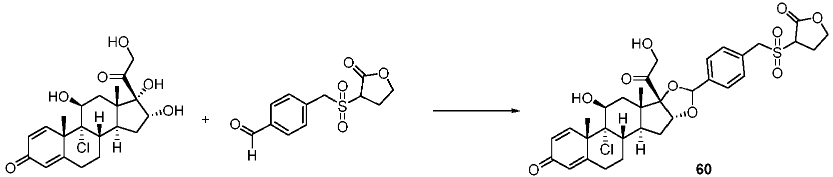

(9α,11β,16α)-9-Chloro-11,16,17,21-tetrahydroxy-pregna-1,4-diene-3,20-dione (0.5 g, 1.22 mmol), 4-(((2-oxotetrahydrofuran-3-yl)sulfonyl)methyl)benzaldehyde (0.39 g, 1.46 mmol), and 30 mL of acetonitrile were added to a reaction flask. Perchloric acid (0.3 mL, 3.65 mmol) was slowly added dropwise, and the mixture was allowed to react at room temperature. TLC monitoring was performed until no (9α,11β,16α)-9-chloro-11,16,17,21-tetrahydroxy-pregna-1,4-diene-3,20-dione was detected. After completion of the reaction, the mixture was concentrated under reduced pressure. Water and dichloromethane were added for extraction and liquid-liquid separation. The organic phases were combined, washed with saturated brine, and concentrated under reduced pressure. The crude product was separated and purified by silica gel column chromatography (dichloromethane/acetone = 4/1) and concentrated under reduced pressure to obtain compound **60** (0.43 g, yield: 54%). ¹H NMR (400 MHz, d₆-DMSO): δ 8.79 (m, 2H), 7.02 (m, 1H), 6.35 (m, 1H), 6.31 (s, 1H), 6.02 (m, 1H), 5.04 (s, 1H), 4.94 (s, 1H), 4.72-4.44 (m, 4H), 4.21 (m, 1H), 3.68 (s, 2H), 3.33 (m, 1H), 2.56-2.37 (m, 4H), 2.33-1.87 (m, 6H), 1.74-1.68 (m, 4H), 1.55-1.42 (m, 3H), 1.22 (m, 1H), 0.86 (m, 3H). MS m/z (ESI): 661.32 [M+H]⁺

### Pharmacological Example 1: Assay for Agonist Activity on Glucocorticoid Receptor (GR)

### 1.1 Preparation of stock solutions

Test compound stock solutions: The test compounds (compounds 1 to 60) were accurately weighed, dissolved in DMSO to prepare stock solutions at a concentration of 20 mM, and stored at -20 °C.

### 1.2 Preparation of test sample solutions

The test compounds were subjected to a 4-fold serial dilution starting from an initial concentration of 10 µ M, generating a 10-point concentration gradient with duplicate wells for each concentration. The blank control consisted of 0.1 % DMSO.

### 1.3 Cell culture

The HEK293 stable cell line transiently transfected with glucocorticoid receptor (GR) (ICE Bioscience, Lot No. TYP-20220519) was cultured in Dulbecco's Modified Eagle Medium (DMEM) supplemented with 10% fetal bovine serum (FBS) and 200 µ g/ml Hygromycin B in a cell incubator containing 5% CO₂ at 37 °C. The spent medium was aspirated and the cells were washed once with phosphate-buffered saline (PBS). Then, 1 mL of TrypLE^{™} Express solution was added, and the cells were incubated at 37 °C for approximately 2 min. When the cells detached from the bottom of the cell culture dish, approximately 5 mL of pre-warmed complete medium at 37 °C was added. The cell suspension was gently pipetted with a pipette to dissociate clumped cells. The cell suspension was transferred to a sterile centrifuge tube and centrifuged at 1000 rpm for 5 min to pellet the cells for experiments or subculture.

### 1.4 Assay for the agonist activity of compounds on GR receptor

HEK293 cells were digested and seeded into 6-cm culture dishes. The pBIND-GR plasmid was transfected into the HEK293 cells using a transfection reagent, resulting in HEK293 cells successfully transfected with the pBIND-GR plasmid. The cells were resuspended in phenol red-free DMEM medium containing 5% charcoal-stripped serum, counted, and then seeded into 96-well plates. The test compounds were added, and the plates were then incubated in an incubator. The luminescence signal values were measured using a multimode microplate reader following the instructions of the luciferase assay kit. The EC₅₀ values of each test compound were calculated using the nonlinear fitting formula with GraphPad Prism 8.0 software.

### 1.5 Results

**Table 1. Agonist activity for glucocorticoid receptor**

| Group | GR agonist EC₅₀ (nM) | Group | GR agonist EC₅₀ (nM) |
|---|---|---|---|
| Compound 1 | 20.33 | Compound 31 | 8.13 |
| Compound 2 | 12.17 | Compound 32 | 3.67 |
| Compound 3 | 17.60 | Compound 33 | 4.38 |
| Compound 4 | 11.84 | Compound 34 | 0.10 |
| Compound 5 | 5.23 | Compound 35 | 0.21 |
| Compound 6 | 8.69 | Compound 36 | 0.12 |
| Compound 7 | 1.21 | Compound 37 | 0.78 |
| Compound 8 | 0.33 | Compound 38 | 0.11 |
| Compound 9 | 3.27 | Compound 39 | 3.31 |
| Compound 10 | 2.65 | Compound 40 | 30.72 |
| Compound 11 | 0.81 | Compound 41 | 6.19 |
| Compound 12 | 4.06 | Compound 42 | 19.50 |
| Compound 13 | 3.43 | Compound 43 | 7.71 |
| Compound 14 | 7.71 | Compound 44 | 15.15 |
| Compound 15 | 6.12 | Compound 45 | 1.62 |
| Compound 16 | 0.43 | Compound 46 | 0.23 |
| Compound 17 | 0.30 | Compound 47 | 0.34 |
| Compound 18 | 1.62 | Compound 48 | 0.37 |
| Compound 19 | 0.91 | Compound 49 | 1.12 |
| Compound 20 | 0.33 | Compound 50 | 0.98 |
| Compound 21 | 0.63 | Compound 51 | 0.82 |
| Compound 22 | 0.55 | Compound 52 | 6.92 |
| Compound 23 | 0.21 | Compound 53 | 8.31 |
| Compound 24 | 1.12 | Compound 54 | 5.64 |
| Compound 25 | 1.30 | Compound 55 | 1.33 |
| Compound 26 | 0.37 | Compound 56 | 1.56 |
| Compound 27 | 1.82 | Compound 57 | 0.86 |
| Compound 28 | 4.24 | Compound 58 | 3.67 |
| Compound 29 | 3.66 | Compound 59 | 4.01 |
| Compound 30 | 7.20 | Compound 60 | 3.50 |

The results showed that all compounds disclosed herein exhibited agonist activity toward the glucocorticoid receptor (GR). Among them, compounds 7, 8, 10, 11, 16, 17, 18, 19, 20, 21, 22, 23, 24, 25, 26, 27, 34, 35, 36, 37, 38, 45, 46, 47, 48, 49, 50, 51, 55, 56, and 57 showed EC₅₀ values below 3 nM. Furthermore, compounds 8, 11, 16, 17, 19, 20, 21, 22, 23, 26, 34, 35, 36, 37, 38, 46, 47, 48, 50, 51, and 57 showed EC₅₀ values below 1 nM.

### Pharmacological Example 2: Assay for Agonist Activity on Mineralocorticoid Receptor (MR) and Progesterone Receptor (PR)

### 2.1 Preparation of stock solutions

Test compound stock solutions: The test compounds (compounds 4, 5, 7, 8, 9, 11, 13, 16, 17, 18, 22, 23, 24, 34, 35, 36, 38, 41, 45, 47, 53, 55, and 59) were accurately weighed, dissolved in DMSO to prepare stock solutions at a concentration of 20 mM, and stored at -20 °C.

### 2.2 Preparation of test sample solutions

The test compounds were subjected to a 4-fold serial dilution starting from an initial concentration of 50 µ M, generating a 12-point concentration gradient with duplicate wells for each concentration. The blank control consisted of 0.1% DMSO.

### 2.3 Cell culture

The HEK293 stable cell lines transiently transfected with mineralocorticoid receptor (MR) and progesterone receptor (PR) (ICE Bioscience, Lot No. TYP-20220819, TPJ-20220812) were cultured in Dulbecco's Modified Eagle Medium (DMEM) supplemented with 10% fetal bovine serum (FBS) and 200 µ g/ml Hygromycin B in a cell incubator containing 5% CO₂ at 37 °C. The spent medium was aspirated, and the cells were washed once with phosphate-buffered saline (PBS). Then, 1 mL of TrypLE^{™} Express solution was added, and the cells were incubated at 37 °C for approximately 2 min. When the cells detached from the bottom of the cell culture dish, approximately 5 mL of pre-warmed complete medium at 37 °C was added. The cell suspension was gently pipetted with a pipette to dissociate clumped cells. The cell suspension was transferred to a sterile centrifuge tube and centrifuged at 1000 rpm for 5 min to pellet the cells for experiments or subculture..

### 2.4 Assay for the agonist activity of compounds on MR and PR

HEK293 cells were digested and seeded into 6-cm culture dishes. The pBIND-MR and pBIND-PR plasmids were separately transfected into the HEK293 cells using a transfection reagent, resulting in HEK293 cells successfully transfected with the pBIND-MR and pBIND-PR plasmids, respectively. The cells were resuspended in phenol red-free DMEM medium containing 5% charcoal-stripped serum, counted, and then seeded into 96-well plates. The test compounds were added, and the plates were then incubated in an incubator. The luminescence signal values were measured using a multimode microplate reader following the instructions of the luciferase assay kit. The EC₅₀ values of each test compound were calculated using the nonlinear fitting formula with GraphPad Prism 8.0 software.

### 2.5 Results

**Table 2. Agonist activity for mineralocorticoid receptor and progesterone receptor**

| Group | Nuclear receptor enzyme activity (nM) | | | Target selectivity | |
|---|---|---|---|---|---|
| | GR | MR | PR | MR/GR | PR/GR |
| Compound 4 | 11.84 | > 50000 | > 50000 | > 4223 | > 4223 |
| Compound 5 | 5.23 | > 50000 | > 50000 | > 9560 | > 9560 |
| Compound 7 | 1.21 | > 50000 | > 50000 | > 4.1×10⁴ | > 4.1×10⁴ |
| Compound 8 | 0.33 | > 50000 | > 50000 | > 1.5×10⁵ | > 1.5×10⁵ |
| Compound 9 | 3.27 | > 50000 | > 50000 | > 1.5×10⁴ | > 1.5×10⁴ |
| Compound 11 | 0.81 | > 50000 | > 50000 | > 6.2×10⁴ | > 6.2×10⁴ |
| Compound 13 | 3.43 | > 50000 | > 50000 | > 1.4×10⁴ | > 1.4×10⁴ |
| Compound 16 | 0.43 | > 50000 | > 50000 | > 1.2×10⁵ | > 1.2×10⁵ |
| Compound 17 | 0.30 | > 50000 | > 50000 | > 1.7×10⁵ | > 1.7×10⁵ |
| Compound 18 | 1.62 | > 50000 | > 50000 | > 3.1×10⁴ | > 3.1×10⁴ |
| Compound 22 | 0.55 | > 50000 | > 50000 | > 9.1×10⁴ | > 9.1×10⁴ |
| Compound 23 | 0.21 | > 50000 | > 50000 | > 2.4×10⁵ | > 2.4×10⁵ |
| Compound 24 | 1.12 | > 50000 | > 50000 | > 4.5×10⁴ | > 4.5×10⁴ |
| Compound 34 | 0.10 | > 50000 | > 50000 | > 5.0×10⁵ | > 5.0×10⁵ |
| Compound 35 | 0.21 | > 50000 | > 50000 | > 2.4×10⁵ | > 2.4×10⁵ |
| Compound 36 | 0.12 | > 50000 | > 50000 | > 4.2×10⁵ | > 4.2×10⁵ |
| Compound 38 | 0.11 | > 50000 | > 50000 | > 4.5×10⁵ | > 4.5×10⁵ |
| Compound 41 | 6.19 | > 50000 | > 50000 | > 8077 | > 8077 |
| Compound 45 | 1.62 | > 50000 | > 50000 | > 3.1×10⁴ | > 3.1×10⁴ |
| Compound 47 | 0.34 | > 50000 | > 50000 | > 1.5×10⁵ | > 1.5×10⁵ |
| Compound 53 | 8.31 | > 50000 | > 50000 | 6017 | 6017 |
| Compound 55 | 1.33 | > 50000 | > 50000 | > 3.8×10⁴ | > 3.8×10⁴ |
| Compound 59 | 4.01 | > 50000 | > 50000 | > 1.2×10⁴ | > 1.2×10⁴ |

The results showed that none of the compounds disclosed herein exhibited agonist activity toward the mineralocorticoid receptor (MR) and the progesterone receptor (PR). Compared with the MR receptor and the PR receptor, the compounds displayed enhanced selectivity for the glucocorticoid receptor (GR) .

Under the same experimental conditions, compounds 20, 21, 26, 37, 46, 48, 51, and others as disclosed herein demonstrated target selectivity comparable to that of compound 23; therefore, a detailed description will not be reiterated..

### Pharmacological Example 3: Determination of Stability in Plasma in Vitro

### 3.1 Preparation of working solution

Test compound 8 was accurately weighed and dissolved in DMSO to prepare a stock solution at a concentration of 10 mM. The stock solution was diluted with acetonitrile to obtain a working solution at a concentration of 100 µ M. The solution should be freshly prepared prior to use and maintained at room temperature during application.

### 3.2 Plasma incubation

**Table 3. Plasma incubation experimental conditions**

| Test compound | Compound 8 |
|---|---|
| Incubation system | Human, Mouse |
| Final concentration of test compound | 1 µM |
| Incubation temperature | 37 °C |
| Sampling time points | 0, 3, 5, 15, 30, 60 min |
| Experimental group setup | Test drug group, two-sample |
| Reaction termination method | Terminate the reaction with 3 volumes of ice-cold precipitation agent |
| Analytical determination | Analysis by liquid chromatography-tandem mass spectrometry (LC-MS/MS) |

990 µ L of human and mouse plasma (N = 2) were separately pipetted and pre-incubated at 37 °C for 5 min. Then, 10 µ L of the test compound working solution was added and vortexed thoroughly (the final concentration of the test compound was 1 µ M). Immediately, 50 µ L of the plasma sample was added to 150 µ L of ice-cold precipitation agent containing the internal standard and vortexed thoroughly as the 0-time sample. The remaining plasma sample was continuously incubated at 37 °C, and 50 µ L of the plasma sample was aspirated and added to 150 µ L of ice-cold precipitation agent containing the internal standard at preset sampling time points, followed by thorough vortexing. The collected samples were stored in a freezer at -60 to -90 °C until analysis.

### 3.3 Sample analysis

The concentration of the test compound was determined by LC-MS/MS, and its degradation was monitored. (semi-quantitative determination was conducted by comparing the peak area of the analyte to that of the internal standard).

### 3.4 Results

**Table 4. Half-life T_{1/2} (min) in plasma of different species**

| Group | Human plasma | Mouse plasma |
|---|---|---|
| Compound 8 | 21.5 | 27.0 |

The results showed that compound 8 disclosed herein had a half-life of less than 30 min in both human and mouse plasma, exhibiting the characteristic of plasma instability.

Additionally, compound 8 disclosed herein was relatively stable in animal lung tissue homogenate, skin tissue homogenate, and aqueous humor.

Under the same experimental conditions, compounds 16, 17, 20, 21, 22, 23, 26, 34, 35, 36, 37, 46, 47, 48, 51, and others of the present disclosure all exhibited the characteristic of plasma instability and were relatively stable in animal lung tissue homogenate, skin tissue homogenate, and aqueous humor.

### Pharmacological Example 4: Pharmacodynamic Experiment on Anti-Asthma

### 4.1 Preparation method of test samples

Test samples: The jet-milled compounds 8, 11, and 23 were accurately weighed and suspended in a citrate-sodium citrate buffer (pH 5.4) containing 2% Tween 80 to prepare suspensions at a concentration of 0.3 mg/ml as the test samples. The suspensions were stored at room temperature protected from light until use.

Positive control drug: Budesonide Suspension for Inhalation (specification: 2 mL: 1 mg; AstraZeneca Inc.; Lot No. : 327392) was diluted with injection-grade sodium chloride solution to 0.3 mg/ml as the positive control drug, and stored at room temperature protected from light until use.

### 4.2 Model preparation

Sixty female BALB/c mice underwent a one-week adaptive feeding period. Ten mice were randomly selected as the blank control group, while 50 mice were selected to establish asthma models. On days 0, 7, and 14, the 50 model mice were intraperitoneally injected with 0.2 mL of sensitization solution containing 50 µ g of OVA and 2 mg of Al(OH)₃, while the mice in the blank control group were injected with an equal volume of normal saline.

From days 21 to 23 after sensitization, the mice in the model group and drug administration group were exposed to a 30-minute aerosol challenge with 5% OVA, while the mice in the blank control group were exposed to normal saline aerosol for the same duration.

### 4.3 Grouping and drug administration

Thirty minutes before daily challenge, 50 µL of normal saline was administered intratracheally to the mice in the blank control group and model group, and 50 µL of the corresponding drugs were administered intratracheally to the mice in the compound 8 group, compound 11 group, compound 23 group, and positive drug budesonide group, once daily for 3 consecutive days.

**Table 5. Animal grouping and drug administration dosages**

| Group | Drug concentration (mg/mL) | Administration volume (µL) | Number of animals |
|---|---|---|---|
| Blank control | - | 50 | 10 |
| Model | - | 50 | 10 |
| Budesonide | 0.3 | 50 | 10 |
| Compound 8 | 0.3 | 50 | 10 |
| Compound 11 | 0.3 | 50 | 10 |
| Compound 23 | 0.3 | 50 | 10 |

### 4.4 Determination of airway hyperresponsiveness

Airway responsiveness was measured in all groups of mice after the last challenge. The mice were placed into a plethysmography chamber, and baseline Penh values were recorded. Subsequently, the mice were challenged with aerosolized methacholine at concentrations from low to high of 0, 6.25, and 12.5 mg/mL, with an aerosol volume of 100 µ L each time for 60 s. After completion of atomization at each concentration, the mice were observed for tachypnea, head scratching, dysphoria, and other hypoxic manifestations. Penh values were then recorded for 3 min and averaged to compare the airway responsiveness of each group of mice.

### 4.5 Sample collection and detection of inflammatory cytokines in alveolar lavage fluid

At the end of airway hyperresponsiveness testing in mice, the mice were euthanized by cervical dislocation and fixed on a dissection tray. The abdomens and thoracic cavities of the mice were opened, and the skin and excess tissues of the neck were excised to expose the trachea of the mice. A small incision was made transversely on the trachea with a small scissors. A pre-treated 1 mL syringe needle was inserted and fastened with surgical thread. A 1 mL syringe was used to slowly inject 0.5 mL of pre-chilled phosphate-buffered saline (PBS) into the lungs of the mice, followed by slow aspiration. Each mouse was lavaged once, and a total of approximately 0.4 ml of lavage fluid was collected. The bronchoalveolar lavage fluid was centrifuged at 3000 rpm for 5 min. After centrifugation, the supernatant was aliquoted, and the levels of inflammatory cytokines IL-6 and IL-12 in the bronchoalveolar lavage fluid were determined using ELISA kits.

### 4.6 Data statistics

Statistical analysis was performed using SPSS software. Measurement data were expressed as mean ± standard deviation. One-way ANOVA was used for intergroup comparisons. For data with homogeneous variance, LSD test was used; for data with heterogeneous variance, Dunnett-t test was used. A value of *P* < 0.05 was considered statistically significant.

### 4.7 Results

### 4.7.1 Effects on airway hyperresponsiveness in mice

**Table 6. Effects on Penh values in mice**

| Group | Methacholine concentration (mg/ml) | | |
|---|---|---|---|
| | 0 | 6.25 | 12.5 |
| Blank control | 0.92 ± 0.15 | 1.28 ± 0.22 | 1.73 ± 0.15 |
| Model | 1.29 ± 0.31^{#} | 2.75 ± 0.53^{###} | 3.34 ± 0.71^{###} |
| Budesonide | 1.12 ± 0.30 | 1.65 ± 0.29*** | 2.14 ± 0.38** |
| Compound 8 | 1.17 ± 0.23 | 1.78 ± 0.46** | 2.07 ± 0.66** |
| Compound 11 | 1.08 ± 0.18 | 1.45 ± 0.31*** | 1.92 ± 0.34*** |
| Compound 23 | 1.12 ± 0.30 | 1.55 ± 0.29*** | 1.84 ± 0.38*** |

| | | | |
|---|---|---|---|
| Note: Compared with the blank control group, *^{##}P* < 0.01, *^{###}P* < 0.001; compared with the model group, **P <* 0.05, ***P <* 0.01, ****P* < 0.001. | | | |

After the mice were sensitized with OVA + Al(OH)₃ for 3 weeks, then challenged with 5% OVA for 3 consecutive days, and then challenged with 6.25 mg/ml and 12.5 mg/ml methacholine, the Penh values were significantly higher than those of the blank control group (*P* < 0.001), demonstrating the successful establishment of the asthma model in mice.

Compared with the model group, aerosol administration of the positive drug budesonide before challenge significantly decreased the Penh values in mice (*P* < 0.01); aerosol administration of compounds 8, 11, and 23 before challenge significantly decreased the Penh values in mice, with compounds 11 and 23 showing extremely significant differences (*P* < 0.001).

Under the same experimental conditions, compounds 16, 17, 20, 21, 22, 26, 34, 35, 36, 37, 46, 47, 48, 51, and others of the present disclosure exhibited pharmacological effects similar to those of compound 23. All of them significantly decreased the Penh values in mice, showing extremely significant differences (*P* < 0.001) compared with the model group..

### 4.7.2 Effects on inflammatory cytokines in bronchoalveolar lavage fluid of mice

**Table 7. Effects on inflammatory cytokines in bronchoalveolar lavage fluid of mice**

| Group | IL-6 (pg/mL) | IL-12 (pg/mL) |
|---|---|---|
| Blank control | 13.4 ± 22.0 | 3.6 ± 6.7 |
| Model | 569.8 ± 71.8^{###} | 83.7 ± 32.1^{###} |
| Budesonide | 21.5 ± 6.8*** | 17.5 ± 4.9*** |
| Compound 8 | 19.9 ± 5.3*** | 20.1 ± 7.1*** |
| Compound 11 | 16.3 ± 7.9*** | 11.1 ± 3.7*** |
| Compound 23 | 11.1 ± 5.5*** | 7.0 ± 3.9*** |

| | | |
|---|---|---|
| Note: Compared with the blank control group, ###*P* < 0.001; compared with the model group, ****P* < 0.001. | | |

After the mice were sensitized with OVA + Al(OH)₃ for 3 weeks and then challenged with 5% OVA for 3 consecutive days, the levels of IL-6 and IL-12 in bronchoalveolar lavage fluid significantly increased (*P* < 0.001), demonstrating the successful establishment of the asthma model in mice.

Compared with the model group, aerosol administration of the positive drug budesonide, compounds 8, 11, and 23 before challenge extremely significantly decreased the levels of IL-6 and IL-12 in mice (*P* < 0.001), indicating that compounds 8, 11, and 23 significantly inhibited airway hyperresponsiveness in asthma model mice, suppressed inflammatory cytokines in bronchoalveolar lavage fluid, and had significant anti-inflammatory activity.

Under the same experimental conditions, compounds 16, 17, 20, 21, 22, 26, 34, 35, 36, 37, 46, 47, 48, 51, and others of the present disclosure exhibited pharmacological effects similar to those of compound 23. All of them significantly inhibited airway hyperresponsiveness in asthma model mice, showing extremely significant differences (P < 0.001) compared with the model group.

### Pharmacological Example 5. Pharmacodynamic Experiment on Anti-Uveitis

### 5.1 Preparation method of test samples

Test samples: Compounds 23 and 26 were prepared as 5 mg/5 mL ophthalmic suspensions with reference to the formulation of Fluorometholone Eye Drops, and stored at room temperature protected from light until use.

Positive control drug: Fluorometholone Eye Drops (specification: 5 mL/5 mg; Santen Pharmaceutical (CHINA) Co., Ltd.; Lot No. : J20180068) was used as the positive control drug and stored at room temperature protected from light until use.

### 5.2 Model preparation

Fifty male SD rats underwent a one-week adaptive feeding period. The animals were randomly divided into 5 groups: Group 1 served as the blank control group, and Groups 2 to 5 were the model group, fluorometholone group, compound 23 group, and compound 26 group, respectively. Groups 2 to 5 were used to establish rat uveitis models by single subcutaneous injection of 1 mg/kg LPS (Salmonella; Sigma; Lot No. 0000164114) into the plantar tissue of the hindpaw. Salmonella endotoxin was dissolved in normal saline to prepare an injection solution at a concentration of 2 mg/mL, which was injected subcutaneously at 1 mg/kg into the plantar tissue of one paw of the rats. Group 1 was injected with an equal volume of normal saline.

### 5.3 Grouping and drug administration

After modeling, 40 µL of normal saline was administered to the eyes (bilateral) of the rats in the blank control group and model group, and 40 µL of the corresponding drugs were administered to the eyes (bilateral) of the mice in the compound 23 group, compound 26 group, and positive drug fluorometholone group, 3 times a day for 2 days.

**Table 8. Animal grouping and drug administration dosages**

| Group | Drug concentration (mg/mL) | Administration volume (µL) / eye | Number of animals |
|---|---|---|---|
| Blank control | - | 40 | 10 |
| Model | - | 40 | 10 |
| Fluorometholone | 1 | 40 | 10 |
| Compound 23 | 1 | 40 | 10 |
| Compound 26 | 1 | 40 | 10 |

### 5.4 Ocular injury documentation

At the end of the experiment, photos of the rats' eyes were taken using a slit-lamp microscope, and the differences in ocular lesions of rats among groups were compared and analyzed.

### 5.5 Sample collection and detection of inflammatory cytokines in aqueous humor

Thirty hours after modeling, aqueous humor was collected from both eyes of rats, and partial eyeball tissues of rats were collected. The level of the inflammatory cytokines (TNF-α) was detected using a rat ELISA assay kit.

### 5.6 Data statistics

Statistical analysis was performed using SPSS software. Measurement data were expressed as mean ± standard deviation. One-way ANOVA was used for intergroup comparisons. For data with homogeneous variance, LSD test was used; for data with heterogeneous variance, Dunnett-t test was used. A value of *P* < 0.05 was considered statistically significant.

### 5.7 Results

### 5.7.1 Ocular pathological score

**Table 9. Ocular symptom score in rats**

| Group | Ocular pathological score |
|---|---|
| Blank control | 5.5 ± 0.4 |
| Model | 9.9 ± 0.5^{###} |
| Fluorometholone | 7.1 ± 0.7*** |
| Compound 23 | 7.0 ± 0.5*** |
| Compound 26 | 7.6 ± 0.5*** |

| | |
|---|---|
| Note: Compared with the blank control group, *^{##}P* < 0.01, *^{###}P* < 0.001; compared with the model group, **P <* 0.05, ***P* < 0.01, ****P* < 0.001. | |

After subcutaneous injection of LPS into the plantar tissue of rats, the model group showed significant ocular abnormalities compared with the blank control group, which demonstrated the successful establishment of the uveitis model in rats.

Compared with the model group, administration of the positive drug fluorometholone, compounds 23 and 26 to the rat eyes significantly improved the ocular abnormalities and reduced the ocular pathological score.

Moreover, compounds 23 and 26 provided by the present disclosure also significantly inhibited the ocular inflammatory response in uveitis model rats, suppressed inflammatory cytokines in aqueous humor, and had significant anti-inflammatory activity.

Under the same experimental conditions, compounds 16, 17, 20, 21, 22, 34, 35, 36, 37, 46, 47, 48, 51, and others of the present disclosure exhibited pharmacological effects similar to those of compound 23. All of them significantly improved ocular abnormalities, suppressed inflammatory cytokines in aqueous humor, and had significant anti-inflammatory activity.

### Pharmacological Example 6: Therapeutic Pharmacodynamic Experiment on Dry Eye Syndrome in New Zealand Rabbits

### 6.1 Preparation method of test samples

Test samples: Compounds 20 and 23 were prepared as 5 mg/5 mL ophthalmic suspensions with reference to the formulation of Fluorometholone Eye Drops, and stored at room temperature protected from light until use.

Positive control drug: Fluorometholone Eye Drops (specification: 5 mL/5 mg; Santen Pharmaceutical (CHINA) Co., Ltd.; Lot No.: 1FM6625) was used as the positive control drug and stored at room temperature protected from light until use.

### 6.2 Model preparation

Thirty male New Zealand White rabbits (conventional grade) were prepared. Six rabbits were randomly selected as the blank control group, and the remaining animals were administered 0.1% benzalkonium chloride to the left eye twice daily (morning and evening) , 10 µL/animal each time. On day 6 of modeling, tear secretion was measured using phenol red cotton threads to determine whether the model was successfully established. Once the model was confirmed, the animals were randomly divided into 4 groups based on the length of phenol red cotton threads: model group, fluorometholone group, compound 20 group, and compound 23 group, with 6 animals in each group. During the administration, all animals except the blank control group were administered 10 µL of 0.1% benzalkonium chloride to the left eye once daily to maintain dry eye symptoms..

### 6.3 Grouping and drug administration

50 µL of normal saline was administered to the eyes (bilateral) of New Zealand rabbits in the blank control group and model group, and 50 µL of the corresponding drugs were administered to the eyes (bilateral) of the rabbits in the compound 20 group, compound 23 group, and positive drug fluorometholone group, 3 times a day for 14 consecutive days.

**Table 10. Animal grouping and drug administration dosages**

| Group | Drug concentration (mg/mL) | Administration volume (µL) / eye | Number of animals |
|---|---|---|---|
| Blank control | - | 50 | 6 |
| Model | - | 50 | 6 |
| Fluorometholone | 1 | 50 | 6 |
| Compound 20 | 1 | 50 | 6 |
| Compound 23 | 1 | 50 | 6 |

### 6.4 Tear secretion test

Test time: D₃, D₇, D₁₅;
Test animals: All animals were tested before grouping; all enrolled surviving animals were tested after administration;
Test method: Tear secretion was measured using phenol red cotton threads. The tear secretion test paper was folded and placed at the middle and outer 1/3 of the rabbit palpebral conjunctival sac for 1 min. The test paper was then removed, observed, and recorded for its wetting length. The test was repeated 3 times, and the values were averaged.

### 6.5 Tear film break-up time

Test time: D₃, D₇, D₁₅;
Test animals: All animals were tested before grouping; all enrolled surviving animals were tested after administration;
Test method: 1% Fluorescein sodium was dropped onto the inner side of the rabbit's lower eyelid, 10 µL/animal. The rabbit's eyes were closed and gently massaged to evenly distribute the fluorescein sodium on the eye surface. The rabbit's eyes were then sequentially placed under a slit lamp. The tear film break-up time was observed and recorded as the time from eye opening to the appearance of the first dark spot. If no dark spot appeared, the time was calculated as 60 seconds.

### 6.6 Corneal fluorescein sodium staining

Test time: D₃, D₇, D₁₅;
Test animals: All animals were tested before grouping; all enrolled surviving animals were tested after administration;
Test method: 2 µL of 1% Fluorescein sodium was dropped into the conjunctival sac. After 5 s, the excess fluorescein was rinsed with 0.9% normal saline. The conjunctival staining was observed under a slit lamp with cobalt blue diffused light.

### 6.7 Data statistics

The normality and homogeneity of variance were tested by Levene's test. If there was no statistical significance (P > 0.05), one-way analysis of variance (ANOVA) was used for statistical analysis. If ANOVA was statistically significant (P ≤ 0.05), LSD test (parametric method) was used for comparative analysis. If the variance was heterogeneous (*P* ≤ 0.05), Kruskal-Wallis test was used. If the Kruskal-Wallis test was statistically significant (*P* ≤ 0.05), Dunnett's test (nonparametric method) was used for comparative analysis.

### 6.8 Results

### 6.8.1 Tear secretion test

**Table 11. Tear secretion test**

| Group | Wetting length of phenol red cotton threads (mm) | | | |
|---|---|---|---|---|
| | D0 | D3 | D7 | D15 |
| Blank control | 43.21 ± 4.41 | 40.28 ± 2.35 | 39.58 ± 5.37 | 40.05 ± 5.11 |
| Model | 26.35 ± 6.47^{##} | 23.29 ± 3.57^{##} | 20.03 ± 3.68^{##} | 22.52 ± 3.34^{##} |
| Fluorometholone | 26.35 ± 5.52 | 29.45 ± 5.48' | 30.41 ± 5.29" | 32.26 ± 3.69** |
| Compound 20 | 27.76 ± 6.04 | 23.39 ± 3.93 | 29.78 ± 2.74** | 35.28 ± 3.21** |
| Compound 23 | 28.24 ± 6.22 | 29.4 ± 4.63* | 31.88 ± 6.20** | 36.05 ± 5.74" |

| | | | | |
|---|---|---|---|---|
| Note: Compared with the blank control group, *^{##}P* < 0.01, *^{###}P* < 0.001; compared with the model group, **P <* 0.05, ***P <* 0.01, ****P* < 0.001. | | | | |

After dropping benzalkonium chloride into the eyes of New Zealand rabbits, the tear secretion in the model group was significantly decreased compared with that in the blank control group, which demonstrated the successful establishment of the dry eye syndrome model in New Zealand rabbits.

Compared with the model group, administration of the positive drug fluorometholone, compounds 20 and 23 to the eyes of New Zealand rabbits significantly increased the tear secretion on day 15.

Under the same experimental conditions, compounds 16, 17, 21, 22, 26, 34, 35, 36, 37, 46, 47, 48, 51, and others of the present disclosure exhibited pharmacological effects similar to those of compound 23. All of them significantly increased the tear secretion.

### 6.8.2 Tear film break-up time test

**Table 12. Tear film break-up time test**

| Group | Tear film break-up time (s) | | |
|---|---|---|---|
| | D3 | D7 | D15 |
| Blank control | 60 ± 0.00 | 51.67 ± 7.31 | 60 ± 0.00 |
| Model | 33.33 ± 5.54^{##} | 28.67 ± 6.77^{##} | 29.5 ± 7.18^{##} |
| Fluorometholone | 43.17 ± 14.47 | 47.33 ± 16.37** | 51.33 ± 13.44" |
| Compound 20 | 37.00 ± 13.36 | 48.00 ± 9.94** | 48.28 ± 8.21** |
| Compound 23 | 45.67 ± 13.69 | 49.33 ± 8.91" | 50.67 ± 10.41" |

| | | | |
|---|---|---|---|
| Note: Compared with the blank control group, *^{##}P* < 0.01, *^{###}P* < 0.001; compared with the model group, **P <* 0.05, ***P <* 0.01, ****P* < 0.001. | | | |

After dropping benzalkonium chloride into the eyes of New Zealand rabbits, the tear film break-up time in the model group was significantly decreased compared with that in the blank control group, which demonstrated the successful establishment of the dry eye syndrome model in New Zealand rabbits.

Compared with the model group, administration of the positive drug fluorometholone, compounds 20 and 23 to the eyes of New Zealand rabbits significantly increased the tear film break-up time on day 15.

Under the same experimental conditions, compounds 16, 17, 21, 22, 26, 34, 35, 36, 37, 46, 47, 48, 51, and others of the present disclosure exhibited pharmacological effects similar to those of compound 23. All of them significantly increased the tear film break-up time.

### 6.8.3 Corneal fluorescein sodium staining score

**Table 13. Corneal fluorescein sodium staining score**

| Group | Corneal fluorescein sodium staining score | | |
|---|---|---|---|
| | D3 | D7 | D15 |
| Blank control | 0.5 ± 0.84 | 0.33 ± 0.52 | 0.5 ± 0.55 |
| Model | 2.67 ± 0.52^{##} | 2.33 ± 1.03^{##} | 2.5 ± 0.84^{##} |
| Fluorometholone | 1.67 ± 0.82 | 1.17 ± 0.41* | 1.0 ± 0** |
| Compound 20 | 2.33 ± 0.82 | 1.5 ± 0.55 | 1.1 ± 0.52^{##} |
| Compound 23 | 2.17 ± 0.98 | 1.0 ± 0.98* | 1.0 ± 0.63" |

| | | | |
|---|---|---|---|
| Note: Compared with the blank control group, *^{##}P* < 0.01, *^{###}P* < 0.001; compared with the model group, **P <* 0.05, ***P <* 0.01, ****P* < 0.001. | | | |

After dropping benzalkonium chloride into the eyes of New Zealand rabbits, the corneal fluorescein sodium staining score in the model group was significantly increased compared with that in the blank control group, which demonstrated the successful establishment of the dry eye syndrome model in New Zealand rabbits.

Compared with the model group, administration of the positive drug fluorometholone, compounds 20 and 23 to the eyes of New Zealand rabbits significantly inhibited the inflammatory response on the ocular surface on day 15, indicating that both compounds 20 and 23 significantly alleviated the ocular symptoms in dry eye syndrome model New Zealand rabbits, inhibited the inflammatory response on the ocular surface, and had significant anti-inflammatory activity.

Under the same experimental conditions, compounds 16, 17, 21, 22, 26, 34, 35, 36, 37, 46, 47, 48, 51, and others of the present disclosure exhibited pharmacological effects similar to those of compound 23. All of them significantly alleviated the ocular symptoms in dry eye syndrome model New Zealand rabbits.

### Pharmacological Example 7: Pharmacodynamic Experiment on Inflammatory Bowel Disease in Rats

### 7.1 Preparation method of test samples

Test samples: The jet-milled compounds 23 and 34 were accurately weighed and prepared as 2 mg/mL clear solutions using anhydrous ethanol, respectively. The solutions were then diluted to 0.2 mg/mL with double-distilled water as the test samples, and stored at room temperature protected from light until use.

Positive control drug: Dexamethasone was prepared as a 0.2 mg/mL suspension with 0.5% sodium carboxymethylcellulose and stored at room temperature protected from light until use.

### 7.2 Model preparation

Thirty-eight quarantine-qualified rats were randomly divided into 5 groups: Group 1 served as the blank control group, and Groups 2 to 5 were the model group, dexamethasone group, compound 23 group, and compound 34 group, respectively. Animals in Groups 2 to 5 were subjected to trinitrobenzene sulfonic acid (TNBS) modeling as follows: A 5% TNBS aqueous solution was adjusted to 20 mg/mL TNBS (50% ethanol solution) with alcohol and water. After fasting for 24 h, the rats were anesthetized using an inhalation anesthesia machine. A silicone hose was inserted approximately 8 cm into the anus, and then TNBS was injected at a dose of 50 mg/kg. After injection, the rats were placed vertically for 1 min to prevent the backflow of TNBS solution. Then, the rats were placed into their cages for observation until the animals recovered.

### 7.3 Grouping and drug administration

Drug administration started one day before modeling, with one dose administered 1 hour before modeling and then once a day for 5 consecutive days. The rats in the blank control group and model group were administered intraintestinally with 20% ethanol solution, the rats in the dexamethasone group were gavaged with 2 mg/kg of the drug, and the rats in the compound 23 group and compound 34 group were administered intraintestinally with 2 mg/kg of the corresponding drugs.

**Table 14. Animal grouping and drug administration dosages**

| Group | Drug concentration (mg/mL) | Administration dosage (mg/kg) | Number of animals |
|---|---|---|---|
| Blank control | - | - | 6 |
| Model | - | - | 8 |
| Dexamethasone | 0.2 | 2 | 8 |
| Compound 23 | 0.2 | 2 | 8 |
| Compound 34 | 0.2 | 2 | 8 |

### 7.4 Systematic (or Gross) anatomy and histopathological examination

The experiment ended on day 5 after TNBS modeling. After isoflurane anesthesia, blood was collected from the hearts, and the rats were euthanized. The abdominal cavity was rapidly opened, and the macroscopic injury scoring of the colon in rats was performed based on the adhesion, obstruction, thickening of the colonic wall, colonic congestion, and necrosis in the colon area. After clearing the colonic contents, the colonic samples were longitudinally incised. The proximal 2/3 of the colon was excised, prepared into Swiss rolls, and stored in 4% neutral buffered formalin for histopathological analysis. Tissues were trimmed, dehydrated, paraffin-embedded, and sectioned (approximately 5 µm thickness), stained with hematoxylin-eosin (HE), and observed for pathological changes. Pathologists performed blind scoring of colonic lesions.

### 7.5 Data statistics

Quantitative indices were first subjected to Bartlett's test for homogeneity of variance. When the variance was homogeneous (*P >* 0.05), one-way analysis of variance (ANOVA) was used for a statistical test; if there was statistical significance (*P* ≤ 0.05), Dunnett's *t*-test (Dunnett method) was used to compare intergroup differences; if there was no statistical significance (*P* > 0.05), the statistical analysis was concluded. When the variance was heterogeneous (*P* ≤ 0.05), Kruskal-Wallis H rank-sum test (K-W method) was used for statistical analysis: if there was statistical significance (*P* ≤ 0.05), Mann-Whitney U test (M-W method) was further performed to compare intergroup differences; if there was no statistical significance (P > 0.05), the statistical analysis was concluded.

### 7.6 Results

### 7.6.1 Macroscopic injury score of the colon in rats

**Table 15. Macroscopic injury score of the colon in rats**

| Group | Macroscopic injury score |
|---|---|
| Blank control | 0.00 ± 0.00 |
| Model | 9.88 ± 1.01^{###} |
| Dexamethasone | 5.00 ± 0.66** |
| Compound 23 | 4.83 ± 1.10" |
| Compound 34 | 5.00 ± 0.95** |

| | |
|---|---|
| Note: Compared with the blank control group, ^{##}P < 0.01, *^{###}P* < 0.001; compared with the model group, **P <* 0.05, ***P <* 0.01, ****P* < 0.001. | |

After rectal administration of TNBS to rats, the model group showed significant abnormalities in the colon surface compared with the blank group, which demonstrated the successful establishment of the inflammatory bowel disease model in rats.

Compared with the model group, administration of the positive drug dexamethasone, compounds 23 and 34 to the colon of rats significantly inhibited the inflammatory response on the intestinal surface, indicating that both compounds 23 and 34 significantly alleviated the intestinal symptoms in inflammatory bowel disease model rats, inhibited the inflammatory response on the intestinal surface, and had significant anti-inflammatory activity.

Under the same experimental conditions, compounds 16, 17, 20, 21, 22, 26, 35, 36, 37, 46, 47, 48, 51, and others of the present disclosure exhibited pharmacological effects similar to those of compound 23. All of them significantly alleviated the intestinal symptoms in inflammatory bowel disease model rats.

### Pharmacological Example 8: Pharmacodynamic Experiment on Psoriasis in Mice

### 8.1 Preparation method of test samples

Test samples: Compounds 8, 17, and 23 were prepared as 0.1% creams with reference to the formulation of Fluticasone Propionate Cream, and stored at room temperature protected from light until use.

Positive control drug: Fluticasone Propionate Cream (specification: 0.05% × 15 g, Zhejiang Xianju Pharmaceutical Co. Ltd., Lot No. 211106), stored at room temperature protected from light until use.

### 8.2 Model preparation

Adult mice were selected, and the skin of 2 cm × 3 cm on the back of mice was locally depilated. The mice in the model group and drug administration group were applied with 62.5 mg of 5% imiquimod cream on their back for modeling, while the mice in the blank control group were applied with medical petroleum jelly on their back daily. The above procedures were performed once a day for 7 consecutive days.

### 8.3 Grouping and drug administration

Forty-eight mice were randomly divided into 6 groups, including the blank control group, model group, fluticasone propionate cream group, compound 8 group, compound 17 group, and compound 23 group, with 8 animals in each group. The mice in the drug administration group were applied with the corresponding drugs on their backs daily at a dose of 0.2 g per mouse, while the blank control group and the model group were only applied with 0.2 g of the test sample drug matrix. One hour after drug administration, the drugs were wiped off with sterile cotton swabs, followed by the application of imiquimod. The above procedures were performed once a day for 7 consecutive days.

**Table 16. Animal grouping and drug administration dosages**

| Group | Drug concentration (mg/g) | Administration dosage (mg) | Number of animals |
|---|---|---|---|
| Blank control | - | 200 | 8 |
| Model | - | 200 | 8 |
| Fluticasone propionate | 0.5 | 200 | 8 |
| Compound 8 | 1 | 200 | 8 |
| Compound 17 | 1 | 200 | 8 |
| Compound 23 | 1 | 200 | 8 |

### 8.4 Index detection

### 8.4.1 Skin lesion score

Referring to the clinical Psoriasis Area and Severity Index (PASI) scoring criteria, erythema, scaling, and thickness at the skin lesion sites were evaluated on a 0-4 scale on day 8 of modeling, and the total score was obtained by summing the three items.

### PASI scoring criteria:

(1) Erythema: a score of 0 points indicates no erythema, a score of 1 point indicates erythema appearing light red, a score of 2 points indicates erythema appearing red, a score of 3 points indicates erythema appearing dark red, and a score of 4 points indicates erythema appearing extremely deep red;
(2) Scaling: a score of 0 points indicates no scaling on the surface, a score of 1 point indicates partial skin lesions covered with scaling, a score of 2 points indicates most skin lesions covered with scaling, a score of 3 points indicates almost all skin lesions covered with scaling in layers, and a score of 4 points indicates all skin lesions covered with scaling;
(3) Thickness: a score of 0 points indicates skin lesions flush with normal skin, a score of 1 point indicates skin lesions slightly elevated above normal skin surface, a score of 2 points indicates moderate elevation of skin lesions, a score of 3 points indicates thickened skin lesions with obvious elevation, and a score of 4 points indicates highly thickened skin lesions with prominent elevation.

### 8.4.2 Determination of the level of inflammatory cytokines in serum

After the PASI scoring on day 8 of modeling, blood was collected from the orbital sinus of mice and centrifuged at 3000 rpm for 10 min. The supernatant was extracted to measure the level of IL-17. All procedures were performed following the instructions of the ELISA kit.

### 8.5 Data statistics

Statistical analysis was performed using SPSS software, and the results were expressed as mean ± standard deviation. One-way ANOVA was used for intergroup comparisons. For data with homogeneous variance, the LSD test was used; for data with heterogeneous variance, the Dunnett-t test was used. A value of *P* < 0.05 was considered statistically significant.

### 8.6 Results

### 8.6.1 Effects on PASI score in mice

**Table 17. PASI score of mice**

| Group | PASI score |
|---|---|
| Blank control | 0.0 ± 0.0 |
| Model | 7.9 ± 0.6^{###} |
| Fluticasone propionate | 4.0 ± 0.6*** |
| Compound 8 | 4.8 ± 1.3*** |
| Compound 17 | 4.5 ± 0.6*** |
| Compound 23 | 3.8 ± 1.0*** |

| | |
|---|---|
| Note: Compared with the blank control group, ###P < 0.001; compared with the model group, ****P* < 0.001. | |

After treatment with imiquimod on the backs of mice, the model group showed significant epidermal thickening compared with the blank control group, scaling and erythema developed, and the PASI score significantly increased, demonstrating the successful establishment of the psoriasis model in mice.

Compared with the model group, the PASI scores significantly decreased on day 7 after the administration of the positive drug fluticasone propionate, compounds 8, 17, and 23 on the backs of mice.

Under the same experimental conditions, compounds 16, 20, 21, 22, 26, 34, 35, 36, 37, 46, 47, 48, 51 and others of the present disclosure exhibited pharmacological effects similar to those of compound 23, with significantly decreased PASI scores on day 7.

### 8.6.2 Effects on inflammatory cytokines in serum

**Table 18. Effects on the level of serum IL-17 in mice**

| Group | IL-17 (pg/mL) |
|---|---|
| Blank | 19.54 ± 14.29 |
| Model | 253.50 ± 174.92^{##} |
| Fluticasone propionate | 25.04 ± 10.02** |
| Compound 8 | 53.35 ± 64.19* |
| Compound 17 | 31.30 ± 14.99** |
| Compound 23 | 19.56 ± 8.13** |

| | |
|---|---|
| Note: Compared with the blank control group, ##*P* < 0.01; compared with the model group, **P <* 0.05, ***P* < 0.01. | |

After mice were treated with imiquimod for 7 days, the level of IL-17 in serum significantly increased, demonstrating the successful establishment of the psoriasis model in mice.

Compared with the model group, administration of the positive drug fluticasone propionate, compounds 8, 17, and 23 to the skin of mice significantly decreased the level of IL-17, indicating that compounds 8, 17, and 23 significantly inhibited inflammatory cytokines in the skin of psoriasis model mice, and had significant anti-inflammatory activity.

Under the same experimental conditions, compounds 16, 20, 21, 22, 26, 34, 35, 36, 37, 46, 47, 48, 51 and others of the present disclosure exhibited pharmacological effects similar to those of compound 23. All of them significantly inhibited inflammatory cytokines in the skin of psoriasis model mice.

### Pharmacological Example 9: Experiment on Adverse Reactions of Continuous Ocular Administration in Guinea Pigs

### 9.1 Experimental method

Test samples: Compounds 17 and 23 were prepared as 5 mg/5 mL ophthalmic suspensions with reference to the formulation of Fluorometholone Eye Drops, and stored at room temperature protected from light until use.

Positive control drug: Fluorometholone Eye Drops (specification: 5 mL/5 mg; Santen Pharmaceutical (CHINA) Co., Ltd.; Lot No.: 1FM6545), Loteprednol Etabonate Eye Drops (specification 0.5% (5 mg/mL); Shandong Bausch & Lomb Freda Pharmaceutical Co., Ltd; Lot No. 386521), stored at room temperature protected from light until use.

Animals: 50 guinea pigs, conventional grade, with an equal number of males and females, weighing 300-350 g..

Animal grouping: After 5 days of adaptive feeding, the guinea pigs were randomly divided into 5 groups based on body weight. Group 1 was the blank control group, and 50 µL of normal saline was dropped into the eyes (bilateral) of the guinea pigs. Groups 2 to 5 were the fluorometholone group, loteprednol group, compound 17 group, and compound 23 group, respectively, and the corresponding drugs were dropped into the eyes (bilateral) of the guinea pigs.

**Table 19. Animal grouping and drug administration dosages for the experiment on adverse reactions of continuous ocular administration in guinea pigs**

| Group | Drug concentration (mg/mL) | Administration volume (µL) / eye | Number of animals |
|---|---|---|---|
| Blank control | - | 50 | 10 |
| Fluorometholone | 1 | 50 | 10 |
| Loteprednol | 5 | 50 | 10 |
| Compound 17 | 1 | 50 | 10 |
| Compound 23 | 1 | 50 | 10 |

Intraocular pressure measurement time: The intraocular pressure of animals in each group was measured before administration and on days 7, 14, 21, and 28 post-administration.

### 9.2 Results

**Table 20. Changes in intraocular pressure of guinea pigs**

| Group | Intraocular pressure of guinea pigs (mmHg) | | | | |
|---|---|---|---|---|---|
| | D0 | D7 | D14 | D21 | D28 |
| Blank control | 14.3 ± 2.6 | 15.3 ± 2.2 | 14.7 ± 1.5 | 14.3 ± 0.6 | 14.5 ± 1.2 |
| Fluorometholone | 14.3 ± 1.5 | 20.5 ± 3.3*** | 21.2 ± 2.1*** | 23.0 ± 3.1*** | 22.5 ± 2.1*** |
| Loteprednol | 14.0 ± 1.8 | 19.0 ± 3.5** | 18.5 ± 1.5*** | 18.5 ± 0.7*** | 19.5 ± 0.6*** |
| Compound 17 | 13.5 ± 0.6 | 16.0 ± 1.4 | 14.5 ± 1.9 | 13.3 ± 2.5 | 14.3 ± 1.5 |
| Compound 23 | 13.5 ± 0.6 | 15.3 ± 1.7 | 15.3 ± 1.5 | 15.3 ± 2.2 | 14.9 ± 1.6 |

| | | | | | |
|---|---|---|---|---|---|
| Note: Compared with the blank control group, ***P* < 0.01, ****P* < 0.001. | | | | | |

The results showed that compared with baseline intraocular pressure before administration, intraocular pressure elevation was detected on day 7 after ocular administration of the positive drugs fluorometholone and loteprednol in guinea pigs, while no abnormal intraocular pressure was observed after administration of compounds 17 and 23. This indicated that the safety profile of continuous ocular administration of compounds 17 and 23 was significantly superior to that of the positive drugs fluorometholone and loteprednol.

Under the same experimental conditions, compounds 16, 20, 21, 22, 26, 34, 35, 36, 37, 46, 47, 48, 51, and others of the present disclosure exhibited safety similar to that of compounds 17 and 23, with no abnormal intraocular pressure observed.

### Pharmacological Example 10: Experiment on Adverse Reactions of Continuous Dermal Administration in Rats

### 10.1 Method

Test samples: Compounds 17 and 23 were prepared as 0.1% creams with reference to the formulation of Compound Dexamethasone Acetate Cream, and stored at room temperature protected from light until use.

Positive control drug: Compound Dexamethasone Acetate Cream (specification: 0.75 mg/g; China Resources Sanjiu Medical & Pharmaceutical Co., Ltd.; Lot No. 2112002X), stored at room temperature protected from light until use.

Animals: SD rats, SPF grade, with an equal number of males and females, weighing 230-250 g.

Animal grouping: After adaptive feeding, the rats were randomly divided into 4 groups. Group 1 was the blank control group, and Groups 2 to 4 were the Compound Dexamethasone Acetate Cream group, compound 17 group, and compound 23 group, respectively. One day before administration, the backs of rats were shaved at an area of 5 cm × 6 cm. The rats in the Compound Dexamethasone Acetate Cream group, compound 17 group, and compound 23 group were applied with the corresponding drugs on their backs, respectively, 100 mg each time, twice a day. The rats in the blank control group were applied with the blank matrix of corresponding mass on their backs, twice a day for a consecutive week.

**Table 21. Animal grouping and drug administration dosages for the experiment on adverse reactions of continuous dermal administration in rats**

| Group | Drug concentration (mg/g) | Administration dosage (mg) | Number of animals |
|---|---|---|---|
| Blank control | Blank Matrix | 100 | 10 |
| Compound Dexamethasone Acetate | 0.75 | 100 | 10 |
| Compound 17 | 1 | 100 | 10 |
| Compound 23 | 1 | 100 | 10 |

### 10.2 Measurement of the thickness of the epidermal layer in rats

To examine the effects of continuous administration of the test samples on the epidermal layer, the back skin tissues were stained with hematoxylin and eosin (H&E) (magnification ×40), and the thickness of the epidermal layer was quantitatively measured based on the H&E-stained sections.

### 10.3 Results

**Table 22. The thickness of the epidermal layer in rats**

| Group | Epidermal layer thickness (µm) |
|---|---|
| Blank control | 28.7 ± 2.2 |
| Compound Dexamethasone Acetate | 10.0 ± 4.8*** |
| Compound 17 | 23.5 ± 3.5 |
| Compound 23 | 24.3 ± 1.1 |

| | |
|---|---|
| Note: Compared with the blank control group, ****P* < 0.001. | |

The results showed that compared with the blank control group, the thickness of the epidermal layer in rat skin significantly thinned after administration of the positive drug Compound Dexamethasone Acetate, while no significant changes in the thickness of the epidermal layer were observed after administration of compounds 17 and 23. This indicated that the safety profile of continuous dermal administration of compounds 17 and 23 was superior to that of the positive drug Compound Dexamethasone Acetate.

Under the same experimental conditions, compounds 16, 20, 21, 22, 26, 34, 35, 36, 37, 46, 47, 48, 51, and others of the present disclosure exhibited safety similar to that of compounds 17 and 23, with no significant changes in the thickness of the epidermal layer observed.

### Pharmacological Example 11. Pharmacokinetic (PK) Experiment in Rats

### 11.1 Method

Test sample: Compound 8 was accurately weighed and dissolved in 10% DMSO + 25% PEG400 + 65% normal saline to prepare a 1 mg/mL solution as the test sample. The solution was prepared before administration.

Animals: 6 male SD rats, SPF grade (Beijing Vital River Laboratory Animal Technology Co., Ltd.).

Animal grouping: The animals were weighed prior to administration, and the dosage was calculated based on body weight. The animals in the oral administration group were fasted overnight (10-14 hours) before administration, and food was reintroduced 4 hours post-administration.

**Table 23. Animal grouping and drug administration dosages for pharmacokinetic (PK) experiment in rats**

| Group | Grouping | Number of animals | Dosage | Concentration | Administration volume | Administration route |
|---|---|---|---|---|---|---|
| | | | (mg/kg) | (mg/mL) | (mL/kg) | |
| 1 | Compound 8 | 3 | 2 | 1 | 2 | Intravenous, single |
| 2 | Compound 8 | 3 | 10 | 1 | 10 | Gavage, single |

Blood collection time points: Blood was collected from the jugular vein at 2 min, 5 min, 15 min, 30 min, 1 h, 2 h, 4 h, 8 h, and 24 h after administration. Approximately 0.25 mL of blood was collected for each sample, anticoagulated with heparin sodium, and then placed on ice immediately.

Plasma sample processing: Blood samples were placed on ice after collection and centrifuged to separate plasma within 1 hour (centrifugation conditions: 6000 rpm, 3 minutes, 2-8 °C ). Plasma samples were stored in a -80°C freezer before analysis.

Data processing: Pharmacokinetic parameters were calculated using Phoenix WinNonlin 8.2.0 based on the plasma drug concentration data at different time points.

### 11.2 Results

**Table 24. Pharmacokinetic parameters in rats**

| PK parameters | Cl_obs | T_{1/2} | Cₘₐₓ | AUCₗₐₛₜ | F |
|---|---|---|---|---|---|
| | mL/min/kg | h | ng/mL | h • ng/mL | % |
| Compound 8 (IV, 2 mg/kg) | 474 ± 109 | 0.45 ± 0.20 | 924 ± 504 | 105.7 ± 35.9 | / |
| Compound 8 (PO, 10 mg/kg) | / | 5.21 ± 3.17 | 23.5 ± 14.4 | 56.3 ± 36.7 | 10.6 ± 7 |

Pharmacokinetic parameters showed that after intravenous administration to rats, compound 8 provided by the present disclosure exhibited a high plasma clearance rate and a short half-life, with Cl_obs greater than 400 mL/min/kg and T1/2 less than 0.5 h; after intragastric administration to rats, it showed reduced drug exposure and low bioavailability, with AUC less than 70 h • ng/mL and bioavailability F of approximately 10%. The results indicated that the compound provided by the present disclosure had a high plasma clearance rate and reduced systemic exposure, thereby effectively reducing the systemic adverse reactions associated with high exposure to glucocorticoid drugs.

Under the same experimental conditions, compounds 16, 17, 20, 21, 22, 23, 26, 34, 35, 36, 37, 46, 47, 48, 51, and others of the present disclosure exhibited pharmacokinetic properties similar to those of compound 8, including a high plasma clearance rate and reduced systemic exposure, thereby effectively reducing the systemic adverse reactions associated with high exposure to glucocorticoid drugs.

Finally, it should be noted that the above examples are merely intended to describe the technical solutions of the present disclosure, rather than to limit the present disclosure. Although the present disclosure has been described in detail with reference to the above examples, those of ordinary skill in the art should understand that they can still make modifications to the technical solutions described in the above examples or make equivalent replacements to some or all of the technical features, without deviating from the essence of the technical solutions in the examples of the present disclosure.

## Claims

1. A compound represented by formula I, having the following structural formula: 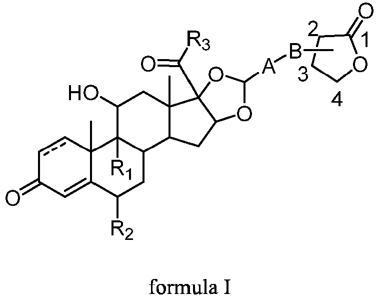
an optical isomer thereof, a pharmaceutically acceptable salt thereof, or a solvate thereof,
wherein A, B, R₁, R₂, and R₃ as shown in the structural formula of the compound represented by formula I are selected independently of each other, and:
A is selected from substituted or unsubstituted aryl having 6 to 10 carbon atoms, substituted or unsubstituted heteroaryl or heterocyclyl having 4 to 10 carbon atoms, and substituted or unsubstituted cycloalkyl having 3 to 10 carbon atoms, wherein the substituents are selected from one or more, or one, of the following: halogen, hydroxyl, carbonyl, amino, cyano, carboxyl, aryl having 6 to 10 carbon atoms, heteroaryl having 4 to 10 carbon atoms, cycloalkyl having 3 to 6 carbon atoms, alkoxy having 1 to 10 carbon atoms, alkylamino having 1 to 10 carbon atoms, or alkyl ester having 1 to 10 carbon atoms;
preferably, A is selected from substituted or unsubstituted phenyl, substituted or unsubstituted piperidinyl, substituted or unsubstituted cyclohexyl, substituted or unsubstituted pyridinyl, and substituted or unsubstituted pyrimidinyl, wherein the substituents are selected from one or more, or one, of the following: halogen or cyano;
more preferably, A is selected from substituted or unsubstituted phenyl, substituted or unsubstituted cyclohexyl, substituted or unsubstituted pyridyl, and substituted or unsubstituted pyrimidinyl, wherein the substituents are selected from one or more, or one, of the following: halogen or cyano;
further preferably, A is selected from substituted or unsubstituted phenyl, substituted or unsubstituted cyclohexyl, and substituted or unsubstituted pyridyl, wherein the substituents are selected from one or more, or one, of the following: halogen;
even more preferably, A is selected from substituted or unsubstituted phenyl and substituted or unsubstituted pyridyl, wherein the substituents are selected from one or more, or one, of the following: halogen;
B is selected from absent, O, S, NH, CH₂, OCH₂, SCH₂, NHCH₂, CH₂O, CH₂S, CH₂SO, CH₂SO₂, CH₂NH, C=O, or NH(CO);
preferably, B is selected from O, S, NH, CH₂, OCH₂, SCH₂, NHCH₂, CH₂O, CH₂S, CH₂SO, CH₂SO₂, CH₂NH, C=O, or NH(CO);
more preferably, B is selected from S, CH₂, OCH₂, SCH₂, NHCH₂, CH₂O, CH₂S, CH₂SO, CH₂SO₂, CH₂NH, or NH(CO);
further preferably, B is selected from S, CH₂O, CH₂S, CH₂SO, or CH₂NH;
even more preferably, B is selected from CH₂O, CH₂S, or CH₂NH;
B is attached to the 2-, 3-, or 4-position of the ring 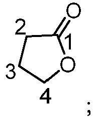
preferably, B is attached to the 2- or 3-position of the ring 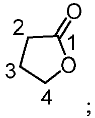
R₁ is selected from H or halogen;
preferably, R₁ is selected from halogen;
R₂ is selected from H, CH₃, or halogen;
preferably, R₂ is selected from H or halogen;
R₃ is selected from CH₂R₄ where R₄ is selected from OH, halogen, OR₅, or OCOR₅, or R₃ is selected from SCH₂R₆ or OCH₂R₆;
wherein R₅ is selected from alkyl having 1 to 6 carbon atoms, alkenyl having 2 to 6 carbon atoms, alkynyl having 2 to 6 carbon atoms, aryl having 6 to 10 carbon atoms, heteroaryl or heterocyclyl having 4 to 10 carbon atoms; R₆ is selected from halogen or CN;
preferably, R₃ is selected from CH₂R₄, wherein R₄ is selected from OH or halogen, or R₃ is selected from SCH₂R₆ or OCH₂R₆, wherein R₆ is selected from halogen or CN;
more preferably, R₃ is selected from CH₂R₄, wherein R₄ is selected from OH, or R₃ is selected from SCH₂R₆ or OCH₂R₆, wherein R₆ is selected from halogen or CN;
preferably, the halogen is selected from F or Cl; 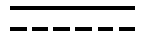 is a single bond or a double bond, preferably a double bond.

2. The compound according to claim 1, wherein the compound represented by formula I has the following structural formula I': 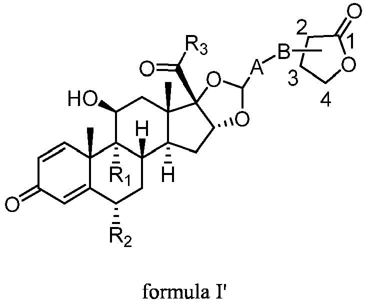 wherein the variables A, B, R₁, R₂, R₃, and the position of attachment of B to the ring 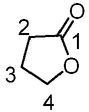 are as defined in claim 1.

3. The compound according to claims 1 or 2, wherein the compound represented by formula I is selected from the following structural formulas I-1 or I-2, and the compound represented by formula I' is selected from the following structural formulas I'-1 or I'-2: 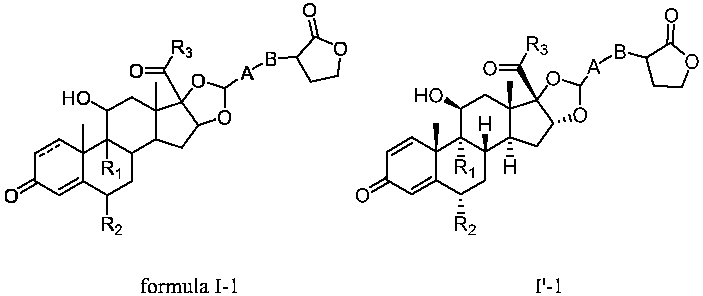 wherein A, B, R₁, R₂, and R₃ as shown in the structural formulas of the compounds represented by formulas I-1 and I'-1 are selected independently of each other, and:
A is selected from substituted or unsubstituted cyclohexyl, substituted or unsubstituted piperidinyl, substituted or unsubstituted phenyl, substituted or unsubstituted pyridinyl, and substituted or unsubstituted pyrimidinyl, wherein the substituents are selected from one or more, or one, of the following: halogen, preferably F or Cl;
preferably, A is selected from: 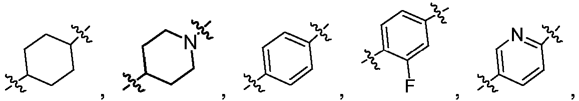 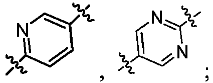
B is selected from O, S, NH, CH₂, OCH₂, SCH₂, NHCH₂, CH₂O, CH₂S, CH₂SO, CH₂SO₂, CH₂NH;
R₁ is selected from H or halogen; preferably, R₁ is selected from H, F, or Cl;
R₂ is selected from H or halogen; preferably, R₂ is selected from H or F;
R₃ is selected from the following: CH₂R₄, wherein R₄ is selected from OH or halogen; SCH₂R₆ or OCH₂R₆, wherein R₆ is selected from halogen or CN;
preferably, R₃ is selected from the following: CH₂R₄, wherein R₄ is selected from OH or Cl; SCH₂R₆ or OCH₂R₆, wherein R₆ is selected from F or CN; 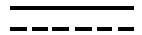 is a single bond or a double bond, preferably a double bond; 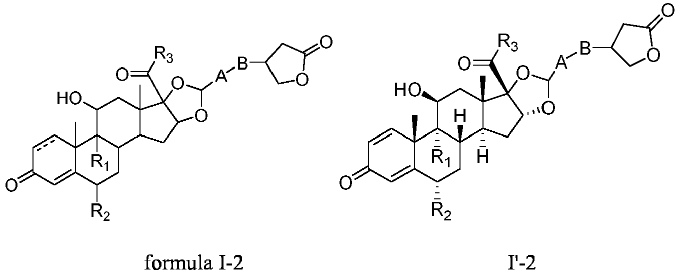 wherein A, B, R₁, R₂, and R₃ as shown in the structural formulas of the compounds represented by formulas I-2 and I'-2 are selected independently of each other, and:
A is selected from substituted or unsubstituted cyclohexyl, substituted or unsubstituted piperidinyl, and substituted or unsubstituted phenyl, wherein the substituents are selected from one or more, or one, of the following: halogen, preferably F or Cl, or cyano;
preferably, A is selected from: 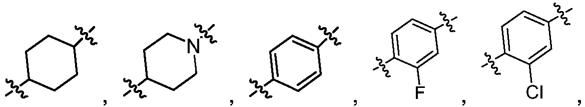 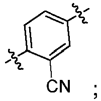
B is selected from C=O, NH(CO), CH₂O, CH₂S, CH₂NH;
R₁ is selected from H or halogen; preferably, R₁ is selected from H, F, or Cl;
R₂ is selected from H or halogen; preferably, R₂ is selected from H or F;
R₃ is selected from the following: CH₂R₄, wherein R₄ is selected from OH or halogen; SCH₂R₆, wherein R₆ is selected from halogen;
preferably, R₃ is selected from the following: CH₂R₄, wherein R₄ is selected from OH or Cl; SCH₂R₆, wherein R₆ is selected from F; 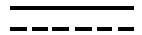 is a single bond or a double bond, preferably a double bond.

4. The compound according to claims 1 or 2, wherein
A, B, R₁, R₂, and R₃ are selected independently of each other, and:
A is selected from substituted or unsubstituted cyclohexyl, substituted or unsubstituted phenyl, substituted or unsubstituted pyridyl, and substituted or unsubstituted pyrimidinyl, wherein the substituents are one or more, or one, of the following: halogen, preferably F or Cl, or cyano;
preferably, A is selected from: 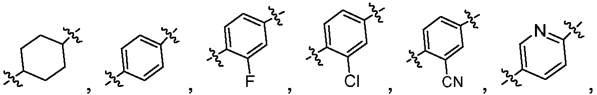 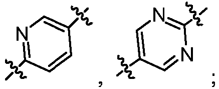
B is selected from S, CH₂, OCH₂, SCH₂, NHCH₂, CH₂O, CH₂S, CH₂SO, CH₂SO₂, CH₂NH, NH(CO);
R₁ is selected from H or halogen; preferably, R₁ is selected from H, F, or Cl;
R₂ is selected from H or halogen; preferably, R₂ is selected from H or F;
R₃ is selected from the following: CH₂R₄, wherein R₄ is selected from OH or halogen; SCH₂R₆ or OCH₂R₆, wherein R₆ is selected from halogen or CN;
preferably, R₃ is selected from the following: CH₂R₄, wherein R₄ is selected from OH or Cl; SCH₂R₆ or OCH₂R₆, wherein R₆ is selected from F or CN; 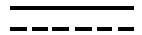 is a single bond or a double bond, preferably a double bond.

5. The compound according to any one of claims 1, 2, and 4, wherein
the compound represented by formula I is selected from the following structural formulas 1-1 or I-2, and the compound represented by formula I' is selected from the following structural formulas I'-1 or I'-2: 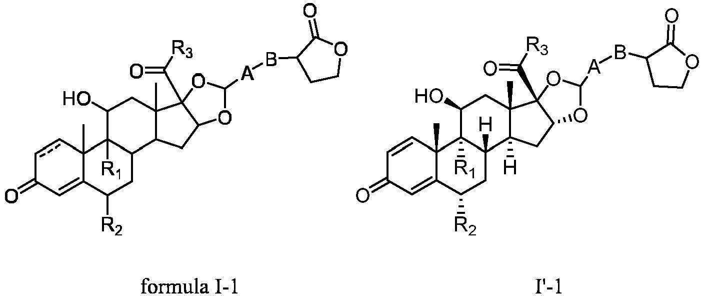 wherein A, B, R₁, R₂, and R₃ as shown in the structural formulas of the compounds represented by formulas I-1 and I'-1 are selected independently of each other, and:
A is selected from substituted or unsubstituted cyclohexyl, substituted or unsubstituted phenyl, substituted or unsubstituted pyridyl, and substituted or unsubstituted pyrimidinyl, wherein the substituents are selected from one or more, or one, of the following: halogen, preferably F or Cl;
preferably, A is selected from: 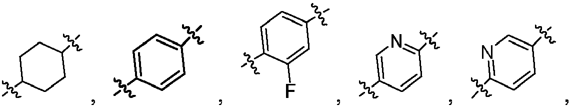 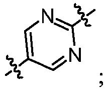
B is selected from S, CH₂, OCH₂, SCH₂, NHCH₂, CH₂O, CH₂S, CH₂SO, CH₂SO₂, CH₂NH;
R₁ is selected from H or halogen; preferably, R₁ is selected from H, F, or Cl;
R₂ is selected from H or halogen; preferably, R₂ is selected from H or F;
R₃ is selected from the following: CH₂R₄, wherein R₄ is selected from OH or halogen; SCH₂R₆ or OCH₂R₆, wherein R₆ is selected from halogen or CN;
preferably, R₃ is selected from the following: CH₂R₄, wherein R₄ is selected from OH or Cl; SCH₂R₆ or OCH₂R₆, wherein R₆ is selected from F or CN; 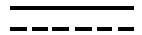 is a single bond or a double bond, preferably a double bond; 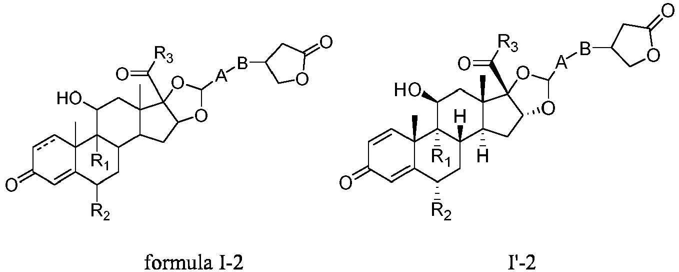 wherein A, B, R₁, R₂, and R₃ as shown in the structural formulas of the compounds represented by formulas I-2 and I'-2 are selected independently of each other, and:
A is selected from substituted or unsubstituted phenyl, wherein the substituents are selected from one or more, or one, of the following: halogen, preferably F or Cl, or cyano;
preferably, A is selected from: 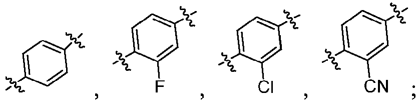
B is selected from NH(CO), CH₂O, CH₂S, CH₂NH;
R₁ is selected from H or halogen; preferably, R₁ is selected from H, F, or Cl;
R₂ is selected from H or halogen; preferably, R₂ is selected from H or F;
R₃ is selected from the following: CH₂R₄, wherein R₄ is selected from OH or halogen; SCH₂R₆, wherein R₆ is selected from halogen;
preferably, R₃ is selected from the following: CH₂R₄, wherein R₄ is selected from OH or Cl; SCH₂R₆, wherein R₆ is selected from F; 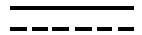 is a single bond or a double bond, preferably a double bond.

6. The compound according to claims 1 or 2, wherein
A, B, R₁, R₂, and R₃ are selected independently of each other, and:
A is selected from substituted or unsubstituted cyclohexyl, substituted or unsubstituted phenyl, and substituted or unsubstituted pyridyl, wherein the substituents are one or more, or one, of the following: halogen, preferably F or Cl;
preferably, A is selected from: 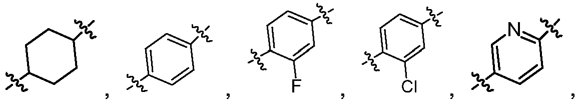 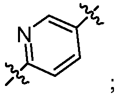
B is selected from S, CH₂O, CH₂S, CH₂SO, CH₂NH;
R₁ is selected from halogen; preferably, R₁ is selected from F or Cl;
R₂ is selected from H or halogen; preferably, R₂ is selected from H or F;
R₃ is selected from the following: CH₂R₄, wherein R₄ is selected from OH or halogen; SCH₂R₆ or OCH₂R₆, wherein R₆ is selected from halogen or CN;
preferably, R₃ is selected from the following: CH₂R₄, wherein R₄ is selected from OH or Cl; SCH₂R₆ or OCH₂R₆, wherein R₆ is selected from F or CN; 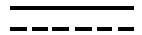 is a single bond or a double bond, preferably a double bond.

7. The compound according to any one of claims 1, 2, and 6, wherein
the compound represented by formula I is selected from the following structural formulas I-1 or I-2, and the compound represented by formula I' is selected from the following structural formulas I'-1 or I'-2: 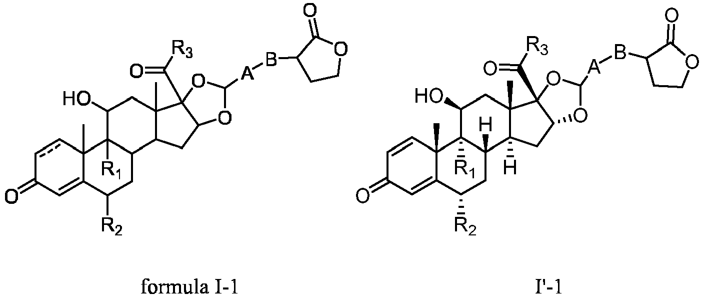 wherein A, B, R₁, R₂, and R₃ as shown in the structural formulas of the compounds represented by formulas I-1 and I'-1 are selected independently of each other, and:
A is selected from substituted or unsubstituted cyclohexyl, substituted or unsubstituted phenyl, and substituted or unsubstituted pyridyl, wherein the substituents are selected from one or more, or one, of the following: halogen, preferably F or Cl;
preferably, A is selected from: 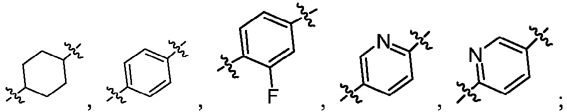
B is selected from S, CH₂O, CH₂S, CH₂SO, CH₂NH;
R₁ is selected from halogen; preferably, R₁ is selected from F or Cl;
R₂ is selected from H or halogen; preferably, R₂ is selected from H or F;
R₃ is selected from the following: CH₂R₄, wherein R₄ is selected from OH or halogen; SCH₂R₆ or OCH₂R₆, wherein R₆ is selected from halogen or CN;
preferably, R₃ is selected from the following: CH₂R₄, wherein R₄ is selected from OH or Cl; SCH₂R₆ or OCH₂R₆, wherein R₆ is selected from F or CN; 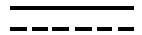 is a single bond or a double bond, preferably a double bond; 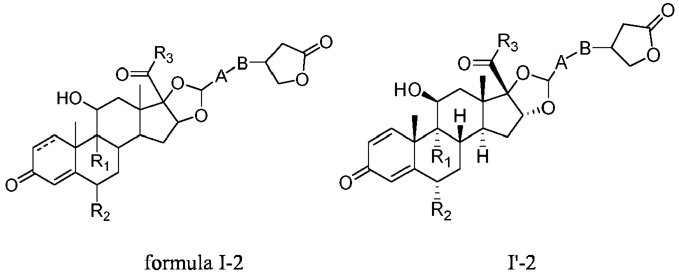 wherein A, B, R₁, R₂, and R₃ as shown in the structural formulas of the compounds represented by formulas I-2 and I'-2 are selected independently of each other, and:
A is selected from substituted or unsubstituted phenyl, where the substituents selected from are one or more, or one, of the following: halogen, preferably Cl;
preferably, A is selected from: 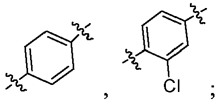
B is selected from CH₂O, CH₂S, CH₂NH;
R₁ is selected from halogen; preferably, R₁ is selected from F or Cl;
R₂ is selected from H or halogen; preferably, R₂ is selected from H or F;
R₃ is selected from the following: CH₂R₄, wherein R₄ is selected from OH; 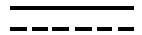 is a single bond or a double bond, preferably a double bond.

8. The compound according to claims 1 or 2, wherein
A, B, R₁, R₂, and R₃ are selected independently of each other, and:
A is selected from substituted or unsubstituted phenyl and substituted or unsubstituted pyridyl, wherein the substituents are one or more, or one, of the following: halogen, preferably F or Cl;
preferably, A is selected from: 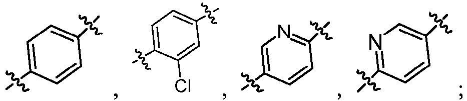
B is selected from CH₂O, CH₂S, CH₂NH;
R₁ is selected from halogen; preferably, R₁ is selected from F or Cl;
R₂ is selected from H or halogen; preferably, R₂ is selected from H or F;
R₃ is selected from the following: CH₂R₄, wherein R₄ is selected from OH; SCH₂R₆ or OCH₂R₆, wherein R₆ is selected from halogen or CN;
preferably, R₃ is selected from the following: CH₂R₄, wherein R₄ is selected from OH; SCH₂R₆ or OCH₂R₆, wherein R₆ is selected from F or CN; 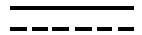 is a single bond or a double bond, preferably a double bond.

9. The compound according to any one of claims 1, 2, and 8, wherein
the compound represented by formula I is selected from the following structural formulas I-1 or I-2, and the compound represented by formula I' is selected from the following structural formulas I'-1 or I'-2: 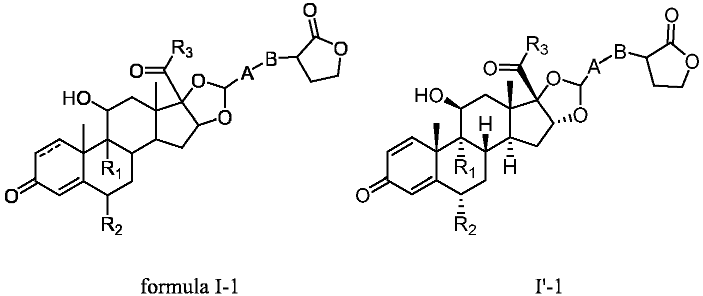 wherein A, B, R₁, R₂, and R₃ as shown in the structural formulas of the compounds represented by formulas I-1 and I'-1 are selected independently of each other, and:
A is selected from substituted or unsubstituted phenyl and substituted or unsubstituted pyridyl, wherein the substituents are one or more, or one, of the following: halogen, preferably F or Cl;
preferably, A is selected from: 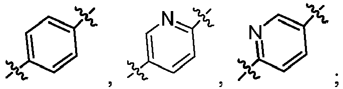
B is selected from CH₂O, CH₂S;
R₁ is selected from halogen; preferably, R₁ is selected from F or Cl;
R₂ is selected from H or halogen; preferably, R₂ is selected from H or F;
R₃ is selected from the following: CH₂R₄, wherein R₄ is selected from OH; SCH₂R₆ or OCH₂R₆, wherein R₆ is selected from halogen or CN;
preferably, R₃ is selected from the following: CH₂R₄, wherein R₄ is selected from OH; SCH₂R₆ or OCH₂R₆, wherein R₆ is selected from F or CN; 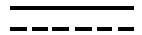 is a single bond or a double bond, preferably a double bond; 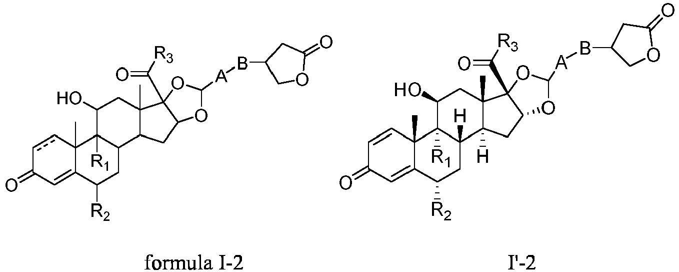 wherein A, B, R₁, R₂, and R₃ as shown in the structural formulas of the compounds represented by formulas I-1 and I'-1 are selected independently of each other, and:
A is selected from substituted or unsubstituted phenyl, wherein the substituents are selected from one or more, or one, of the following: halogen, preferably Cl;
preferably, A is selected from: 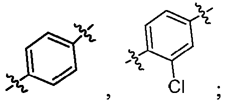
B is selected from CH₂S, CH₂NH;
R₁ is selected from halogen; preferably, R₁ is selected from F or Cl;
R₂ is selected from H;
R₃ is selected from the following: CH₂R₄, wherein R₄ is selected from OH; 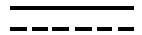 is a single bond or a double bond, preferably a double bond.

10. The compound according to any one of claims 1 to 3, wherein the compound represented by formula I is selected from the following compounds: 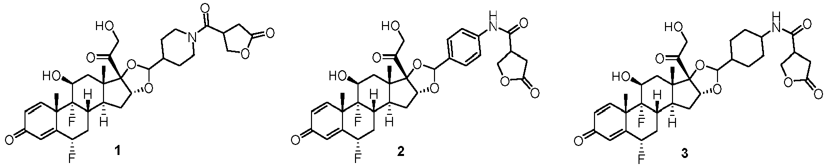 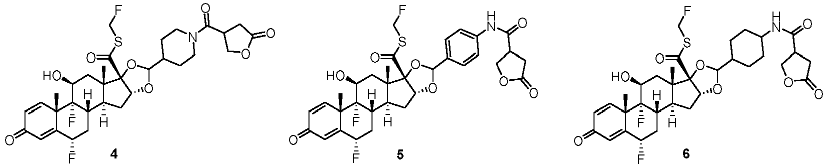 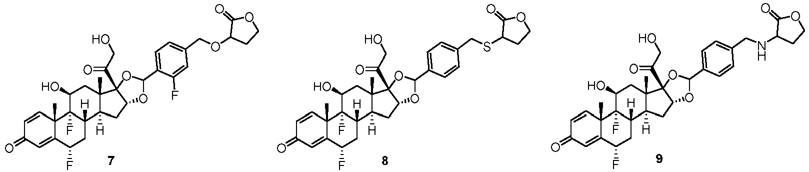 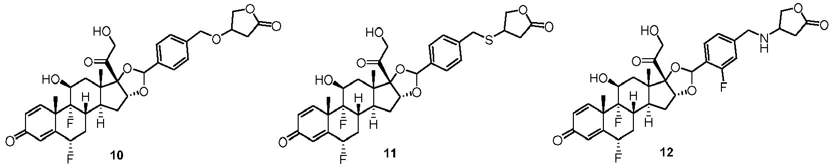 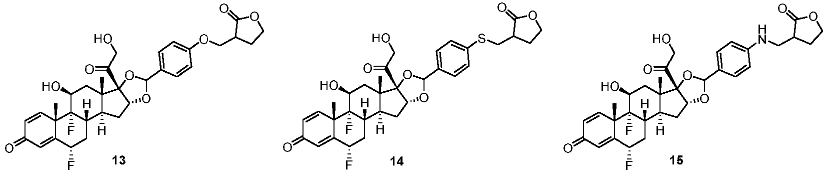 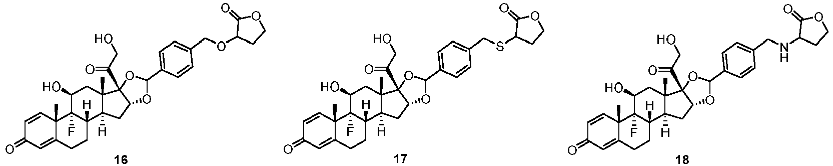 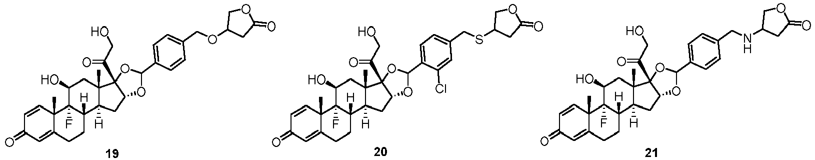 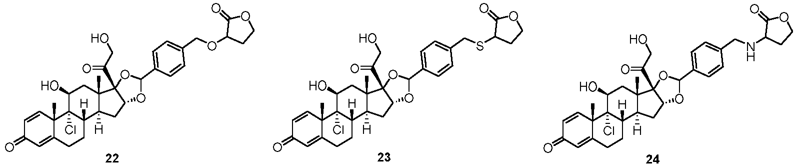 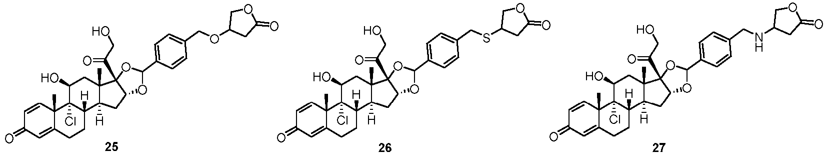 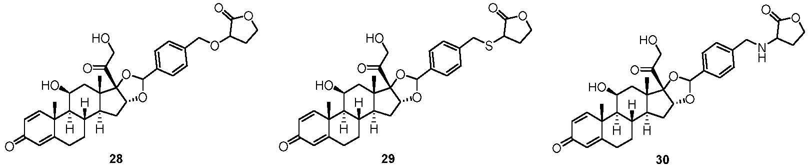 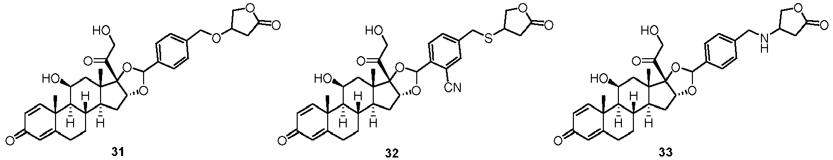 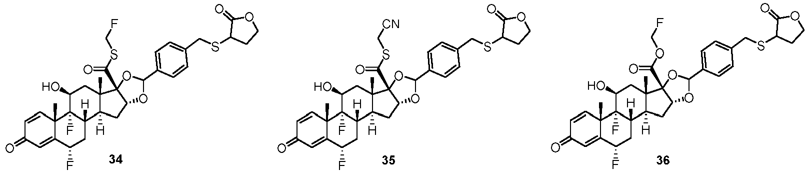 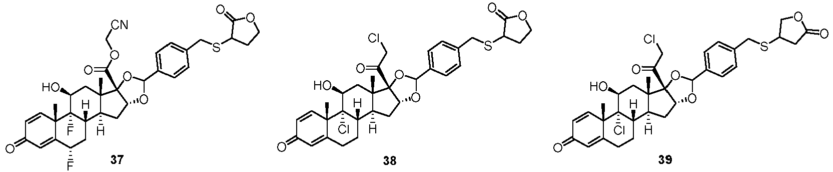 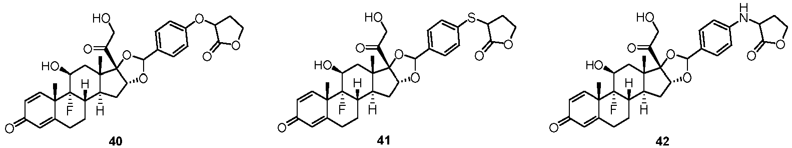 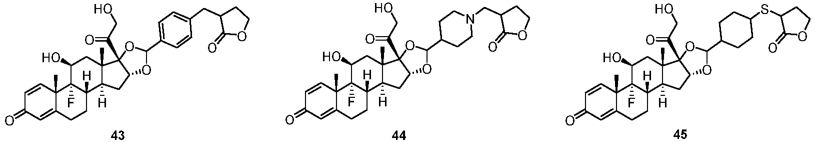 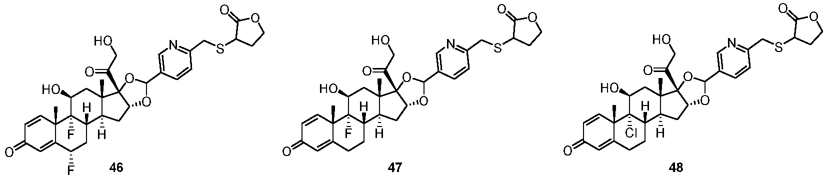 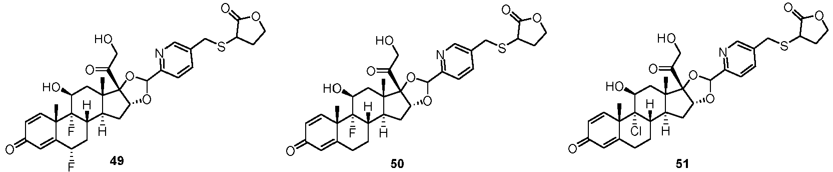 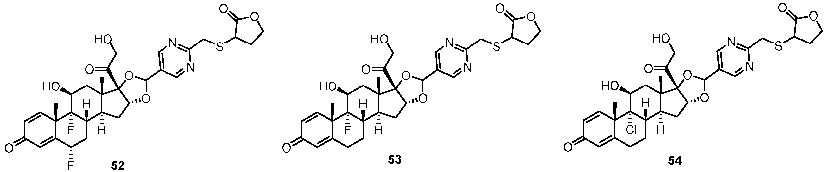 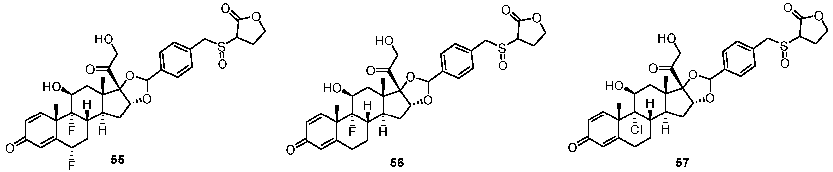 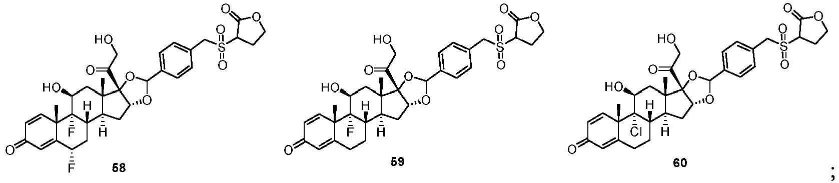
preferably compound 5, compound 7, compound 8, compound 9, compound 10, compound 11, compound 12, compound 13, compound 14, compound 15, compound 16, compound 17, compound 18, compound 19, compound 20, compound 21, compound 22, compound 23, compound 24, compound 25, compound 26, compound 27, compound 28, compound 29, compound 30, compound 32, compound 33, compound 34, compound 35, compound 36, compound 37 compound 38, compound 39, compound 41, compound 43, compound 45, compound 46, compound 47, compound 48, compound 49, compound 50, compound 51, compound 52, compound 54, compound 55, compound 56, compound 57, compound 58, compound 59, compound 60;
further preferably compound 7, compound 8, compound 10, compound 11, compound 16, compound 17, compound 18, compound 19, compound 20, compound 21, compound 22, compound 23, compound 24, compound 25, compound 26, compound 27, compound 34, compound 35, compound 36, compound 37, compound 38, compound 45, compound 46, compound 47, compound 48, compound 49, compound 50, compound 51, compound 57;
even more preferably compound 16, compound 17, compound 20, compound 21, compound 22, compound 23, compound 26, compound 34, compound 35, compound 36, compound 37, compound 46, compound 47, compound 48, compound 51.

11. A method for preparing the compound according to any one of claims 1 to 10, wherein the preparation is implemented by Method I or Method II:
Method I:
reacting a compound represented by formula II with a compound represented by formula III under acidic conditions to obtain a compound represented by formula I: 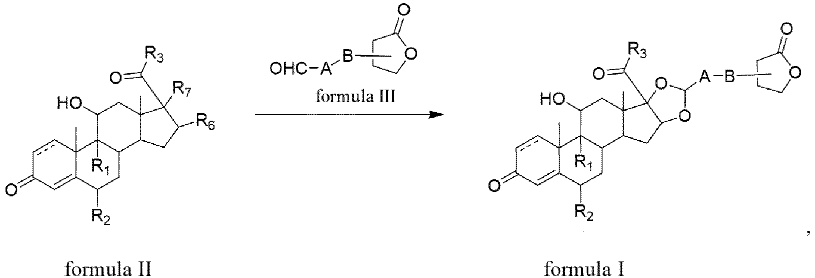
wherein A, B, R₁, R₂, and R₃ in the compound represented by formula I are as defined in any one of claims 1 to 10;
R₁, R₂, and R₃ in the compound represented by formula II are as defined in the compound represented by formula I, R₇ and R₈ are each selected from OH, or R₇ and R₈ together form 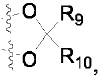 i.e., the positions C₁₆ and C₁₇ are linked via an oxygen bridge, and R₉ and R₁₀ are each independently selected from H or alkyl having 1 to 6 carbons;
A and B in the compound represented by formula III are as defined in the compound represented by formula I;
or,
reacting a compound represented by formula II' with a compound represented by formula III under acidic conditions to obtain a compound represented by formula I': 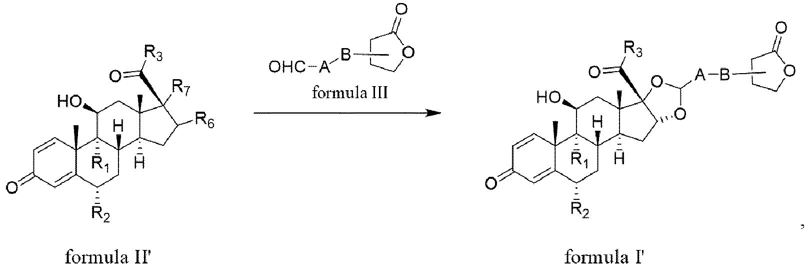
wherein A, B, R₁, R₂, and R₃ in the compound represented by formula I' are as defined in any one of claims 1 to 10;
R₁, R₂, and R₃ in the compound represented by formula II' are as defined in the compound represented by formula I', R₇ and R₈ are each selected from OH, or R₇ and R₈ together form 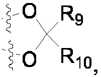 i.e., the positions C₁₆ and C₁₇ are linked via an oxygen bridge, and R₉ and R₁₀ are each independently selected from H or alkyl having 1 to 6 carbons;
A and B in the compound represented by formula III are as defined in the compound represented by formula I';
alternatively, Method II: to obtain a compound represented by formula I, wherein B is selected from CH₂NH, the following processes are used:
(a) reacting a compound represented by formula II with a compound represented by formula V under acidic conditions to obtain a compound represented by formula IV: 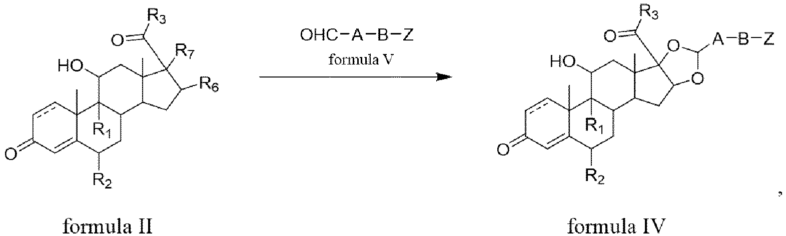
(b) reacting the compound represented by formula IV with a compound represented by formula VI or a compound represented by formula VII to obtain a compound represented by formula I: 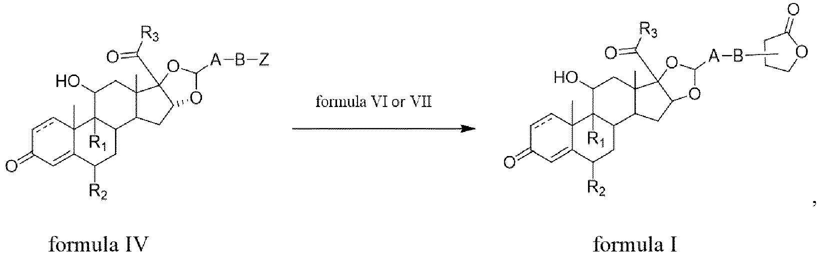
wherein A, R₁, R₂, and R₃ in the compound represented by formula I are as defined in any one of claims 1 to 10, and B is selected from CH₂NH;
R₁, R₂, and R₃ in the compound represented by formula II are as defined in the compound represented by formula I, R₇ and R₈ are each selected from OH, or R₇ and R₈ together form 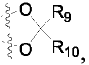 i.e., the positions C₁₆ and C₁₇ are linked via an oxygen bridge, and R₉ and R₁₀ are each independently selected from H or alkyl having 1 to 6 carbons;
A, B, R₁, R₂, and R₃ in the compound represented by formula IV are as defined in the compound represented by formula I;
A in the compound represented by formula V is as defined in the compound represented by formula I, B is selected from CH₂NH, and Z is selected from a Boc protecting group;
the compound represented by formula VI is selected from 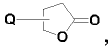 wherein Q is a leaving group selected from Cl or Br;
the formula VII is selected from 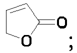 or,
(a) reacting a compound represented by formula II' with a compound represented by formula V under acidic conditions to obtain a compound represented by formula IV': 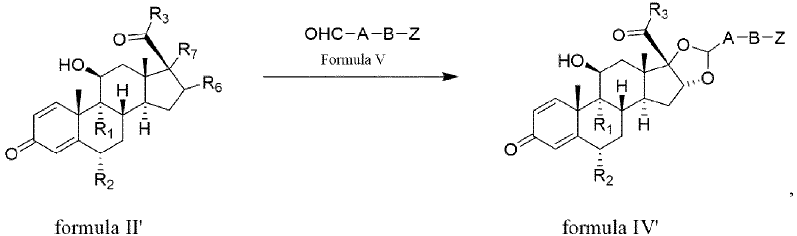
(b) reacting the compound represented by formula IV' with a compound represented by formula VI or a compound represented by formula VII to obtain a compound represented by formula I': 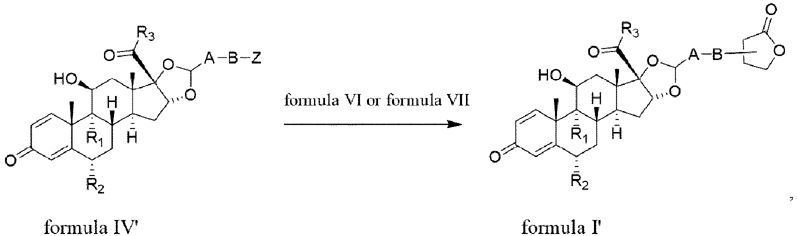
wherein A, R₁, R₂, and R₃ in the compound represented by formula I' are as defined in any one of claims 1 to 10, and B is selected from CH₂NH;
R₁, R₂, and R₃ in the compound represented by formula II' are as defined in the compound represented by formula I', R₇ and R₈ are each selected from OH, or R₇ and R₈ together form 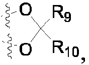 i.e., the positions C₁₆ and C₁₇ are linked via an oxygen bridge, and R₉ and R₁₀ are each independently selected from H or alkyl having 1 to 6 carbons;
A, B, R₁, R₂, and R₃ in the compound represented by formula IV' are as defined in the compound represented by formula I';
A in the compound represented by formula V is as defined in the compound represented by formula I', B is selected from CH₂NH, and Z is selected from a Boc protecting group;
the compound represented by formula VI is selected from 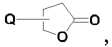 wherein Q is a leaving group selected from Cl or Br;
the formula VII is selected from

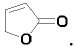

12. A pharmaceutical composition comprising the compound according to any one of claims 1 to 10 or a compound prepared by the method according to claim 11.

13. The pharmaceutical composition according to claim 12, wherein the pharmaceutical composition is in a dosage form selected from creams, ointments, gels, transdermal patches, intradermal injections, eye drops, intraocular injections, ophthalmic implants, nasal sprays, inhalation powders, inhalation aerosols, inhalation sprays, inhalation liquid preparations, vaginal suppositories, vaginal tablets, vaginal gels, and preparations for oral or rectal administration for local gastrointestinal action.

14. Use of the compound according to any one of claims 1 to 10, a compound prepared by the method according to claim 11, or the pharmaceutical composition according to claims 12 or 13, in the manufacture of medicaments for the treatment of glucocorticoid receptor-mediated diseases;
preferably, the diseases are selected from blepharitis, conjunctivitis, keratitis, iritis, iridocyclitis, uveitis, dry eye syndrome, diabetic retinopathy, wet age-related macular degeneration, choroidal neovascularisation, posterior uveitis, cataracts, glaucoma, retinal detachment, post-strabismus surgery inflammation, eczema, psoriasis, atopic dermatitis, allergic dermatitis, pruritus, hypersensitivity, rheumatoid arthritis, multiple sclerosis and lupus erythematosus disseminated, rhinitis, sinusitis, asthma, nasal polyps, asthma, chronic obstructive pulmonary disease, inflammatory bowel disease, Crohn's disease, ulcerative colitis, or chronic glomerulonephritis;
further preferably, the diseases are selected from conjunctivitis, keratitis, uveitis, dry eye syndrome, asthma, chronic obstructive pulmonary disease, allergic rhinitis, nasal polyps, Crohn's disease, eczema, and psoriasis.
